# EUROPÄISCHE PATENTSCHRIFT

(11) **EP 3 121 186 B1**
(45) Veröffentlichungstag und Bekanntmachung des Hinweises auf die Patenterteilung: **12.09.2018**
(21) Anmeldenummer: 16180047.9
(22) Anmeldetag: 19.07.2016
(51) Int. Cl.: B01J 31/22, B01J 31/24, C07C 67/38, C07F 17/02, C07F 9/572, C07F 9/655, C07F 9/6553, C07F 15/00, C07F 9/58, C07F 9/6506

(54) **FERROCENBASIERTE VERBINDUNGEN UND DARAUF BASIERENDE PALLADIUM-KATALYSATOREN FÜR DIE ALKOXYCARBONYLIERUNG ETHYLENISCH UNGESÄTTIGTER VERBINDUNGEN**
FERROCENE-BASED COMPOUNDS AND PALLADIUM CATALYSTS BASED ON SAME FOR THE ALKOXYCARBONYLATION OF ETHYLENICALLY UNSATURATED COMPOUNDS
LIAISONS A BASE DE FERROCENE ET CATALYSEURS EN PALLADIUM A BASE DE CELLES-CI POUR L'ALKOXY-CARBONYLATION DE LIAISONS INSATUREES EN ETHYLENE

(30) Priorität: 23.07.2015 DE 102015213918
(43) Veröffentlichungstag der Anmeldung: 25.01.2017
(73) Patentinhaber: Evonik Degussa GmbH, 45128 Essen (DE)
(72) Erfinder: DONG, Kaiwu, 236800 Bo Zhou (CN); NEUMANN, Helfried, 18055 Rostock (DE); JACKSTELL, Ralf, 27478 Cuxhaven Altenwalde (DE); BELLER, Matthias, 18211 Ostseebad Nienhagen (DE); FRANKE, Robert, 45772 Marl (DE); HESS, Dieter, 45770 Marl (DE); DYBALLA, Katrin Marie, 45657 Recklinghausen (DE); FRIDAG, Dirk, 45721 Haltern am See (DE); GEILEN, Frank, 45721 Haltern am See (DE)

(56) Entgegenhaltungen:
- ARMIN BUHLING ET AL: "Novel Amphiphilic Diphosphines: Synthesis, X-ray Structure, Rhodium Complexes, Use in Hydroformylation, and Rhodium Recycling", ORGANOMETALLICS, Bd. 16, Nr. 13, 1. Juni 1997 (1997-06-01), Seiten 3027-3037, XP55321527, US ISSN: 0276-7333, DOI: 10.1021/om970068w
- SIMON DOHERTY ET AL: "The first insoluble polymer-bound palladium complexes of 2-pyridyldiphenylphosphine: highly efficient catalysts for the alkoxycarbonylation of terminal alkynes", CHEMICAL COMMUNICATIONS - CHEMCOM., Nr. 1, 1. Januar 2006 (2006-01-01), Seiten 88-90, XP55321520, ISSN: 1359-7345, DOI: 10.1039/B512556A
- BIANCHINI C ET AL: "METHOXYCARBONYLATION OF ETHENE BY PALLADIUM(II) COMPLEXES WITH 1,1'-BIS(DIPHENYLPHOSPHINO)FERROCENE (DPPF) AND 1,1'-BIS(DIPHENYLPHOSPHINO)OCTAMETHYLFERRO CENE (DPPOMF)", ORGANOMETALLICS, AMERICAN CHEMICAL SOCIETY, US, Bd. 22, Nr. 12, 9. Juni 2003 (2003-06-09), Seiten 2409-2421, XP001164644, ISSN: 0276-7333, DOI: 10.1021/OM021049B

## Beschreibung

Die vorliegende Erfindung betrifft neue ferrocenbasierte Verbindungen und deren Verwendung in der Alkoxycarbonylierung.

Die Alkoxycarbonylierung ethylenisch ungesättigter Verbindungen ist ein Prozess mit steigender Bedeutung. Unter einer Alkoxycarbonylierung versteht man die Umsetzung von ethylenisch ungesättigten Verbindungen, wie Olefinen, mit Kohlenmonoxid und Alkoholen in Gegenwart eines Metalls bzw. eines Metallkomplexes und eines Liganden zu den entsprechenden Estern:

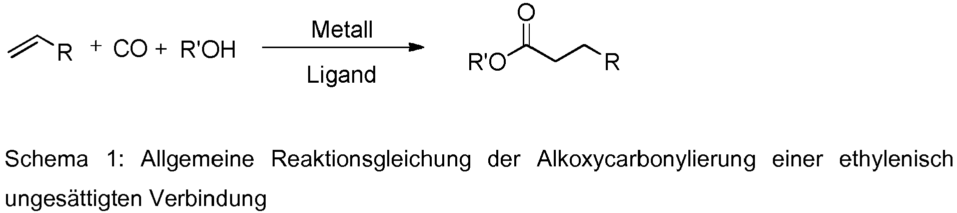

Unter den Alkoxycarbonylierungsreaktionen ist die Ethen-Methoxycarbonylierung zu 3-Methylpropionat von Bedeutung als Zwischenstufe für die Herstellung von Methylmethacrylat (S. G. Khokarale, E. J. García-Suárez, J. Xiong, U. V. Mentzel, R. Fehrmann, A. Riisager, Catalysis Communications 2014, 44, 73-75). Die Ethen-Methoxycarbonylierung wird in Methanol als Lösemittel bei milden Bedingungen mit einem durch Phosphinliganden modifizierten Palladiumkatalysator durchgeführt.

Ein sehr gutes katalytischen System wurde von Lucite - jetzt Mitsubishi Rayon - entwickelt und verwendet einen Liganden auf Basis von 1,2-Bis(di-tert-butylphosphinomethyl)benzol (DTBPMB) (W. Clegg, G. R. Eastham, M. R. J. Elsegood, R. P. Tooze, X. L. Wang, K. Whiston, Chem. Commun 1999, 1877-1878).

Anwendungen der Methoxycarbonylierung auf längerkettige Substrate sind z.B. in EP 0 662 467 beschrieben. Die Patentschrift beschreibt einen Prozess zur Herstellung von Dimethyladipat aus Methyl-3-Pentenoat. Als Pd-Quelle wird Pd(II)acetat verwendet. Als Beispiele geeigneter bidentater Phosphinliganden werden unter anderem 1,1'-Bis(diphenylphosphino)ferrocen, 1-(Diphenylphosphino)-1'-(diisopropylphosphino)ferrocen und 1,1'-Bis(isopropylphenylphosphino)ferrocen genannt. Die Liganden erzielen jedoch nur unzureichende Ausbeuten bei der Methoxycarbonylierung von Olefinen, insbesondere von langkettigen Olefinen wie 2-Octen und Di-n-Buten.

In BIANCHINI C ET AL, "METHOXYCARBONYLATION OF ETHENE BY PALLADIUM(II) COMPLEXES WITH 1,1'-BIS(DIPHENYLPHOSPHINO)FERROCENE (DPPF) AND 1,1'-BIS(DIPHENYLPHOSPHINO)OCTAMETHYLFERROCENE (DPPOMF)", ORGANOMETALLICS, AMERICAN CHEMICAL SOCIETY, US, (20030609), Vol. 22, Nr. 12, Seiten 2409 - 2421 wird ein Verfahren zur Methoxycarbonylierung von Ethen beschrieben. Hierbei kommen die beiden folgenden Pd-Komplexe als Katalysatoren zum Einsatz: [Pd(H₂O)₂(dppf)](OTs)₂ und [Pd(H₂O)₂(dppomf)](OTs)₂.

Die technische Aufgabe, welche der vorliegenden Erfindung zugrunde lag, ist die Bereitstellung neuer ferrocenbasierter Verbindungen als Liganden für Alkoxycarbonylierungsreaktionen. Diese Verbindungen sollen insbesondere bei der Umsetzung von langkettigen Olefinen wie 2-Octen oder Di-n-Buten verbesserte Ausbeuten erzielen. Insbesondere soll bei der Alkoxycarbonylierungsreaktion die Raum-Zeit-Ausbeute gesteigert werden.

Diese Aufgabe wird gelöst durch Diphosphinverbindungen gemäß Formel (**I**) 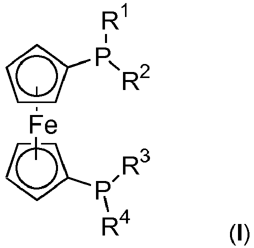 wobei
R¹, R², R³, R⁴ jeweils unabhängig voneinander ausgewählt sind aus -(C₁-C₁₂)-Alkyl, -(C₃-C₁₂)-Cycloalkyl, -(C₃-C₁₂)-Heterocycloalkyl, -(C₆-C₂₀)-Aryl, -(C₃-C₂₀)-Heteroaryl;
mindestens einer der Reste R¹, R², R³, R⁴ für einen -(C₆-C₂₀)-Heteroarylrest mit mindestens sechs Ringatomen steht;
   und
R¹, R², R³, R⁴, falls diese für -(C₁-C₁₂)-Alkyl, -(C₃-C₁₂)-Cycloalkyl, -(C₃-C₁₂)-Heterocycloalkyl,-(C₆-C₂₀)-Aryl, -(C₃-C₂₀)-Heteroaryl oder -(C₆-C₂₀)-Heteroaryl stehen,
jeweils unabhängig voneinander mit einem oder mehreren Substituenten ausgewählt aus -(C₁-C₁₂)-Alkyl, -(C₃-C₁₂)-Cycloalkyl, -(C₃-C₁₂)-Heterocycloalkyl, -O-(C₁-C₁₂)-Alkyl, -O-(C₁-C₁₂)-Alkyl-(C₆-C₂₀)-Aryl, -O-(C₃-C₁₂)-Cycloalkyl, -S-(C₁-C₁₂)-Alkyl, -S-(C₃-C₁₂)-Cycloalkyl, -COO-(C₁-C₁₂)-Alkyl, -COO-(C₃-C₁₂)-Cycloalkyl, -CONH-(C₁-C₁₂)-Alkyl, -CONH-(C₃-C₁₂)-Cycloalkyl,-CO-(C₁-C₁₂)-Alkyl, -CO-(C₃-C₁₂)-Cycloalkyl, -N-[(C₁-C₁₂)-Alkyl]₂, -(C₆-C₂₀)-Aryl, -(C₆-C₂₀)-Aryl-(C₁-C₁₂)-Alkyl, -(C₆-C₂₀)-Aryl-O-(C₁-C₁₂)-Alkyl, -(C₃-C₂₀)-Heteroaryl, -(C₃-C₂₀)-Heteroaryl-(C₁-C₁₂)-Alkyl, -(C₃-C₂₀)-Heteroaryl-O-(C₁-C₁₂)-Alkyl, -COOH, -OH, -SO₃H, -NH₂, Halogen substituiert sein können.

Die erfindungsgemäßen Verbindungen eignen sich als zweizähnige Phosphinliganden für Pd-Komplexe, mit denen sich bei der Alkoxycarbonylierung einer Vielzahl ethylenisch ungesättigter Verbindungen hohe Ausbeuten erzielen lassen. Insbesondere eignen sich die erfindungsgemäßen Verbindungen zur Alkoxycarbonylierung langkettiger Olefine wie 1-Octen oder Di-n-Buten.

Der Begriff (C₁-C₁₂)-Alkyl umfasst geradkettige und verzweigte Alkylgruppen mit 1 bis 12 Kohlenstoffatomen. Vorzugsweise handelt es sich dabei um (C₁-C₈)-Alkylgruppen, besonders bevorzugt (C₁-C₆)-Alkyl, am meisten bevorzugt (C₁-C₄)-Alkyl.

Geeignete (C₁-C₁₂)-Alkylgruppen sind insbesondere Methyl, Ethyl, Propyl, Isopropyl, n-Butyl, *iso*-Butyl, *sec*-Butyl, *tert*-Butyl, *n*-Pentyl, 2-Pentyl, 2-Methylbutyl, 3-Methylbutyl, 1,2-Dimethylpropyl, 1,1-Dimethylpropyl, 2,2-Dimethylpropyl, 1-Ethylpropyl, n-Hexyl, 2-Hexyl, 2-Methylpentyl, 3-Methylpentyl, 4-Methylpentyl, 1,1-Dimethylbutyl, 1,2-Dimethylbutyl, 2,2-Dimethylbutyl, 1,3-Dimethylbutyl, 2,3-Dimethylbutyl, 3,3-Dimethylbutyl, 1,1,2-Trimethylpropyl, 1,2,2-Trimethylpropyl, 1-Ethylbutyl, 1-Ethyl-2-methylpropyl, *n*-Heptyl, 2-Heptyl, 3-Heptyl, 2-Ethylpentyl, 1-Propylbutyl, *n*-Octyl, 2-Ethylhexyl, 2-Propylheptyl, Nonyl, Decyl.

Die Erläuterungen zum Begriff (C₁-C₁₂)-Alkyl gelten insbesondere auch für die Alkylgruppen in -O-(C₁-C₁₂)-Alkyl, -S-(C₁-C₁₂)-Alkyl, -COO-(C₁-C₁₂)-Alkyl, -CONH-(C₁-C₁₂)-Alkyl, -CO-(C₁-C₁₂)-Alkyl und -N-[(C₁-C₁₂)-Alkyl]₂.

Der Begriff (C₃-C₁₂)-Cycloalkyl umfasst mono-, bi- oder tricyclische Kohlenwasserstoffgruppen mit 3 bis 12 Kohlenstoffatomen. Vorzugsweise handelt es sich dabei um (C₅-C₁₂)-Cycloalkyl.

Die (C₃-C₁₂)-Cycloalkylgruppen weisen vorzugsweise 3 bis 8, besonders bevorzugt 5 oder 6, Ringatome auf.

Geeignete (C₃-C₁₂)-Cycloalkylgruppen sind insbesondere Cyclopropyl, Cyclobutyl, Cyclopentyl, Cyclohexyl, Cycloheptyl, Cyclooctyl, Cyclododecyl, Cyclopentadecyl, Norbonyl, Adamantyl.

Die Erläuterungen zum Begriff (C₃-C₁₂)-Cycloalkyl gelten insbesondere auch für die Cycloalkylgruppen in -O-(C₃-C₁₂)-Cycloalkyl, -S-(C₃-C₁₂)-Cycloalkyl, -COO-(C₃-C₁₂)-Cycloalkyl, -CONH-(C₃-C₁₂)-Cycloalkyl, -CO-(C₃-C₁₂)-Cycloalkyl.

Der Begriff (C₃-C₁₂)-Heterocycloalkyl umfasst nichtaromatische, gesättigte oder teilweise ungesättigte cycloaliphatische Gruppen mit 3 bis 12 Kohlenstoffatomen, wobei eins oder mehrere der Ringkohlenstoffatome durch Heteroatome ersetzt sind. Die (C₃-C₁₂)-Heterocycloalkylgruppen weisen vorzugsweise 3 bis 8, besonders bevorzugt 5 oder 6, Ringatome auf und sind ggf. mit aliphatischen Seitenketten substituiert. In den Heterocycloalkylgruppen sind im Unterschied zu den Cycloalkylgruppen eins oder mehrere der Ringkohlenstoffatome durch Heteroatome oder heteroatomhaltige Gruppen ersetzt. Die Heteroatome oder die heteroatomhaltigen Gruppen sind vorzugsweise ausgewählt unter O, S, N, N(=O), C(=O), S(=O). Eine (C₃-C₁₂)-Heterocycloalkylgruppe im Sinne dieser Erfindung ist somit auch Ethylenoxid.

Geeignete (C₃-C₁₂)-Heterocycloalkylgruppen sind insbesondere Tetrahydrothiophenyl, Tetrahydrofuryl, Tetrahydropyranyl und Dioxanyl.

Der Begriff (C₆-C₂₀)-Aryl umfasst mono- oder polycyclische aromatische Kohlenwasserstoffreste mit 6 bis 20 Kohlenstoffatomen. Vorzugsweise handelt es sich dabei um (C₆-C₁₄)-Aryl, besonders bevorzugt (C₆-C₁₀)-Aryl.

Geeignete (C₆-C₂₀)-Arylgruppen sind insbesondere Phenyl, Naphthyl, Indenyl, Fluorenyl, Anthracenyl, Phenanthrenyl, Naphthacenyl, Chrysenyl, Pyrenyl, Coronenyl. Bevorzugte (C₆-C₂₀)-Arylgruppen sind Phenyl, Naphthyl und Antracenyl.

Der Begriff (C₃-C₂₀)-Heteroaryl umfasst mono- oder polycyclische aromatische Kohlenwasserstoffreste mit 3 bis 20 Kohlenstoffatomen, wobei eins oder mehrere der Kohlenstoffatome durch Heteroatome ersetzt sind. Bevorzugte Heteroatome sind N, O und S. Die (C₃-C₂₀)-Heteroarylgruppen weisen 3 bis 20, bevorzugt 6 bis 14, besonders bevorzugt 6 bis 10 Ringatome auf. Somit ist beispielsweise Pyridyl im Rahmen dieser Erfindung ein C₆-Heteroarylrest, Furyl ist ein C₅-Heteroarylrest.

Geeignete (C₃-C₂₀)-Heteroarylgruppen sind insbesondere Furyl, Thienyl, Pyrrolyl, Oxazolyl, Isoxazolyl, Thiazolyl, Isothiazolyl, Imidazolyl, Pyrazolyl, Furazanyl, Tetrazolyl, Pyridyl, Pyridazinyl, Pyrimidyl, Pyrazinyl, Benzofuranyl, Indolyl, Isoindolyl, Benzimidazolyl, Chinolyl, Isochinolyl.

Der Begriff (C₃-C₂₀)-Heteroaryl umfasst auch (C₆-C₂₀)-Heteroarylreste mit mindestens sechs Ringatomen.

Der Begriff (C₆-C₂₀)-Heteroaryl mit mindestens sechs Ringatomen umfasst mono- oder polycyclische aromatische Kohlenwasserstoffreste mit 6 bis 20 Kohlenstoffatomen, wobei eins oder mehrere der Kohlenstoffatome durch Heteroatome ersetzt sind. Bevorzugte Heteroatome sind N, O und S. Die (C₆-C₂₀)-Heteroarylgruppen weisen 6 bis 14, besonders bevorzugt 6 bis 10 Ringatome auf.

Geeignete (C₆-C₂₀)-Heteroarylgruppen mit mindestens sechs Ringatomen sind insbesondere Pyridyl, Pyridazinyl, Pyrimidyl, Pyrazinyl, Benzofuranyl, Indolyl, Isoindolyl, Benzimidazolyl, Chinolyl, Isochinolyl.

Der Begriff Halogen umfasst insbesondere Fluor, Chlor, Brom und lod. Besonders bevorzugt sind Fluor und Chlor.

In einer Ausführungsform können die Reste R¹, R², R³, R⁴, falls sie für -(C₁-C₁₂)-Alkyl, , -(C₃-C₁₂)-Cycloalkyl, -(C₃-C₁₂)-Heterocycloalkyl, -(C₆-C₂₀)-Aryl, -(C₃-C₂₀)-Heteroaryl oder -(C₆-C₂₀)-Heteroaryl stehen, jeweils unabhängig voneinander mit einem oder mehreren Substituenten ausgewählt aus -(C₁-C₁₂)-Alkyl, -(C₃-C₁₂)-Cycloalkyl, -(C₃-C₁₂)-Heterocycloalkyl, -O-(C₁-C₁₂)-Alkyl, -O-(C₁-C₁₂)-Alkyl-(C₆-C₂₀)-Aryl, -O-(C₃-C₁₂)-Cycloalkyl, -S-(C₁-C₁₂)-Alkyl, -S-(C₃-C₁₂)-Cycloalkyl, -(C₆-C₂₀)-Aryl, -(C₆-C₂₀)-Aryl-(C₁-C₁₂)-Alkyl, -(C₆-C₂₀)-Aryl-O-(C₁-C₁₂)-Alkyl, -(C₃-C₂₀)-Heteroaryl, -(C₃-C₂₀)-Heteroaryl-(C₁-C₁₂)-Alkyl, -(C₃-C₂₀)-Heteroaryl-O-(C₁-C₁₂)-Alkyl,-COOH, -OH, -SO₃H, -NH₂, Halogen substituiert sein.

In einer Ausführungsform können die Reste R¹, R², R³, R⁴, falls sie für -(C₁-C₁₂)-Alkyl, -(C₃-C₁₂)-Cycloalkyl, -(C₃-C₁₂)-Heterocycloalkyl, -(C₆-C₂₀)-Aryl, -(C₃-C₂₀)-Heteroaryl oder -(C₆-C₂₀)-Heteroaryl stehen, jeweils unabhängig voneinander mit einem oder mehreren Substituenten ausgewählt aus -(C₁-C₁₂)-Alkyl, -(C₃-C₁₂)-Cycloalkyl, -O-(C₁-C₁₂)-Alkyl, -O-(C₁-C₁₂)-Alkyl-(C₆-C₂₀)-Aryl, -O-(C₃-C₁₂)-Cycloalkyl, -(C₆-C₂₀)-Aryl, -(C₆-C₂₀)-Aryl-(C₁-C₁₂)-Alkyl, -(C₆-C₂₀)-Aryl-O-(C₁-C₁₂)-Alkyl, -(C₃-C₂₀)-Heteroaryl, -(C₃-C₂₀)-Heteroaryl-(C₁-C₁₂)-Alkyl, -(C₃-C₂₀)-Heteroaryl-O-(C₁-C₁₂)-Alkyl, substituiert sein.

In einer Ausführungsform können die Reste R¹, R², R³, R⁴, falls sie für -(C₁-C₁₂)-Alkyl, -(C₃-C₁₂)-Cycloalkyl, -(C₃-C₁₂)-Heterocycloalkyl, -(C₆-C₂₀)-Aryl, -(C₃-C₂₀)-Heteroaryl oder -(C₆-C₂₀)-Heteroaryl stehen, jeweils unabhängig voneinander mit einem oder mehreren Substituenten ausgewählt aus -(C₁-C₁₂)-Alkyl, -O-(C₁-C₁₂)-Alkyl-(C₆-C₂₀)-Aryl, -(C₃-C₂₀)-Heteroaryl, -(C₃-C₂₀)-Heteroaryl-(C₁-C₁₂)-Alkyl, -(C₃-C₂₀)-Heteroaryl-O-(C₁-C₁₂)-Alkyl substituiert sein.

In einer Ausführungsform können die Reste R¹, R², R³, R⁴, falls sie für -(C₁-C₁₂)-Alkyl, -(C₃-C₁₂)-Cycloalkyl, -(C₃-C₁₂)-Heterocycloalkyl, -(C₆-C₂₀)-Aryl, -(C₃-C₂₀)-Heteroaryl oder -(C₆-C₂₀)-Heteroaryl stehen, jeweils unabhängig voneinander mit einem oder mehreren Substituenten ausgewählt aus -(C₁-C₁₂)-Alkyl und -(C₃-C₂₀)-Heteroaryl substituiert sein.

In einer Ausführungsform sind die Reste R¹, R², R³, R⁴ unsubstituiert, falls sie für -(C₁-C₁₂)-Alkyl, -(C₃-C₁₂)-Cycloalkyl, oder -(C₃-C₁₂)-Heterocycloalkyl stehen, und können wie beschrieben substituiert sein, falls sie für -(C₆-C₂₀)-Aryl, -(C₃-C₂₀)-Heteroaryl oder -(C₆-C₂₀)-Heteroaryl stehen.

In einer Ausführungsform sind die Reste R¹, R², R³, R⁴ unsubstituiert, falls sie für -(C₁-C₁₂)-Alkyl, -(C₃-C₁₂)-Cycloalkyl, -(C₃-C₁₂)-Heterocycloalkyl, -(C₆-C₂₀)-Aryl, -(C₃-C₂₀)-Heteroaryl oder -(C₆-C₂₀)-Heteroaryl stehen.

In einer Ausführungsform sind R¹, R², R³, R⁴ jeweils unabhängig voneinander ausgewählt aus -(C₁-C₁₂)-Alkyl, -(C₆-C₂₀)-Aryl, -(C₃-C₂₀)-Heteroaryl;
wobei
mindestens einer der Reste R¹, R², R³, R⁴ für einen -(C₆-C₂₀)-Heteroarylrest mit mindestens sechs Ringatomen steht;
und
R¹, R², R³, R⁴, falls diese für -(C₁-C₁₂)-Alkyl, -(C₆-C₂₀)-Aryl, -(C₃-C₂₀)-Heteroaryl oder -(C₆-C₂₀)-Heteroaryl stehen,
jeweils unabhängig voneinander mit einem oder mehreren der oben beschriebenen Substituenten substituiert sein können.

In einer Ausführungsform stehen mindestens zwei der Reste R¹, R², R³, R⁴ für einen -(C₆-C₂₀)-Heteroarylrest mit mindestens sechs Ringatomen.

In einer Ausführungsform stehen die Reste R¹ und R³ jeweils für einen -(C₆-C₂₀)-Heteroarylrest mit mindestens sechs Ringatomen und können jeweils unabhängig voneinander mit einem oder mehreren der oben beschriebenen Substituenten substituiert sein. Vorzugsweise steht R² dabei für einen -(C₆-C₂₀)-Heteroarylrest mit mindestens sechs Ringatomen oder ist ausgewählt aus -(C₁-C₁₂)-Alkyl, -(C₃-C₁₂)-Cycloalkyl, -(C₃-C₁₂)-Heterocycloalkyl, -(C₆-C₂₀)-Aryl, am meisten bevorzugt aus -(C₁-C₁₂)-Alkyl, -(C₃-C₁₂)-Cycloalkyl, -(C₆-C₂₀)-Aryl. R⁴ ist dabei vorzugsweise ausgewählt aus -(C₁-C₁₂)-Alkyl, -(C₃-C₁₂)-Cycloalkyl, -(C₃-C₁₂)-Heterocycloalkyl, -(C₆-C₂₀)-Aryl, am meisten bevorzugt aus -(C₁-C₁₂)-Alkyl, -(C₃-C₁₂)-Cycloalkyl, -(C₆-C₂₀)-Aryl.

In einer Ausführungsform stehen die Reste R¹ und R³ jeweils für einen -(C₆-C₂₀)-Heteroarylrest mit mindestens sechs Ringatomen und R² und R⁴ sind ausgewählt aus -(C₁-C₁₂)-Alkyl, -(C₃-C₁₂)-Cycloalkyl, -(C₃-C₁₂)-Heterocycloalkyl, -(C₆-C₂₀)-Aryl. R¹, R², R³, R⁴ können dabei jeweils unabhängig voneinander mit einem oder mehreren der oben beschriebenen Substituenten substituiert sein.

Besonders bevorzugt stehen die Reste R¹ und R³ jeweils für einen -(C₆-C₂₀)-Heteroarylrest mit mindestens sechs Ringatomen und R² und R⁴ für-(C₁-C₁₂)-Alkyl. R¹, R², R³, R⁴ können dabei jeweils unabhängig voneinander mit einem oder mehreren der oben beschriebenen Substituenten substituiert sein.

In einer Ausführungsform stehen die Reste R¹, R², R³ jeweils für einen -(C₆-C₂₀)-Heteroarylrest mit mindestens sechs Ringatomen und können jeweils unabhängig voneinander mit einem oder mehreren der oben beschriebenen Substituenten substituiert sein. Vorzugsweise steht R⁴ dabei nicht für einen -(C₃-C₂₀)-Heteroarylrest. Besonders bevorzugt ist R⁴ dabei ausgewählt aus -(C₁-C₁₂)-Alkyl, -(C₃-C₁₂)-Cycloalkyl, -(C₃-C₁₂)-Heterocycloalkyl, -(C₆-C₂₀)-Aryl, am meisten bevorzugt aus -(C₁-C₁₂)-Alkyl, -(C₃-C₁₂)-Cycloalkyl, -(C₆-C₂₀)-Aryl.

In einer Ausführungsform stehen die Reste R¹, R², R³ und R⁴ für einen -(C₆-C₂₀)-Heteroarylrest mit mindestens sechs Ringatomen und können jeweils unabhängig voneinander mit einem oder mehreren der oben beschriebenen Substituenten substituiert sein.

In einer Ausführungsform sind die Reste R¹, R², R³ und R⁴, falls sie für einen Heteroarylrest stehen, jeweils unabhängig voneinander ausgewählt aus Heteroarylresten mit sechs bis zehn Ringatomen.

In einer Ausführungsform stellen die Reste R¹, R², R³ und R⁴, falls sie für einen Heteroarylrest stehen, einen Heteroarylrest mit sechs Ringatomen dar.

Vorzugsweise sind die Reste R¹, R², R³ und R⁴, falls sie für einen Heteroarylrest stehen, jeweils unabhängig voneinander ausgewählt aus Pyridyl, Pyridazinyl, Pyrimidyl, Pyrazinyl, Benzofuranyl, Indolyl, Isoindolyl, Benzimidazolyl, Chinolyl, Isochinolyl, wobei die genannten Heteroarylreste wie oben beschrieben substituiert sein können.

In einer Ausführungsform sind die Reste R¹, R², R³ und R⁴, falls sie für einen Heteroarylrest stehen, jeweils unabhängig voneinander ausgewählt aus Pyridyl, Pyrimidyl, Indolyl, wobei die genannten Heteroarylreste wie oben beschrieben substituiert sein können.

Vorzugsweise sind die Reste R¹, R², R³ und R⁴, falls sie für einen Heteroarylrest stehen, jeweils unabhängig voneinander ausgewählt aus 2-Pyridyl, 2-Pyrimidyl, 2-Indolyl, wobei die genannten Heteroarylreste wie oben beschrieben substituiert sein können.

Besonders bevorzugt sind die Reste R¹, R², R³ und R⁴, falls sie für einen Heteroarylrest stehen, jeweils unabhängig voneinander ausgewählt aus 2-Pyridyl, 2-Pyrimidyl, N-Phenyl-2-Indolyl, 2-Indolyl, wobei die genannten Heteroarylreste nicht weiter substituiert sind.

In einer Ausführungsform stehen die Reste R¹ und R³ jeweils für einen Heteroarylrest ausgewählt aus Pyridyl und Pyrimidyl, insbesondere 2-Pyridyl und 2-Pyrimidyl,
wobei die Reste R² und R⁴ jeweils unabhängig voneinander ausgewählt sind aus -(C₁-C₁₂)-Alkyl, -(C₃-C₁₂)-Cycloalkyl, -(C₃-C₁₂)-Heterocycloalkyl, -(C₆-C₂₀)-Aryl;
und
R¹ und R³ sowie R² und R⁴, falls diese für -(C₁-C₁₂)-Alkyl, -(C₃-C₁₂)-Cycloalkyl, -(C₃-C₁₂)-Heterocycloalkyl oder -(C₆-C₂₀)-Aryl stehen,
jeweils unabhängig voneinander mit einem oder mehreren der oben beschriebenen Substituenten substituiert sein können.

In einer Ausführungsform stehen die Reste R¹ und R³ jeweils für einen Heteroarylrest mit sechs Ringatomen, und die Reste R² und R⁴ stehen jeweils für -(C₁-C₁₂)-Alkyl;
wobei
R¹, R³ jeweils unabhängig voneinander mit einem oder mehreren der oben beschriebenen Substituenten substituiert sein können.

In einer Ausführungsform weist die Verbindung eine Struktur gemäß einer der Formeln (**8**), (**14**) und (**15**) auf:

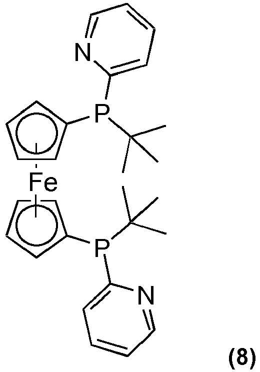

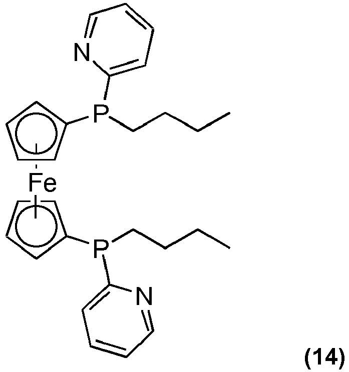

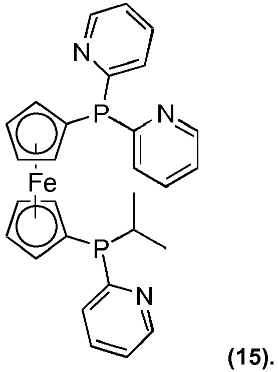

Die erfindungsgemäßen Diphosphinverbindungen können beispielsweise durch Umsetzung von Ferrocen mit Butyllithium und einer Chlorphosphinverbindung gewonnen werden.

Die Erfindung betrifft weiterhin Komplexe umfassend Pd und eine erfindungsgemäße Diphosphinverbindung. Bei diesen Komplexen dient die erfindungsgemäße Diphosphinverbindung als zweizähniger Ligand für das Metallatom. Die Komplexe dienen beispielsweise als Katalysatoren für die Alkoxycarbonylierung. Mit den erfindungsgemäßen Komplexen lassen sich hohe Ausbeuten bei der Alkoxycarbonylierung einer Vielzahl unterschiedlicher ethylenisch ungesättigter Verbindungen erzielen.

Die erfindungsgemäßen Komplexe können außerdem weitere Liganden umfassen, die an das Metallatom koordinieren. Dabei handelt es sich beispielsweise um ethylenisch ungesättigte Verbindungen oder Anionen. Geeignete zusätzliche Liganden sind beispielsweise Styrol, Acetat-Anionen, Maleinimide (z.B. N-Methylmaleinimid), 1,4-Napthochinon, Trifluoroacetat-Anionen oder Chloridanionen.

Die Erfindung betrifft weiterhin die Verwendung einer erfindungsgemäßen Diphosphinverbindung zur Katalyse einer Alkoxycarbonylierungsreaktion. Dabei kann die erfindungsgemäße Verbindung insbesondere als erfindungsgemäßer Metallkomplex eingesetzt werden.

Die Erfindung betrifft außerdem ein Verfahren umfassend die Verfahrensschritte:
a) Vorlegen einer ethylenisch ungesättigten Verbindung;
b) Zugabe einer erfindungsgemäßen Diphosphinverbindung und einer Verbindung, welche Pd umfasst,
   oder Zugabe eines erfindungsgemäßen Komplexes umfassend Pd und eine erfindungsgemäße Diphosphinverbindung;
c) Zugabe eines Alkohols;
d) Zuführen von CO;
e) Erwärmen des Reaktionsgemisches, wobei die ethylenisch ungesättigte Verbindung zu einem Ester umgesetzt wird.

Hierbei können die Verfahrensschritte a), b), c) und d) in beliebiger Reihenfolge erfolgen. Üblicherweise erfolgt die Zugabe von CO jedoch, nachdem die Reaktionspartner in den Schritten a) bis c) vorgelegt wurden. Die Schritte d) und e) können gleichzeitig oder nacheinander erfolgen. Darüber hinaus kann CO auch in mehreren Schritten zugeführt werden, so dass beispielsweise zunächst ein Teil des CO zugeführt, dann erwärmt und anschließend ein weiterer Teil CO zugeführt wird.

Die in dem erfindungsgemäßen Verfahren als Edukt eingesetzten ethylenisch ungesättigten Verbindungen enthalten eine oder mehrere Kohlenstoff-Kohlenstoff-Doppelbindungen. Diese Verbindungen werden auch im Folgenden zur Vereinfachung als Olefine bezeichnet. Die Doppelbindungen können terminal oder intern sein.

Bevorzugt sind ethylenisch ungesättigte Verbindungen mit 2 bis 30 Kohlenstoffatomen, bevorzugt 2 bis 22 Kohlenstoffatomen, besonders bevorzugt 2 bis 12 Kohlenstoffatomen.

In einer Ausführungsform umfasst die ethylenisch ungesättigte Verbindung 4 bis 30 Kohlenstoffatome, bevorzugt 6 bis 22 Kohlenstoffatome, besonders bevorzugt 8 bis 12 Kohlenstoffatome, am meisten bevorzugt 8 Kohlenstoffatome.

Die ethylenisch ungesättigten Verbindungen können zusätzlich zu der einen oder mehreren Doppelbindungen weitere funktionelle Gruppen enthalten. Vorzugsweise umfasst die ethylenisch ungesättigte Verbindung eine oder mehrere funktionelle Gruppen ausgewählt aus Carboxyl-, Thiocarboxyl-, Sulfo-, Sulfinyl-, Carbonsäureanhydrid-, Imid-, Carbonsäureester-, Sulfonsäureester-, Carbamoyl-, Sulfamoyl-, Cyano-, Carbonyl-, Carbonothioyl-, Hydroxyl-, Sulfhydryl- , Amino-, Ether-, Thioether-, Aryl-, Heteroaryl- oder Silylgruppen und/oder Halogensubstituenten. Dabei umfasst die ethylenisch ungesättigte Verbindung vorzugsweise insgesamt 2 bis 30 Kohlenstoffatome, bevorzugt 2 bis 22 Kohlenstoffatome, besonders bevorzugt 2 bis 12 Kohlenstoffatome.

In einer Ausführungsform umfasst die ethylenisch ungesättigte Verbindung keine weiteren funktionellen Gruppen außer Kohlenstoff-Kohlenstoff-Doppelbindungen.

In einer besonders bevorzugten Ausführungsform handelt es sich bei der ethylenisch ungesättigten Verbindung um ein unfunktionalisiertes Alken mit mindestens einer Doppelbindung und 2 bis 30 Kohlenstoffatomen, bevorzugt 6 bis 22 Kohlenstoffatomen, weiter bevorzugt 8 bis 12 Kohlenstoffatomen, am meisten bevorzugt 8 Kohlenstoffatomen.

Geeignete ethylenisch ungesättigte Verbindungen sind beispielsweise:
Ethen;
Propen;
C4-Olefine, wie 1-Buten, cis-2-Buten, trans-2-Buten, Gemisch von cis- und trans-2-Buten, Isobuten, 1,3-Butadien; Raffinat I bis III, Crack-C4
C5-Olefine, wie 1-Penten, 2-Penten, 2-Methyl-1-buten, 2-Methyl-2-buten, 2-Methyl-1,3-butadien (Isopren), 1,3-Pentadien;
C6-Olefine, wie Tetramethylethylen, 1,3-Hexadien, 1,3-Cyclohexadien;
C7-Olefine, wie 1-Methylcyclohexen, 2,4-Heptadien, Norbonadien;
C8-Olefine, wie 1-Octen, 2-Octen, Cycloocten, Di-n-Buten, Di-iso-buten, 1,5-Cyclooctadien, 1,7-Octadien;
C9-Olefine, wie Tripropen;
C10-Olefine, wie Dicylcopentadien;
Undecene;
Dodecene;
interne C14-Olefine;
interne C15- bis C18-Olefine;
lineare oder verzweigte, cyclische, acyclische oder teilweise cyclische, interne C15- bis C30-Olefine;
Triisobuten, Tri-n-buten;
Terpene, wie Limonen, Geraniol, Farnesol, Pinen, Myrcen, Carvon, 3-Caren;
mehrfach ungesättigte Verbindungen mit 18 Kohlenstoffatomen, wie Linolsäure oder Linolensäure;
Ester ungesättigter Carbonsäuren, wie Vinylester von Essig- oder Propansäure, Alkylester ungesättigter Carbonsäuren, Methyl- oder Ethylester von Acrylsäure oder Methacrylsäure, Ölsäureester, Ölsäure Methyl- oder Ethylester, Ester der Linol- oder Linolensäure; Vinylverbindungen, wie Vinylacetat, Vinylcyclohexen, Styrol, alpha-Methylstyrol, 2-Isopropenylnaphthalin;
2-Methyl-2-pentenal, Methyl-3-Pentenoat, Methacrylanhydrid.

In einer Variante des Verfahrens ist die ethylenisch ungesättigte Verbindung ausgewählt aus Propen, 1-Buten, cis- und/oder trans-2-Buten, oder Mischungen davon.

In einer Variante des Verfahrens ist die ethylenisch ungesättigte Verbindung ausgewählt aus 1-Penten, cis- und/oder trans-2-Penten, 2-Methyl-1-buten, 2-Methyl-2-buten, 3-Methyl-1-buten, oder Mischungen davon.

In einer bevorzugten Ausführungsform ist die ethylenisch ungesättigte Verbindung ausgewählt aus Ethen, Propen, 1-Buten, *cis-* und/oder *trans*-2-Buten, iso-Buten, 1,3-Butadien, 1-Penten, *cis-* und/oder *trans*-2-Penten, 2-Methyl-1-buten, 3-Methyl-1-buten, 2-Methyl-2-buten, Hexen, Tetramethylethylen, Hepten, n-Octen, 1-Octen, 2-Octen, oder Mischungen davon.

In einer Variante wird ein Gemisch ethylenisch ungesättigter Verbindungen eingesetzt. Als Gemisch wird im Sinne dieser Erfindung eine Zusammensetzung bezeichnet, die wenigsten zwei verschiedene ethylenisch ungesättigte Verbindungen enthält, wobei der Anteil jeder einzelnen ethylenisch ungesättigten Verbindungen vorzugsweise wenigstens 5 Gewichts-% bezogen auf das Gesamtgewicht des Gemisches beträgt.

Vorzugsweise wird ein Gemisch ethylenisch ungesättigter Verbindungen mit jeweils 2 bis 30 Kohlenstoffatomen, bevorzugt 4 bis 22 Kohlenstoffatomen, besonders bevorzugt 6 bis 12 Kohlenstoffatomen, am meisten bevorzugt 8 bis 10 Kohlenstoffatomen eingesetzt.

Geeignete Gemische ethylenisch ungesättigter Verbindungen sind die sogenannten Raffinate I bis III. Raffinat I umfasst 40 bis 50 % iso-Buten, 20 bis 30 % 1-Buten, 10 bis 20 % cis- und trans-2-Buten, bis zu 1 % 1,3-Butadien und 10 bis 20 % n-Butan und Isobutan. Das Raffinat II ist ein Teil der beim Naphthacracken entstehenden C₄-Fraktion und besteht im Wesentlichen aus den isomeren *n*-Butenen, Isobutan und *n*-Butan nach Abtrennung von Isobuten aus Raffinat I. Raffinat III ist ein Teil der beim Naphthacracken entstehenden C₄-Fraktion und besteht im Wesentlichen aus den isomeren *n*-Butenen und *n*-Butan.

Ein weiteres geeignetes Gemisch ist Di-n-Buten, auch bezeichnet als Dibuten, DNB oder DnB. Di-n-Buten ist ein Isomerengemisch von C8-Olefinen, das aus der Dimerisierung von Gemischen aus 1-Buten, cis-2-Buten und trans-2-Buten entsteht. Technisch werden der Regel Raffinat II oder Raffinat III-Ströme einer katalytischen Oligomerisierung unterworfen, wobei die enthaltenen Butane (n/iso) unverändert hervorgehen und die enthaltenen Olefine ganz oder teilweise umgesetzt werden. Neben dem dimeren Di-n-Buten, entstehen in der Regel auch höhere Oligomere (Tributen C12, Tetrabuten C16), die nach der Reaktion destillativ abgetrennt werden. Diese können ebenfalls als Edukte eingesetzt werden.

In einer bevorzugten Variante wird ein Gemisch umfassend iso-Buten, 1-Buten, cis- und trans-2-Buten eingesetzt. Vorzugsweise umfasst das Gemisch 1-Buten, cis- und trans-2-Buten.

Die erfindungsgemäße Alkoxycarbonylierung wird durch den erfindungsgemäßen Pd-Komplex katalysiert. Der Pd-Komplex kann dabei in Verfahrensschritt b) entweder als präformierter Komplex umfassend Pd und den erfindungsgemäßen Phosphinliganden zugegeben werden, oder *in situ* aus einer Verbindung, welche Pd umfasst, und dem freien Phosphinliganden gebildet werden. Dabei wird die Verbindung, welche Pd umfasst, auch als Katalysatorvorstufe bezeichnet.

In dem Fall, dass der Katalysator *in situ* gebildet wird, kann der Ligand im Überschuss zugegeben werden, so dass im Reaktionsgemisch auch ungebundener Ligand vorliegt.

Auch im Falle des Komplexes, der gleich zu Beginn zugesetzt wird, kann auch weiterer Ligand zugegeben werden, so dass im Reaktionsgemisch auch ungebundener Ligand vorliegt.

In einer Variante ist die Verbindung, welche Pd umfasst, ausgewählt aus Palladiumdichlorid (PdCl₂), Palladium(II)-acetylacetonat [Pd(acac)₂], Palladium(II)-acetat [Pd(OAc)₂], Dichloro(1,5-cyclooctadiene)palladium(II) [Pd(cod)₂Cl₂], Bis(dibenzylideneaceton)palladium [Pd(dba)₂], Bis(acetonitrile)dichloropalladium(II) [Pd(CH₃CN)₂Cl₂], Palladium(cinnamyl)dichlorid [Pd(cinnamyl)Cl₂].

Vorzugsweise handelt es sich bei der Verbindung, welche Pd umfasst, um PdCl₂, Pd(acac)₂ oder Pd(OAc)₂. Besonders geeignet ist PdCl₂.

Der Alkohol im Verfahrensschritt c) kann verzweigt oder linear sein, cyclisch, alicyclisch, teilweise zyklisch oder aliphatisch und stellt insbesondere ein C₁- bis C₃₀-Alkanol dar. Es können Monoalkohole oder Polyalkohole verwendet werden.

Der Alkohol in Verfahrensschritt c) umfasst vorzugsweise 1 bis 30 Kohlenstoffatome, bevorzugt 1 bis 22 Kohlenstoffatome, besonders bevorzugt 1 bis 12 Kohlenstoffatome. Es kann sich dabei um einen Monoalkohol oder einen Polyalkohol handeln.

Der Alkohol kann zusätzlich zu der einen oder mehreren Hydroxylgruppen weitere funktionelle Gruppen enthalten. Vorzugsweise kann der Alkohol zusätzlich eine oder mehrere funktionelle Gruppen ausgewählt aus Carboxyl-, Thiocarboxyl-, Sulfo-, Sulfinyl-, Carbonsäureanhydrid-, Imid-, Carbonsäureester-, Sulfonsäureester-, Carbamoyl-, Sulfamoyl-, Cyano-, Carbonyl-, Carbonothioyl-, Sulfhydryl- , Amino-, Ether-, Thioether-, Aryl-, Heteroaryl- oder Silylgruppen, und/oder Halogensubstituenten enthalten.

In einer Ausführungsform umfasst der Alkohol keine weiteren funktionellen Gruppen außer Hydroxylgruppen.

Der Alkohol kann ungesättigte und aromatische Gruppen enthalten. Vorzugsweise handelt es sich jedoch um einen aliphatischen Alkohol.

Als aliphatischer Alkohol wird im Rahmen dieser Erfindung ein Alkohol bezeichnet, der keine aromatischen Gruppen umfasst, also beispielsweise ein Alkanol, Alkenol oder Alkinol.

In einer Ausführungsform handelt es sich bei dem Alkohol um ein Alkanol mit einer oder mehrerer Hydroxylgruppen und 1 bis 30 Kohlenstoffatomen, bevorzugt 1 bis 22 Kohlenstoffatomen, besonders bevorzugt 1 bis 12 Kohlenstoffatomen, am meisten bevorzugt 1 bis 6 Kohlenstoffatomen.

In einer Variante des Verfahrens ist der Alkohol im Verfahrensschritt c) ausgewählt aus der Gruppe der Monoalkohole.

In einer Variante des Verfahrens ist der Alkohol im Verfahrensschritt c) ausgewählt aus: Methanol, Ethanol, 1-Propanol, Iso-Propanol, Iso-Butanol, tert-Butanol, 1-Butanol, 2-Butanol, 1-Pentanol, 2-Pentanol, 3-Pentanol, 1-Hexanol, Cyclohexanol, Phenol, 2-Ethylhexanol, Isononanol, 2-Propylheptanol.

In einer bevorzugten Variante ist der Alkohol in Verfahrensschritt c) ausgewählt ist aus Methanol, Ethanol, 1-Propanol, 1-Butanol, 1-Pentanol, 1-Hexanol, 2-Propanol, *tert*-Butanol, 3-Pentanol, Cyclohexanol, Phenol, oder Mischungen davon.

In einer Variante des Verfahrens ist der Alkohol im Verfahrensschritt c) ausgewählt aus der Gruppe der Polyalkohole.

In einer Variante des Verfahrens ist der Alkohol im Verfahrensschritt c) ausgewählt aus: Diolen, Triolen, Tetraolen.

In einer Variante des Verfahrens ist der Alkohol im Verfahrensschritt c) ausgewählt aus: Cyclohexan-1,2-diol, 1,2-Ethandiol, 1,3-Propandiol, Glycerol, 1,2,4-Butantriol,2-Hydroxymethyl-1,3-Propandiol, 1,2,6-Trihydroxyhexan, Pentaerythritol, 1,1,1-Tri(hydroxymethyl)ethan, Brenzkatechin, Resorcin und Hydroxyhydrochinon.

In einer Variante des Verfahrens ist der Alkohol im Verfahrensschritt c) ausgewählt aus: Sucrose, Fructose, Mannose, Sorbose, Galactose und Glucose.

In einer bevorzugten Variante des Verfahrens ist der Alkohol im Verfahrensschritt c) ausgewählt aus Methanol, Ethanol, 1-Propanol, 1-Butanol, 1-Pentanol, 1-Hexanol.

In einer besonders bevorzugten Variante des Verfahrens ist der Alkohol im Verfahrensschritt c) ausgewählt aus: Methanol, Ethanol.

In einer besonders bevorzugten Variante des Verfahrens ist der Alkohol im Verfahrensschritt c) Methanol.

In einer Variante des Verfahrens wird der Alkohol im Verfahrensschritt c) im Überschuss eingesetzt.

In einer Variante des Verfahrens wird der Alkohol im Verfahrensschritt c) gleichzeitig als Lösungsmittel eingesetzt.

In einer Variante des Verfahrens wird ein weiteres Lösungsmittel verwendet, ausgewählt aus: Toluol, Xylol, Tetrahydrofuran (THF) oder Methylenchlorid (CH₂Cl₂).

CO wird in Schritt d) vorzugsweise bei einem CO-Partialdruck zwischen 0,1 und 10 MPa (1 bis 100 bar), bevorzugt zwischen 1 und 8 MPa (10 bis 80 bar), besonders bevorzugt zwischen 2 und 4 MPa (20 bis 40 bar) zugeführt.

Die Reaktionsmischung wird Schritt e) des erfindungsgemäßen Verfahrens vorzugsweise auf eine Temperatur zwischen 10 °C und 180 °C, bevorzugt zwischen 20 und 160 °C, besonders bevorzugt zwischen 40 und 120 °C erwärmt, um die ethylenisch ungesättigte Verbindung zu einem Ester umzusetzen.

Das molare Verhältnis von der in Schritt a) vorgelegten ethylenisch ungesättigten Verbindung zu dem in Schritt c) zugegebenen Alkohol beträgt vorzugsweise zwischen 1:1 bis 1:20, bevorzugt 1:2 bis 1:10, besonders bevorzugt 1:3 bis 1:4.

Das Massenverhältnis von Pd zu der in Schritt a) vorgelegten ethylenisch ungesättigten Verbindung beträgt vorzugsweise zwischen 0,001 und 0,5 Gew.-%, bevorzugt zwischen 0,01 und 0,1 Gew.-%, besonders bevorzugt zwischen 0,01 und 0,05 Gew.-%.

Das molare Verhältnis der erfindungsgemäßen Diphosphinverbindung zu Pd beträgt vorzugsweise zwischen 0,1:1 und 400:1, bevorzugt zwischen 0,5:1 und 400:1, besonders bevorzugt zwischen 1:1 und 100:1, am meisten bevorzugt zwischen 2:1 und 50:1.

Vorzugsweise wird das Verfahren unter Zusatz einer Säure durchgeführt. In einer Variante umfasst das Verfahren deshalb zusätzlich den Schritt c'): Zugabe einer Säure zum Reaktionsgemisch. Dabei kann es sich vorzugsweise um eine Brønsted- oder eine Lewis-Säure handeln.

Geeignete Brønsted-Säuren haben vorzugsweise eine Säurestärke von pKₛ ≤ 5, bevorzugt eine Säurestärke von pKₛ ≤ 3. Die angegebene Säurestärke pKₛ bezieht sich auf den bei Normalbedingungen (25°C, 1,01325 bar) bestimmten pKₛ-Wert. Bei einer mehrprotonigen Säure bezieht sich die Säurestärke pKₛ im Rahmen dieser Erfindung auf den pKₛ-Wert des ersten Protolyseschrittes.

Vorzugsweise ist die Säure keine Carbonsäure.

Geeignete Brønsted-Säuren sind beispielsweise Perchlorsäure, Schwefelsäure, Phosphorsäure, Methylphosphonsäure und Sulfonsäuren. Vorzugsweise handelt es sich bei der Säure um Schwefelsäure oder eine Sulfonsäure. Geeignete Sulfonsäuren sind beispielsweise Methansulfonsäure, Trifluormethansulfonsäure, tert-Butansulfonsäure, p-Toluolsulfonsäure (PTSA), 2-Hydroxypropan-2-sulfonsäure, 2,4,6-Trimethylbenzolsulfonsäure und Dodecylsulfonsäure. Besonders bevorzugte Säuren sind Schwefelsäure, Methansulfonsäure, Trifluormethansulfonsäure und p-Toluolsulfonsäure.

Als Lewis-Säure kann beispielsweise Aluminiumtriflat eingesetzt werden.

In einer Ausführungsform beträgt die Menge an in Schritt c') zugesetzter Säure 0,3 bis 40 mol-%, bevorzugt 0,4 bis 15 mol-%, besonders bevorzugt 0,5 bis 5 mol-%, am meisten bevorzugt 0,6 bis 3 mol-%, bezogen auf die Stoffmenge der in Schritt a) eingesetzten ethylenisch ungesättigten Verbindung.

### Beschreibung der Abbildungen

- **Figur 1**: Methoxycarbonylierung von Ethen mit **3** und **8** bei 80 °C und 40 bar CO
- **Figur 2**: Methoxycarbonylierung von Ethen mit **3** und **8** bei 60 °C und 20 bar CO (konstanter Druck)
- **Figur 3**: Alkoholvariation in der Methoxycarbonylierung von Ethen mit Ligand **8** bei 80 °C und 30 bar CO-Druck
- **Figur 4**: Versuche zur Methoxycarbonylierung von Propen, 1-Buten und 2-Buten bei 100 °C und 40 bar mit dem Liganden **8.**
- **Figur 5**: Methoxycarbonylierung von Raffinat 1 mit dem Liganden **8** bei 100 °C und 60 bar CO-Druck.
- **Figur 6**: Methoxycarbonylierung von Raffinat 1 bei 100 °C und 50 bar mit Ligand **8.**
- **Figur 7**: Methoxycarbonylierung einer Mischung von Propen, 1-Buten und 2-Buten bei 100 °C und 60 bar mit dem Liganden **8.**
- **Figur 8**: Methoxycarbonylierung eines Gemischs von C5-Olefinen bei 100 °C und 50 bar CO-Druck mit dem Liganden **8.**
- **Figur 9**: Methoxycarbonylierung von Di-n-Buten mit Ligand **8** bei 120 °C und 20 bar mit konstantem CO-Druck
- **Figur 10**: Methoxycarbonylierung von Di-n-Buten mit **3** und **8** bei 120 °C und 40 bar CO.
- **Figur 11**: Ausbeutekurve für die Methoxycarbonylierung von Di-n-Buten mit **8** als Liganden bei 20 bar konstantem Gesamtdruck und 120 °C.
- **Figur 12**: Gasverbrauchskurven von Reaktionen mit **3** und **8.**

### Beispiele

Im Folgenden wird die Erfindung anhand von Ausführungsbeispielen näher beschrieben.

### Allgemeine Arbeitsvorschriften

Alle nachfolgenden Präparationen wurden mit Standard-Schlenk-Technik unter Schutzgas durchgeführt. Die Lösungsmittel wurden vor Gebrauch über geeigneten Trocknungsmitteln getrocknet (Purification of Laboratory Chemicals, W. L. F. Armarego (Autor), Christina Chai (Autor), Butterworth Heinemann (Elsevier), 6. Auflage, Oxford 2009).
Phosphortrichlorid (Aldrich) wurde vor dem Einsatz unter Argon destilliert. Alle präparativen Arbeiten erfolgten in ausgeheizten Gefäßen. Die Charakterisierung der Produkte erfolgte mittels NMR-Spektroskopie. Chemische Verschiebungen (δ) werden in ppm angegeben. Die Referenzierung der ³¹P-NMR-Signale erfolgte gemäß: SR_{31P} = SR_{1H} * (BF_{31P} / BF_{1H}) = SR_{1H} * 0,4048. (Robin K. Harris, Edwin D. Becker, Sonia M. Cabral de Menezes, Robin Goodfellow, und Pierre Granger, Pure Appl. Chem., 2001, 73, 1795-1818; Robin K. Harris, Edwin D. Becker, Sonia M. Cabral de Menezes, Pierre Granger, Roy E. Hoffman und Kurt W. Zilm, Pure Appl. Chem., 2008, 80, 59-84).
Die Aufnahme von Kernresonanzspektren erfolgte an Bruker Avance 300 bzw. Bruker Avance 400, die gaschromatografische Analyse an Agilent GC 7890A, die Elementaranalyse an Leco TruSpec CHNS und Varian ICP-OES 715, und die ESI-TOF Massenspektrometrie an Thermo Electron Finnigan MAT 95-XP und Agilent 6890 N/5973 Geräten.

### Herstellung von Vorstufe E

### Darstellung von Chlor-2-pyridyl-tert-butylphosphin

Der Grignard für die Synthese von Chlor-2-pyridyl-t-butylphosphin wird nach der "Knochelmethode" mit Isopropylmagnesiumchlorid dargestellt (Angew. Chem. 2004, 43, 2222-2226). Die Aufarbeitung erfolgt nach der Methode von Budzelaar (Organometallics 1990, 9, 1222-1227).

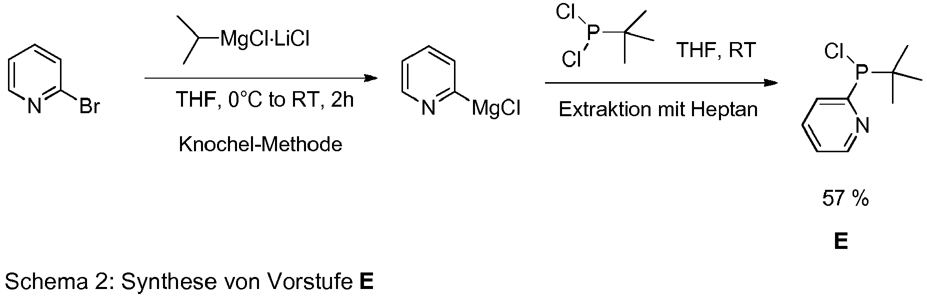

In einen 50 ml Rundkolben mit Magnetrührer und Septum werden unter Argon 8.07 ml einer 1.3 M Isopropylmagnesium-chloridlösung (Knochel-Reagenz) gegeben und auf -15 °C gekühlt. Danach werden zügig 953.5 µl (10 mmol) 2-Bromopyridin zu getropft. Die Lösung wird sofort gelb. Man lässt auf-10 °C aufwärmen. Der Umsatz der Reaktion wird wie folgt bestimmt: man entnimmt ca. 100 µl Lösung und gibt sie in 1 ml einer gesättigten Ammoniumchloridlösung. Wenn die Lösung "sprudelt", dann hat sich noch nicht viel Grignard gebildet. Die wässrige Lösung wird mit einer Pipette Ether extrahiert und die organische Phase über Na₂SO₄ getrocknet. Von der etherischen Lösung wird ein GC aufgenommen. Wenn sich viel Pyridin gebildet hat im Vergleich zu 2-Bromopyridin, dann hat man hohe Umsätze. Bei -10 °C hat sich wenig umgesetzt. Nach Aufwärmen auf Raumtemperatur und 1-2 stündigen Rühren wird die Reaktionslösung braungelb. Ein GC-Test zeigt vollständigen Umsatz. Jetzt kann die Grignardlösung zu einer Lösung von 1.748 g (11 mmol) Dichloro-tert-butylphosphin in 10 ml THF, die vorher auf -15 °C gekühlt worden ist, mit einer Spritzenpumpe langsam zu getropft werden. Wichtig ist, dass die Dichloro-tert-butylphosphinlösung gekühlt wird. Bei Raumtemperatur würde man erhebliche Mengen an Dipyridyl-tert-butylphosphin erhalten. Es entsteht anfangs eine klare gelbe Lösung, die dann trübe wird. Man lässt auf Raumtemperatur aufwärmen und über Nacht Rühren. Man entfernt das Lösungsmittel im Hochvakuum und erhält einen weißlichen Feststoff, der braune Stellen enthält. Der Feststoff wird mit 20 ml Heptan suspendiert und der Feststoff wird im Ultraschallbad zerkleinert. Nach dem Absetzen lassen des weißen Feststoffes wird die Lösung dekantiert. Der Vorgang wird zwei Mal mit je 10-20 ml Heptan wiederholt. Nach dem Einengen der Heptanlösung im Hochvakuum wird diese unter Vakuum destilliert. Bei 4.6 mbar, 120 °C Ölbad und 98 °C Übergangstemperatur kann das Produkt destilliert werden. Man erhält 1.08 g eines farblosen Öls. (50%).
Analytische Daten: ¹H NMR (300 MHz, C₆D₆): δ 8.36 (m, 1H, Py), 7.67 (m, 1H, Py), 7.03-6.93 (m, 1H, Py), 6.55-6.46 (m, 1H, Py), 1.07 (d, J = 13.3 Hz, 9H, t-Bu)
¹³C NMR (75 MHz, C₆D₆): δ 162.9, 162.6, 148.8, 135.5, 125.8, 125.7, 122.8, 35.3, 34.8, 25.9 und 25.8.
³¹P NMR (121 MHz, C₆D₆) δ 97.9.
MS (EI) *m*:*z* (relative Intensität) 201 (M⁺,2), 147(32), 145 (100), 109 (17), 78 (8), 57.1 (17).

### Herstellung von Verbindung 8

### Darstellung von 1,1'-Bis(tert-butyl-2-pyridylphosphino)ferrocen

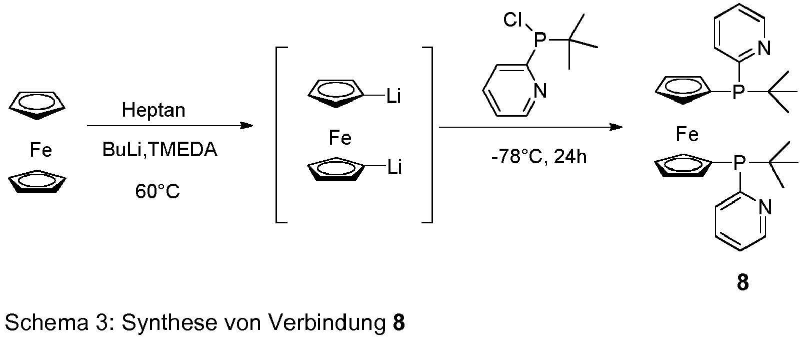

### Variante A:

474.4 mg (2.55 mmol) sublimiertes Ferrocen wird in einen 50 ml Rundkolben mit Magnetrührer und Septum eingewogen und sekuriert. Nach Zugabe von 15 ml Heptan hat sich das Ferrocen vollständig aufgelöst. Dann werden 841 µl Tetramethylethylendiamin (1.1 eq, 5.61 mmol) auf einmal zugegeben und 2.04 ml BuLi (2.5 M in Hexan, 2.0 eq.5.1 mmol) zu getropft. Nach 2-3 Stunden fällt ein oranger Niederschlag aus. Man lässt über Nacht Rühren, dekantiert die Heptanlösung und wäscht den orangen Feststoff zwei Mal mit Heptan. Dann werden erneut 10 ml Heptan zugegeben und die Suspension wird auf -70 °C gekühlt. 1.08 g (2.1 äq, 5.36 mmol) Chlor-2-pyridyl-tert-butylphosphin wird in 7 ml Heptan gelöst. Die Lösung ist trüb und muss über Celite filtriert werden. Etwas unlöslicher weißer Feststoff hat sich gebildet. Diese Lösung wird zur Dilithiumferrocenlösung zu getropft. Während des Aufwärmens auf Raumtemperatur hellt sich die orange Suspension auf. Zur Vervollständigung der Reaktion wird die Reaktionslösung unter Rückfluss erwärmt für ca. 1 Stunde. Es hat sich eine klare orange Lösung und weißer Niederschlag gebildet.
Zur Suspension werden 7 ml mit Argon gesättigtes Wasser gegeben. Der weiße Niederschlag löst sich auf. Nach Entfernen der wässrigen Phase wird der Vorgang zwei Mal wiederholt. Dabei wird die Heptanphase trüb. Nach vollständigem Entfernen der organischen Phase im Hochvakuum, bleibt ein oranger öliger Rückstand zurück. Dieser wird in 10 ml Ether aufgenommen und über Na₂SO₄ getrocknet. (Rohausbeute 913 mg) Bei -28 °C bilden sich über Nacht weder ein Niederschlag noch Kristalle. Auch eine Mischung aus Diethylether und Heptan führt bei -28 °C nicht zur Kristallisation. Ein ³¹P NMR der Lösung zeigt wieder den Produktpeak jetzt bei 7,39 ppm und ein Signal bei 40,4 ppm. Das Produkt kann durch Säulenchromatographie gereinigt werden. Die Etherlösung wird auf eine mit Diethylether eluierte kurze Säule unter Argon aufgetragen. Die orange Produktfront läuft ganz vorne weg und kann leicht aufgefangen werden. Nach Entfernen des Ethers erhält man 241 mg (16 %) eines orangen zähen Öls in ca. 95%iger Reinheit.

### Variante B:

Ansatzgröße: 650.17 mg (3.495 mol) Ferrocen (sublimiert), 2.8 ml (2 eq, 6.99 mmol) 2.5 M BuLi (n-Butyllithium), 1.1 ml (2.1 eq, 7.3 mmol) Tetramethylethylendiamin und 1.48 g (2.1 eq, 7.34 mmol) Chlor-2-pyridyl-tert-butylphosphin.

Das Dilithiumsalz des Ferrocens wird wieder in 15 ml Heptan hergestellt. Das Chlor-2-pyridyl-tert.-butylphosphin wird anstelle von Heptan in 10 ml THF gelöst, weil sich das Chlorphosphin besser in THF löst. Die Aufarbeitung wurde ebenfalls optimiert: Nach dem Kochen unter Rückfluss wird die Reaktionsmischung mit nur 1 ml H₂O gequencht und das Lösemittel (Heptan und THF) vollständig im Hochvakuum entfernt. Der dunkelgelborange zähe Feststoff wird in 8 ml H₂O und 15 ml Diethylether aufgenommen und 1 Minute gerührt. Nach Phasentrennung wird die wässrige Phase via Spritze entfernt und die organische Phase drei Mal mit H₂O gewaschen. Die organische Phase wird über Na₂SO₄ getrocknet und filtriert. 3 Mal mit je 10 ml Diethylether wird das Produkt aus den Na₂SO₄ herausgewaschen, bis die Lösung fast farblos ist. Die dunkelorange Lösung wird auf 10 ml Volumen eingeengt und über eine Säule mit Kieselgel 60 unter Argon geschickt. Als Laufmittel wird wieder Diethylether verwendet. Das Filtrat ist wesentlich heller und orange. Nach Entfernung des Lösungsmittels erhält man 1.16 g eines zähen orangen Feststoffes. (64%)

### Herstellung von Verbindung 10 (Vergleichsverbindung)

Ausgehend von 1,1'-(Ferrocenediyl)phenylphosphin wird der gespannte Phosphanring mit PhLi geöffnet und die resultierende Zwischenstufe mit einem Chlorphosphin gequencht.

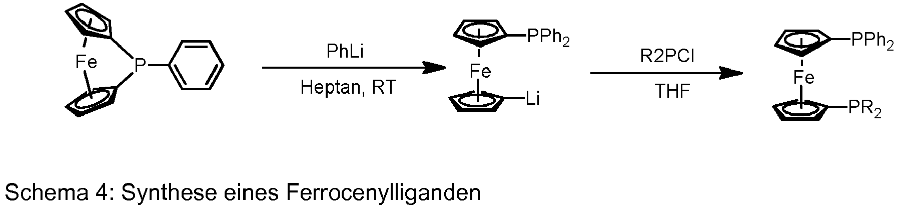

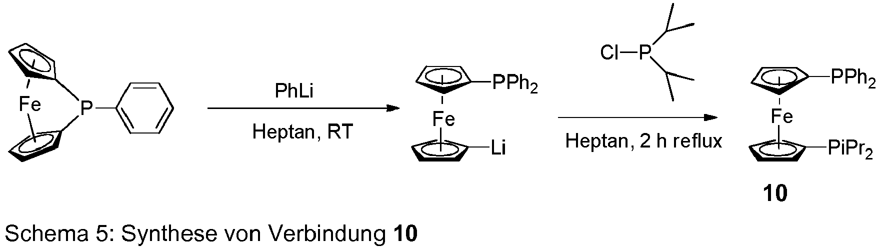

In einen 50 mL Rundkolben mit Magnetrührkern und Stickstoffansatz werden 1,13 mmol (565 µl) Phenyllithium (PhLi) vorgelegt und eine Lösung aus 1,03 mmol (300mg) cyclischen Phosphan in 20 ml Heptan langsam via Spritzenpumpe zugetropft. Das Li-Salz wird zweimal mit Heptan gewaschen und mit 6 ml Heptan versetzt. Zur Suspension wird eine Heptanlösung von 0,8 eq (0,824 mmol, 131 µl) CIP*i*Pr₂ in 7 ml Heptan bei Raumtemperatur zugetropft. Die rotbraune Suspension verfärbt sich kaum. Nach 20 min Rühren wird die Suspension unter Rückfluss 1,5 Stunden erwärmt. Der Feststoff hellt sich etwas auf. Lösungsmittel wird vollständig entfernt und der braun-rote Rückstand wird in H₂O und Ether aufgenommen. Die org. Phase wird zweimal mit H₂O gewaschen und über Na₂SO₄ getrocknet. Von der Etherphase wird ein ³¹P-Spektrum aufgenommen. Das Spektrum zeigt 2 Singuletts. Das Chlorphosphin ist vollständig verbraucht worden. Die Etherphase wird zur Trockene gebracht und man erhält 300 mg (Ausbeute: 61%) eines braungelben Öls, das sich in MeOH auf dem Wasserbad bei 65 °C gelöst. Die Lösung wird über Nacht in den Tiefkühler (-78 °C) gestellt. Es fallen 76 mg eines braungelben Öles aus, das NMR-spektroskopisch untersucht wird.
¹H NMR (300 MHz, CDCl₃) δ 7.46-7.23 (m, 10H, Ph), 4.36 (m, 2H, Cp), 4.21 (m, 2H, Cp), 34.24 (m, 4H, Cp), 1.88 (m, 2H, iPr), 1.15-0.96 (m, 12H, iPr).
¹³C NMR (75 MHz, CDCl₃) δ 139.9 (J = 9.8 Hz, Ph), 133.4 (J = 19.2 Hz, Ph), 128.4, 128.1, 128.0 (Ph), 77.1, 76.8, 76.2, 76.1 (Cp), 73.5 (J = 14.5 Hz, Cp), 72.8 (J = 2.9 Hz, Cp), 71.9 (J = 10.5 Hz, Cp),72.1 (Cp), 23.3 (J = 11.0 Hz, iPr), 20.1, 20.0, 19.9, 19.8 (iPr).
³¹P NMR (121 MHz, C₆D₆) δ = 0.88 und -16.62

### Herstellung von Verbindung 14

### Darstellung von Bis(2-pyridyl-n-butylphosphino)-ferrocen

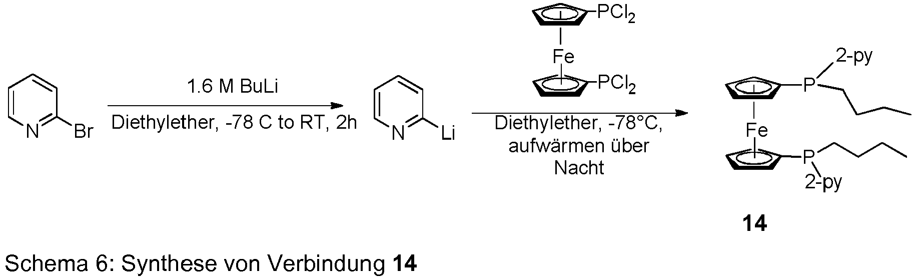

In einem 25 ml Rundkolben mit Magnetrührkern und Hahn werden 1.45 ml (2. 33 mmol) 1.6 M BuLi auf -78 °C (Trockeneis/EtOH) gekühlt. Dazu werden 208 µl (2.18 mmol) 2-Brompyridin gelöst in 2 mL Ether zugetropft. Die Reaktionslösung wird anfangs gelb, verfärbt sich dann zu orange, bleibt aber klar. Nach 15 Minuten Rühren wird ein Probe (100 µL) entnommen und mit NH₄Cl/H₂O gequencht. Laut GC haben sich neben Pyridin noch zahlreiche andere Verbindungen gebildet. Dann werden bei dieser Temperatur 1,1'-Bis(dichlorophosphin)ferrocen gelöst in 2 ml Ether zugetropft und die Reaktionsmischung läßt man über Nacht aufwärmen. Es hat sich eine hellorange Suspension gebildet, die über eine Fritte (G4) filtriert wird. Man erhält eine klare orange Etherlösung. Nach dem Abziehen des Lösungsmittels im Vakuum bekommt man 173 mg eines orangen Feststoffes, der unter Argon chromatographiert wird. Zuerst wird mit reinem Diethylether gesäult (Säulenparameter: 4 cm Durchmesser, Kieselgel 60) und man erhält 50 mg eines zähen gelben Feststoffes. Der Feststoff wird nochmals mit 2:1 Heptan /Diethylether gesäult und man erhält 31 mg von Bis(2-pyridyl-n-butylphosphino)ferrocen (18%).
¹H NMR (300 MHz, C₆D₆): δ 8,54 (d, *J* = 4,6 Hz, 2H, py), 7,43-7,32 (m, 2H, py), 6,94-6,88 (m, 2H, py), 6,58-6,49 (m, 2H, py), 4,47 (m, 1H, ferrocenyl), 4,37(m, 1H, ferrocenyl), 4,33(m, 1H, ferrocenyl), 4,23-4,14 (m, 5H, ferrocenyl), 2,56-2,44 (m,2H, CH₂), 2,23 (m, 2H, CH₂), 1,80-1,65 (m, 4H, CH₂), 1,57-1,39 (m, 4H, CH₂), 0,93-0,85 (m, 6H, CH₃).
¹³C NMR (75 MHz, C₆D₆): δ 166,5, 166,2, 166,1, 150,1, 134,8 und 122,1 (py), 78,7, 78,6, 78,5, 74,9, 74,7, 74,3, 74,1, 72,8, 72,6, 72,1 und 71,7 (ferrocenyl), 29,7, 29,6, 29,5, 29,4, 28,2, 28,1, 27,9, 27,8, 24,8, 24,7, 24,6 und 14,1 (CH₂), 14,1 (CH₃).
³¹P NMR (121 MHz, C₆D₆) δ -24,7 und -24,9.
HRMS (ESI) m/z⁺ berechnet für C₂₈H₃₄FeN₂P₂ (M+H)⁺ 517,16197; gefunden: 517,16238.

### Herstellung von Verbindung 15

### Darstellung von Bis(2-pyridyl-n-butylphosphino)-ferrocen

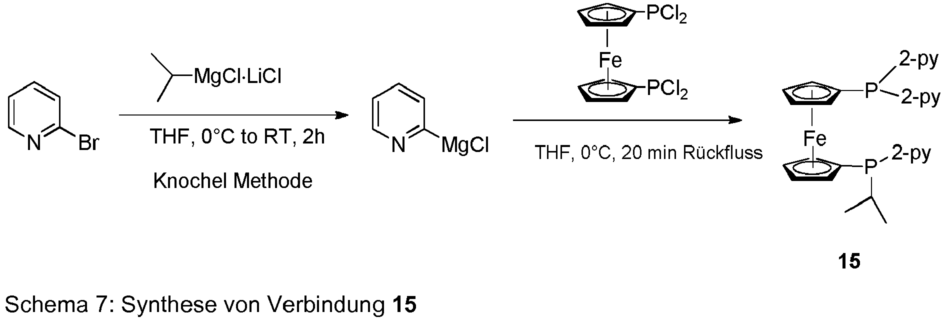

In einen 25 ml-Rundkolben mit Magnetrührer werden 5,3 ml (1,1 eq) einer 1,3 M Isopropylmagnesium-chloridlösung (Knochel Reagenz) auf -20 °C gekühlt und auf einmal 603 µl (6,32 mmol) 2-Brompyridin zugegeben. Es wird eine Stunde bei -20 °C und danach 2 Stunden bei Raumtemperatur gerührt, um vollständigen Umsatz zu erreichen. In einem zweiten 50 ml-Rundkolben werden in der Glovebox 490,7 mg (1,26 mmol) 1,1'-Bis(dichlorphosphino)ferrocen eingewogen und nach dem Ausschleusen in 10 ml THF gelöst. Nach Abkühlen auf -20 °C wird in die orange-gelbe Lösung der zuvor hergestellten Grignard-Verbindung mittels Spritzenpumpe zugetropft. Nach dem Zutropfen hat sich die Lösung auf 0 °C erwärmt und eine braun schwarze Lösung hat sich gebildet. Zur Vervollständigung der Reaktion wird noch 20 Minuten unter Rückfluss erhitzt. Am nächsten Tag wird zur schwarzen Reaktionslösung 0,5 ml Wasser zugegeben, wobei sich die Lösung zu einer dunkelrotbraunen Suspension aufhellt. Das Lösungsmittel wird im Hochvakuum abgezogen und der Rückstand in 15 ml Ether und 10 ml H₂O aufgenommen. Die Suspension wird über Celite filtriert und man erhält eine orange organische Phase und eine grüne wässrige Phase. Die organische Phase wird über Na₂SO₄ getrocknet und nach Abziehen des Ethers erhält man 410 mg eines grünschwarzen Feststoffes. Der dunkelgrüne fast schwarze Feststoff wird in reinem Diethylether gesäult. Nach Entfernen des Ethers bekommt man 112 mg des gelben Produktes **15.**
¹H NMR (300 MHz, C₆D₆): δ 8,56 (m, 1H, py), 8,48-8,38 (m, 2H, py), 7,58 (m, 1H, py), 7,39-7,27 (m, 2H, py), 7,00-6,84 (m, 3H, py), 6,65-6,56 (m, 1H, py), 6,55-6,44 (m, 2H, py), 4,50-4,39 (m, 3H, ferrocenyl), 4,26-4,18 (m, 2H, ferrocenyl), 4,18-4,12(m, 1H, ferrocenyl), 4,12-4,04 (m, 2H, ferrocenyl), 2,69 (oct, *J* = 7,0Hz, 1H *i*pr), 1,14-0,94 (m, 6H, *i*pr).
¹³C NMR (75 MHz, C₆D₆): δ 165,4, 163,7, 150,2, 150,0, 149,9, 134,9, 134,8, 134,7, 131,1, 130,6, 129,1, 128,8, 128,6, 122,7, 122,2, und 122,0 (py), 77,5, 77,3, 76,9, 76,5, 75,4, 75,2, 74,8, 74,6, 74,4, 72,8, 72,7, 72,5, 72,0 und 71,9 (ferrocenyl), 32,2, 28,3, 28,2, 23,0, 20,6, 20,3, 19,7, 19,5 und 14,3 (*i*pr).
³¹P NMR (121 MHz, C₆D₆) δ -6,2 und -12,9.
HRMS (ESI) m/z⁺ berechnet für C₂₈H₂₇FeN₃P₂ (M+H)⁺ 524,11027; gefunden: 524,11022.

### Herstellung von Verbindung 19 (Vergleichsverbindung)

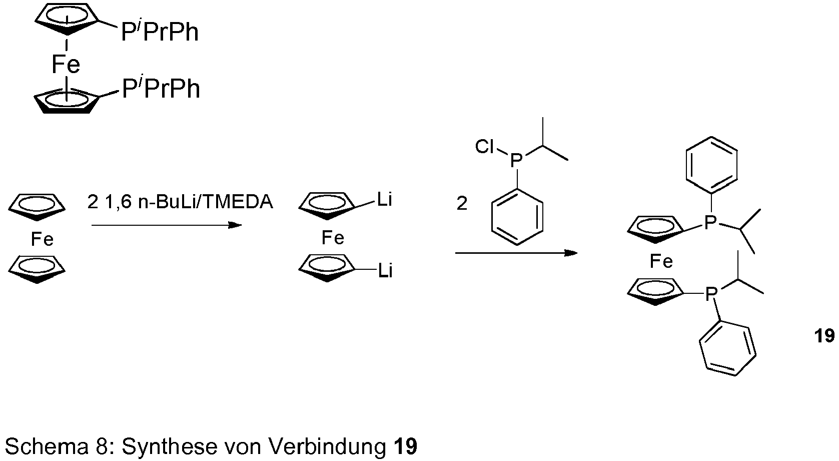

0,93 g Ferrocen werden in 50 ml absolutem Heptan in einem 100 ml Dreihalskolben, versehen mit einem Thermometer, Magnetrührer und Rückflusskühler, gelöst. Es werden 1,3 g TMEDA (1,6 ml) und 7,5 ml 1,6 n-BuLi/Hexan bei Raumtemperatur mittels Spritzen zugesetzt. Die Lösung wird 5 Stunden stehen gelassen. Es fallen große orangebraune Kristalle des dilithiierten Ferrocens aus. Die überstehende Lösung wird mittels einer Spritze entfernt. Und es werden 20 ml absolutes Heptan zugesetzt. Anschließend wird das Chlorphosphin gelöst in 10 ml Heptan zugetropft. Es wird ein Stunde unter Rückfluss erwärmt. Nach dem Abkühlen wird Die organische Phase dreimal mit jeweils 10 ml entgastem Wasser gewaschen. Es wird ins Trockene eingeengt und es werden 10 ml Diethylether zugesetzt. Diese Lösung wird mit Diethylether als Lösungsmittel durch 10 cm Kieselgel 60 unter Argon filtriert, eingeengt ins Trockene und aus wenig heißem Methanol kristallisiert das Zielprodukt in ca. 50 %-iger nicht optimierter Ausbeute.

### Analytik:

³¹P (121MHz, CDCl₃), - 7,8 s, - 8,15 s,
¹³C (75 MHz, CDCl₃);137,77, (d, J = 12 Hz), 137,4 (d, J = 11,3 Hz), 134,2 (d, J = 20,3 Hz), 129,1 s, 128,1 (d, J = 7,5 Hz), 77,4 (d, J = 11,3 Hz), 75,0 (d, J = 26,2 Hz), 74,0 (d, J = 22,3 Hz), 72,1 bs, 71,9-71,5 m, 71,1 s, 69,0 s, 27,6 (d, J = 10 Hz), 27,55 8d, J = 10 Hz), 20,3-19,9 m
¹H (300 MHz, CDCl₃): 7,52-7,44 (m, 4H), 7,33-7,23 (m, 6H), 4,23 (sept, J = 1,2 Hz, 1H), 4,1-4,0 (m, 4 H), 3,93-3,9 (m, 1H), 3,87-3,84 (m, 1H), 3,58-3,54 (m, 1H),
2,1-1,9 (m, 2 H), 0,99 (d, J = 7 Hz, 3H), 0,94 (d, J = 7 Hz, 3 H), 0,83-0.7 (m, 6H)

### Darstellung von Palladiumkomplexen

### Versuch 52: Herstellung von Komplex K4

### Darstellung von [Pd(Cp₂Fe)1,1'-(P(2-pyridyl)(t-butyl))₂]η²-N-Methylmaleinimid] K4

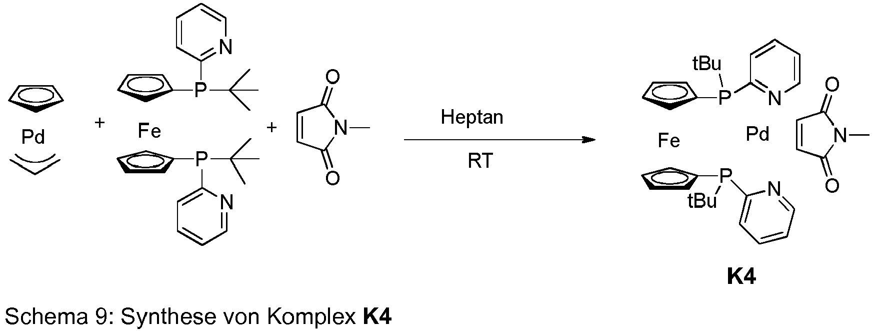

172.9 mg (0.816 mmol) Palladiumprecursor (siehe Schema 9) und 90.64 mg (0.816 mmol) sublimiertes N-Methyl-maleinimid (siehe Versuch 51) werden jeweils in ein 50 ml Schlenkgefäß in der Glovebox eingewogen. 446.6 mg (0.866mmol) des zähen orangen Ferrocenliganden **8** werden in 15 ml Heptane gelöst und zu dem N-Methylmaleinimid gegeben. Die Lösung wird auf 60 °C auf dem Wasserbad erwärmt, bis sich alles gelöst hat. Um eine klare orange Lösung zu erhalten, wird die Lösung über Celite filtriert. Ebenso wird der Palladiumprecursor in 10 ml Heptan gelöst und über Celite filtriert. Bei Raumtemperatur wird die klare orange Ligand/N-Methylmaleinimid Lösung zu dem tiefroten Palladiumprecursor zu getropft. Die dunkelrote Lösung hellt sich auf und ein hellgelber Feststoff fällt aus. Man lässt über Nacht Rühren und dekantiert die überstehende Lösung nach Absetzen des Feststoffes.

Nach zwei Mal Waschen mit Heptan wird der Feststoff im Hochvakuum getrocknet und man erhält 541 mg (86%) Produkt.
Elementaranalyse berechnet für: C₃₃H₃₉FeN₃O₂P₂Pd: C, 54,01; H, 5,36; N, 5,73; P, 8,44, Gefunden: C, 53,44; H, 5,48; N, 5,72; P,8,48.

### Hochdruckexperimente

### Einsatzstoffe:

### Methanol (MeOH)

### Ethen (auch als Ethylen bezeichnet)

Crack-C4 bezeichnet einen Nebenproduktstrom des sogenannten Steam-Cracking-Prozesses zur Ethylen-Herstellung und besteht in der Regel zu über 95% aus einer Mischung verschiedener, verzweigter und linearer Kohlenwasserstoffe, die vier Kohlenstoffatome enthalten und gesättigt, einfach ungesättigt oder mehrfach ungesättigt sein können. Die Hauptkomponenten eines Crack-C4-Stromes sind n-Butan, Isobutan, Isobuten, n-Butene, Butadiene.

Raffinat 1 entsteht aus Crack-C4 nach (in der Regel extraktiver) Abtrennung der Butadiene. Raffinat 1 setzt sich zusammen aus ungefähr 42 % Isobuten, 26 % 1-Buten, 17 % cis- und trans-2- Buten sowie 0,3 % 1,3-Butadien und 15 % n-Butan und Isobutan. Die exakte Zusammensetzung kann je nach Quelle und auch saisonal schwanken. Die angegebenen Werte sind daher nur typische aber nicht limitierende Beispiele.

Das Raffinat II ist ein Teil der beim Naphthacracken entstehenden C₄-Fraktion und besteht im Wesentlichen aus den Isomeren *n*-Butenen, Isobutan und *n*-Butan nach Abtrennung von Isobuten aus Raffinat 1.

Raffinat III ist ein Teil der beim Naphthacracken entstehenden C₄-Fraktion und besteht im Wesentlichen aus den Isomeren *n*-Butenen und *n*-Butan.

2-Buten 99+%, Mischung von *cis* und *trans,* Sigma Aldrich, Katalognummer 36,335-9, LOT No. 14205MS

Das eingesetzte Isobuten hat eine Reinheit von min. 99,9 % (m/m). Hersteller ist die Evonik Industries AG, Performance Materials.

### Di-n-Buten wurde auch wie folgt bezeichnet: Dibuten, DNB oder DnB

Di-n-Buten ist ein Isomerengemisch von C8-Olefinen, das aus der Dimerisierung von Gemischen aus 1-Buten, cis-2-Buten und trans-2-Buten entsteht. Technisch werden der Regel Raffinat II oder Raffinat III-Ströme einer katalytischen Oligomerisierung unterworfen, wobei die enthaltenen Butane (n/iso) unverändert hervorgehen und die enthaltenen Olefine ganz oder Teilweise umgesetzt werden. Neben dem dimeren Di-n-Buten, entstehen in der Regel auch höhere Oligomere (Tributen C12, Tetrabuten C16), die nach der Reaktion destillativ abgetrennt werden.

Ein industriell praktiziertes Verfahren zur Oligomerisierung von C4-Olefinen ist der sogenannte "OCTOL-Prozess".
Innerhalb der Patentliteratur beschreibt beispielsweise DE102008007081A1 eine auf dem OCTOL-Prozess beruhende Oligomerisierung. EP1029839A1 befasst sich mit der Fraktionierung der in dem OCTOL-Prozess entstehenden C8-Olefine.
Technisches Di-n-Buten besteht in der Regel 5 bis 30 % aus n-Octenen, 45 bis 75 % aus 3-Methylheptenen und zu 10 bis 35 % aus 3,4-Dimethylhexenen. Bevorzuge Ströme enthalten 10 bis 20 % n-Octene, 55 bis 65 % aus 3-Methylheptene und 15 bis 25 % 3,4-Dimethylhexenen.

Para-Toluolsulfonsäure wurde wie folgt abgekürzt: pTSA, PTSA oder p-TSA.
PTSA bezeichnet in diesem Text immer das para-Toluolsulfonsäure-Monohydrat.

### Allgemeine Vorschrift zur Durchführung der Hochdruckexperimente:

### Allgemeine Versuchsbeschreibung für Reaktionen im Batchversuch:

Die entsprechenden Mengen an Substrat, Palladiumsalz, Säure und Alkohol werden unter Argon und Magnetrührung in einem 50 ml Schlenkgefäß vermischt.
Ein 100 ml Stahlautoklav der Firma Parr versehen mit einem Gaseinlass und einem Gasauslassventil, einem digitalem Druckaufnehmer, einem Temperaturfühler und einem

Kugelhahn sowie einer eingebauten Kapillare zur Probennahme wird dreimal mittels Vakuum und Argonspülung von Sauerstoff befreit. Anschließend wird die Reaktionslösung aus dem Schlenkgefäß mittels einer Kapillare in den Autoklaven im Argongegenstrom durch den Kugelhahn befüllt. Anschließend wird entweder bei Raumtemperatur die entsprechende Menge an CO aufgepresst und dann auf Reaktionstemperatur hochgeheizt (Reaktionen, die nicht unter konstantem Druck gefahren werden) oder es wird erst auf Reaktionstemperatur hochgeheizt und dann wird das CO über eine Bürette, die mittels eines Druckminderers mit dem Autoklaven verbunden ist, aufgepresst. Diese Bürette wird dann noch auf ca. 100 bar mit CO befüllt und liefert während der Reaktion das benötigte CO bei einem konstanten Druck nach. Diese Bürette hat ein Totvolumen von ca. 30 ml und ist mit einem digitalen Druckaufnehmer versehen. Dann wird die Reaktion bei der benötigten Temperatur die entsprechende Zeit unter Rührung durchgeführt. Hierbei werden mittels einer Software (Specview der Firma SpecView Corporation) und einem Prozesscontroller der Firma Parr 4870 sowie einem 4875 Powercontroler Daten über den Druckverlauf im Autoklaven und in der Gasbürette aufgezeichnet. Aus diesen werden Exceltabellen generiert, aus denen später Diagramme erstellt werden, die Gasverbräuche und damit Umsätze über die Zeit dargestellt. Falls benötigt werden über die Kapillare über die GC Proben gesammelt und analysiert. Hierzu wird vor der Reaktion eine geeignete exakte Menge (2-10 ml) Isooctan als interner Standard mit in das Schlenkgefäß gegeben. Diese geben auch Auskunft über den Reaktionsverlauf. Am Ende der Reaktion wird der Autoklav auf Raumtemperatur heruntergekühlt, der Druck vorsichtig abgelassen, falls nötig Isooctan als interner Standard hinzugefügt und eine GC-Analyse bzw. bei neuen Produkten auch eine GC MS Analyse durchgeführt.

### Allgemeine Versuchsvorschrift für Autoklavenversuche in Glasvials:

Es wird ein 300ml Reaktor der Fa. Parr verwendet. Diesem angepasst ist ein selbstgefertigter Aluminiumblock entsprechender Dimension welcher zu Heizen mittels handelsüblicher Magnetrührer z.B. der Firma Heidolph geeignet ist. Für das Innere des Autoklaven wurde eine runde Metallplatte der Stärke von ca. 1,5 cm angefertigt die 6 Bohrungen enthält, die dem Außendurchmesser der Glasvials entsprechen. Diesen Glasvials angepasst werden sie mit kleinen Magnetrührern bestückt. Diese Glasvials werden mit Schraubkappen und geeigneten Septen versehen und mit einer in der Glasbläserei angefertigten Spezialapparatur unter Argon mit den entsprechenden Reaktanden, Lösungsmittel und Katalysatoren und Additiven befüllt. Hierzu werden 6 Gefäße gleichzeitig befüllt, dies ermöglicht die Durchführung von 6 Reaktionen bei gleicher Temperatur und gleichem Druck in einem Experiment. Dann werden diese Glasgefäße mit Schraubkappen und Septen geschlossen und es wird jeweils ein kleine Spritzenkanüle geeigneter Größe durch die Septen gestochen. Dies ermöglicht später in der Reaktion den Gasaustausch. Diese Vials werden nun in der Metallplatte platziert und diese wird unter Argon in den Autoklaven überführt. Der Autoklav wird mit CO gespült und bei Raumtemperatur mit dem vorgesehenen CO-Druck befüllt. Dann wird mittels dem Magnetrührer unter Magnetrührung auf Reaktionstemperatur erhitzt und die Reaktion die entsprechende Zeit durchgeführt. Anschließend wird auf Raumtemperatur heruntergekühlt und der Druck langsam abgelassen. Anschließend wird der Autoklav mit Stickstoff gespült. Die Vials werden dem Autoklaven entnommen und mit einer definierten Menge eines geeigneten Standards versetzt. Es erfolgt eine GC Analyse mit dessen Ergebnisse Ausbeuten und Selektivitäten bestimmt wurden.

### Allgemeine Vorschrift für Versuche in den 12fach-Autoklaven (600 ml Parr-Autoklav):

In ausgeheizte Glasvials werden jeweils Di-n-Buten (DBN) und Methanol vorgelegt, mit einer Lösung aus Pd(acac)₂ (0,5 mg, 0,0016 mmol) und Ligand (0,0064 mmol) in 0,2 ml Methanol versetzt und H₂SO₄ (Lösung: 1 ml H₂SO₄ in 50 ml MeOH) zugegeben. Im Autoklaven werden die Ansätze 2-mal mit 10 bar CO gespült, mit dem gewünschten Druck CO beladen und bei der gewünschten Temperatur 20 h gerührt. Nach Beendigung der Reaktion werden jeweils Isooctan (interner Standard) und 1 ml EtOAc zugegeben. Die organische Phase wird per GC analysiert.
Die Ausbeuten der Reaktionen werden mittels GC (Isooctan als interner Standard) bestimmt.

### Analytik:

GC Analytik der Produkte aus Ethen: Für die GC Analyse wird ein Chromatograph der Firma Agilent GC Agilent 7890A mit einer 30 m HP Säule verwendet. Temperaturprofil: 35 °C, 10 min; 10 °C/min zu 200 °C, 16.5 min; das Injektionsvolumen beträgt 1 µl mit einem Split von 50:1. Retentionszeit von Methylpropionat : 6.158 min

### GC-Analytik der Produkte aus 2-Buten:

Für die GC Analyse wird ein Chromatograph der Firma Agilent GC Agilent 7890A mit einer 30 m HP Säule verwendet. Temperaturprofil: 35 °C, 10 min; 10 °C/min zu 200 °C, 16.5 min; das Injektionsvolumen beträgt 1 µl mit einem Split von 50:1.
Retentionszeit für iso-C5 Ester: 12.118 min
Retentionszeit für n-C5 Ester: 13.807 min

GC Analytik der Produkte aus Raffinat 1: Für die GC Analyse wird ein Chromatograph der Firma Agilent GC Agilent 7890A mit einer 30 m HP Säule verwendet. Temperaturprofil: 35 °C, 10 min; 10 °C/min zu 200 °C, 16.5 min; das Injektionsvolumen beträgt 1 µl mit einem Split von 50:1.
Retentionszeit für MTBE: 5.067 min
Retentionszeit für iso-C5 Ester: 12.118 min
Retentionszeit für n-C5 Ester: 13.807 min

GC Analytik der Produkte aus Crack-C4: Agilent 7890A Chromatograph mit einer 30 m HP5 Säule; Temperaturprofil: 35 °C, 10min; 10 °C/min to 200 °C, 16.5 min; Das Injektionsvolunmen beträgt 1 µl mit einem Split von 50:1.
Retentionszeit für Methyl pentanoat: 13.842 min
Retentionszeit für Methyl pent-3-enoat: 14.344 min, 14.533 min
Retentionszeit für Dimethyl adipat: 21.404 min

### GC-Analytik der Produkte aus Isobuten:

Agilent 7890A Chromatograph mit einer 30 m HP5Säule; Temperaturprofil: 35 °C, 10min; 10 °C/min zu 200 °C, 16.5 min; Das Injektionsvolumen beträgt 1 µl mit einem Split von 50:1.
Retentionszeit für MTBE: 5.045 min
Retentionszeit für C5 Ester: 12.105 min

GC Analytik der Produkte aus Tetramethylethen: Für die GC Analyse wird ein Chromatograph der Firma Agilent GC Agilent 7890A mit einer 30 m HP Säule verwendet. Temperaturprofil: 35 °C, 10 min; 10 °C/min zu 200 °C, 16.5 min; das Injektionsvolumen beträgt 1 µl mit einem Split von 50:1.
Retentionszeit für Tetramethylethylen und Produkte: 7.436 min
Retentionszeit für den Ether: 11.391 min
Retentionszeit für Methyl 3,4-dimethylpentanoat: 17.269 min

GC Analytik C-5-Gemisch und Produkte: Für die GC Analyse wird ein Chromatograph der Firma Agilent GC Agilent 7890A mit einer 30 m HP Säule verwendet. Temperaturprofil: 35 °C, 10 min; 10 °C/min zu 200 °C, 16.5 min; das Injektionsvolumen beträgt 1 µl mit einem Split von 50:1.
Retentionszeiten für die C5-Olefine: 4.498, 4.437, 4.533, 4.533, 5.465, 5.793 min; Retentionszeiten für die C6-Methylester und deren Isomere: 14.547-16.362 min
(Hauptpeak:16.362 min)

GC Analytik Di-n-Buten: Für die GC Analyse wird ein Chromatograph der Firma Agilent GC Agilent 7890A mit einer 30 m HP5-Säule verwendet. Temperaturprofil: 35 °C, 10 min; 10 °C/min zu 200 °C; das Injektionsvolumen beträgt 1 µl mit einem Split von 50:1.
Retentionszeiten für Di-n-Buten und Produkte: 10.784-13.502 min
Die aus dem Di-n-Buten gebildeten Ester werden im Folgenden als MINO bezeichnet (Methylisononanoat).
Retentionszeit für Etherprodukte unbekannter Isomerenverteilung: 15.312, 17.042, 17.244, 17.417 min
Retentionszeit für iso-C9-Ester 19.502-20.439 min (Hauptpeak: 19.990 min)
Retentionszeit für n-C9-Ester: 20.669, 20.730, 20.884, 21.266 min.

GC Analytik der Produkte aus 1,3-Butadien: Für die GC Analyse wird ein Chromatograph der Firma Agilent GC Agilent 7890A mit einer 30 m HP Säule verwendet. Temperaturprofil: 35 °C, 10 min; 10 °C/min zu 200 °C, 16.5 min; das Injektionsvolumen beträgt 1 µl mit einem Split von 50:1. Retentionszeit für Methyl-pent-3-enoat: 14.430 min, Retentionszeit für Dimethyladipat: 21.404 min.

GC Analytik für Methyl-tert-butylether (MTBE) und Produkte: Agilent 7890A Chromatograph mit einer 30 m- HP5-Säule; Temperaturprofil: 35 °C, 10min; 10 °C/min zu 200 °C, 16.5 min; Das Injektionsvolumen beträgt 1 µl mit einem Split von 50:1.
Retentionszeit Methyl 3-methylbutanoat: 12.070 min
Retentionszeit MTBE: 5.067 min

GC-Analytik für aromatische Alkohole und Produkte: Agilent 7890A Chromatograph mit einer 30 m HP5-Säule; Temperaturprofil: 35 °C, 10min; 10 °C/min zu 200 °C, 16,5 min; Das Injektionsvolumen beträgt 1 µl mit einem Split von 50:1.
Retentionszeit: 21.197 min. 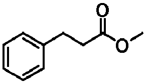 Retentionszeit: 21.988 min.

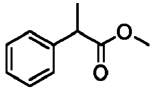

GC-Analytik für sekundäre Alkohole und Produkte: Agilent 7890A Chromatograph mit einer 30 m HP5Säule; Temperaturprofil: 35 °C, 10min; 10 °C/min zu 200 °C, 16,5 min; Das Injektionsvolumen beträgt 1 µl mit einem Split von 50:1.
Retentionszeit für 3,3-Dimethylbutan-2-ol: 10,975
Retentionszeit für Methyl-2,3,3-trimethylbutanoat: 15.312 min,
Retentionszeit Methyl-4,4-dimethylpentanoat: 17.482 min.

GC Analytik für tert.-Butanol und Produkte: Agilent 7890A Chromatograph mit einer 30 m HP5Säule; Temperaturprofil: 35 °C, 10min; 10°C/min bis 200 °C, 16.5 min; Das Injektionsvolumen beträgt 1 µl mit einem Split von 50:1.
Retentionszeit tert-Butanol: 4,631
Retentionszeit Methyl-3-methylbutanoat: 12.063 min.

### GC-Analytik für Methyloleat und Produkte:

Für die GC Analyse wird ein Chromatograph der Firma Agilent GC Agilent 7890A mit einer 30 m HP Säule verwendet. Temperaturprofil: 50 °C, 0 min; 8 °C/min zu 260 °C, 15 min; das Injektionsvolumen beträgt 1 µL mit einem Split von 50:1. Retentionszeit für Methyloleat: 23,823 min, Retentionszeit für 1,19 Dimethyl nonadecanedioat: 28,807 min, Retentionszeit für 1,X Dimethyl nonadecanedioat: 27.058 min Hauptpeak, 27,058, min, 27,206 min, 27,906 min, 28,831 min (Nebenpeaks). Die Position X ist analytisch unbestimmt.

### Analytik Methanol

Methanol wurde in einer Lösungsmitteltrocknungsanlage vorbehandelt: Pure Solv MD-/ Lösungsmittel Purification System, Firma Innovative Technology Inc. One Industrial Way, Amesbury MA 01013

### Wasserwerte:

Bestimmt mit Karl Fischer Tritration: TitraLab 580-TIM580, Firma Radiometer Analytical SAS (Karl- Fischer Titration), Wassergehalt: Messbereiche, 0,1-100% w/w, gemessener Wassergehalt: 0,13889%

### Eingesetzt wurden:

Technisches Methanol Applichem: Nr. A2954,5000, Chargennummer: LOT: 3L005446 Wassergehalt max. 1 %
Methanol Acros Organics (über Molsieb): Wassergehalt 0,005 %, Codenummer: 364390010, Chargennummer: LOT 1370321
TON: Turnovernumber, definiert als mol Produkt pro mol Katalysatormetall
TOF: Turnoverfrequenz, definiert als TON pro Zeit für das Erreichen eines bestimmten Umsatzes, z.B. 50 %

Das n/iso-Verhältnis gibt das Verhältnis von endständig zu Estern umgesetzten Olefinen zu innenständig zu Estern umgesetzten Olefinen an.

Die im Folgenden angegebenen n-Selektivitäten beziehen sich auf den Anteil der endständigen Methoxycarbonylierung bezogen auf die Gesamtausbeute an Methoxycarbonylierungsprodukten.

### Methoxycarbonylierung von Ethen mit Liganden 3 und 8 bei 80 °C und 40 bar

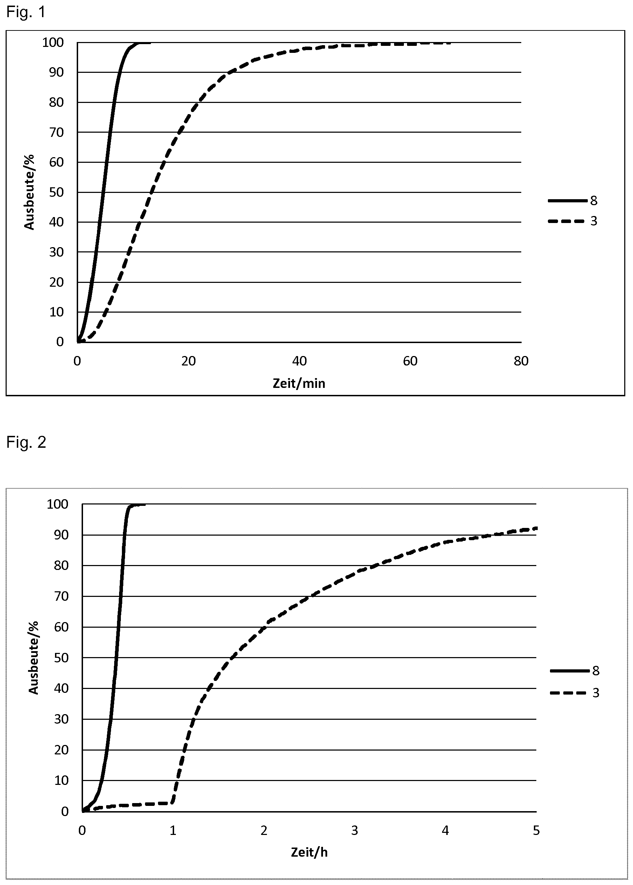

Der Ligand 8 wurde im Vergleich mit dem DTBPMB-Liganden 3 bei 80 °C und 40 bar CO getestet. Die Ergebnisse sind in **Figur 1** dargestellt (**Figur 1****:** Methoxycarbonylierung von Ethen mit **3** und **8** bei 80 °C und 40 bar CO).

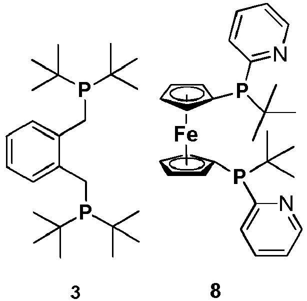

Es ist in **Figur 1** sehr deutlich zu sehen, dass der Katalysatoren mit Ligand **8** wesentlich aktiver bei 80 °C ist, als jener mit DTBPMB (Ligand **3**), ungefähr um den Faktor 5-6. Während das System mit **8** schon nach 10 Minuten fertig ist, braucht **3** ca. 60-70 Minuten. Beide erreichen die höchstmögliche Chemoselektivität (100 %) zum Methylpropionat. Somit zeigt der erfindungsgemäße Ligand eine deutliche Verbesserung gegenüber dem System aus dem Stand der Technik.

Deshalb wurde das System mit **8** genauer untersucht und Reaktionen bei 60 °C und 20 bar (technisch wichtige Druckstufe) CO durchgeführt, wobei der Druck von 20 bar konstant gehalten wurde.

### Methoxycarbonylierung von Ethen mit Liganden 3 und 8 bei 60 °C und 20 bar:

**3** (Vergleichsbeispiel): Ein 100 ml Stahlautoklav wird unter Argon mit [Pd(acac)₂] (6.53 mg, 0.04 mol%), und dem entsprechenden Liganden **3** (33 mg, 0.16 mol%) und p-Toluolsulfonsäure (PTSA 61 mg, 0.6 mol%) befüllt. Anschließend werden MeOH (20 ml) und Ethen der Reinheit 3.0 (1.5 g, 53 mmol) hinzugefügt. Der Autoklav wird auf 60 °C erhitzt und anschließend wird CO bis zu einem Gesamtdruck von 20 bar aufgepresst. Dieser Druck wird durch Zudosierung von CO aus einem Druckvorratsgefäß bei 20 bar konstant gehalten. Die Reaktion wird eine Stunde durchgeführt und der Gasverbrauch im Druckvorratsgefäß gemessen. Anschließend wird der Autoklav heruntergekühlt und der Druck langsam abgelassen. Der Autoklaveninhalt wird in ein Schlenkgefäß überführt und 5 ml Isooctan als interner Standard hinzugefügt. Die Ausbeute wurde mittels GC-Analyse bestimmt (100% Ausbeute). Die TOF bei 50 % Ausbeute beträgt 758 h⁻¹.

**8:** Ein 100 ml Stahlautoklav wird unter Argon mit [Pd(acac)₂] (6.53 mg, 0.04 mol%), und dem entsprechenden Liganden **8** (44 mg, 0.16 mol%) und p-Toluolsulfonsäure (PTSA 61 mg, 0.6 mol%) befüllt. Anschließend werden MeOH (20 ml) und Ethen der Reinheit 3.0 (1.5 g, 53 mmol) hinzugefügt. Der Autoklav wird auf 60 °C erhitzt und anschließend wird CO bis zu einem Gesamtdruck von 20 bar aufgepresst. Dieser Druck wird durch Zudosierung von CO aus einem Druckvorratsgefäß bei 20 bar konstant gehalten. Die Reaktion wird eine Stunde durchgeführt und der Gasverbrauch im Druckvorratsgefäß gemessen. Anschließend wird der Autoklav heruntergekühlt und der Druck langsam abgelassen. Der Autoklaveninhalt wird in ein Schlenkgefäß überführt und 5 ml Isooctan als interner Standard hinzugefügt. Die Ausbeute wurde mittels GC-Analyse bestimmt (100% Ausbeute). Die TOF bei 50 % Ausbeute beträgt 3213 h⁻¹.

**Figur 2** zeigt den Gasverbrauch aus einem Druckvorratsgefäß. Die Reaktion wurde gestartet mit dem Aufpressen von CO bei 60 °C (**Figur 2****:** Methoxycarbonylierung von Ethen mit **3** und **8** bei 60 °C und 20 bar CO (konstanter Druck)).
Auch hier zeigt sich, dass **8** wesentlich schneller und ohne Präformierungsphase die Reaktion durchführt. Es handelt sich hierbei somit um ein wesentlich schnelleres und hochselektives Katalysator-System, welches deutliche Vorteile gegenüber dem Stand der Technik (Ligand **3**) aufweist.

### Alkoxycarbonylierung (Vergleichsversuch)

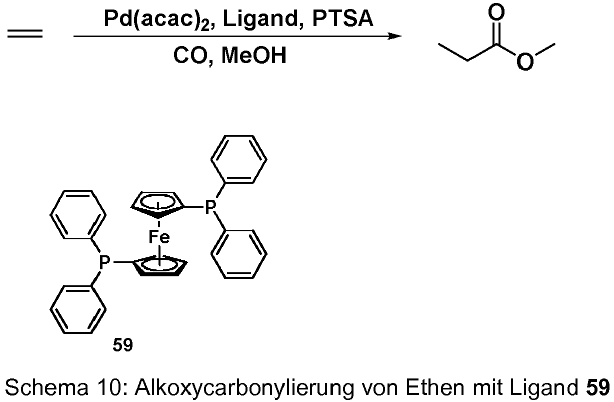

### Ligand 59:

Ligand **59,** 1,1'-Bis(diphenylphosphino)ferrocen, ist kommerziell verfügbar.

Ein 100 mL-Stahlautoklav wird mit Pd(acac)₂ (6.52 mg, 0.04 mol%) und Ligand **59** (47,9 mg, 0.16 mol%) und PTSA (61,1 mg, 0.6 mol%) und Methanol (20 mL) unter Argon befüllt. Dann werden 1.5 g, (53.6 mmol) Ethylen (3.5 von Linde AG) in den Autoklaven überführt. (Massekontrolle des Autoklaven). Nach dem Aufheizen auf eine Reaktionstemperatur von 80 °C (Druck ungefähr 10 bar), wird bei dieser Temperatur CO (30 bar) aufgepresst. Bei dieser Temperatur wird die Reaktion 20 Stunden durchgeführt. Dann wird der Autoklav auf Raumtemperatur heruntergekühlt und entspannt. Der Inhalt wird in ein 50 ml Schlenkgefäß transferiert und Isooctan (interner Standard, 5.0 mL) wird zugesetzt. Die Ausbeute und Selektivität wurde mittels GC Analyse bestimmt. (Ausbeute: 54%).

### Alkoxycarbonylierung von Ethen mit verschiedenen Alkoholen.

Allgemeine Arbeitsvorschrift: Ein 100 ml Stahlautoklav wird unter Argon mit Pd(acac)₂ (6.52 mg, 0.04 mol%), **8** (44.3 mg, 0.16 mol%) und PTSA (61.1 mg, 0.6 mol%) befüllt. 20 mL des entsprechenden Alkohols werden unter Argon zugesetzt. Dann werden 1,5 g Ethen (53,6 mmol) in den Autoklaven überführt (Massekontrolle). Es wird auf 80 °C erwärmt (der Druck beträgt nun ca. 10 bar). Bei dieser Temperatur werden 30 bar CO aufgepreßt und die Reaktion wird 20 h unter Rührung durchgeführt. Der Gasverbrauch wird gemessen mit Druckaufnehmer und der Specviewsoftware von Parr Instruments und stimmt mit dem Ausbeuteverlauf über die Zeit überein. Der Autoklav wird auf Raumtemperatur heruntergekühlt und der Restdruck langsam abgelassen. Der Inhalt wird in ein 50 ml Schlenkgefäß überführt, es werden 5 ml Isooctan als interner Standard zugesetzt und die Ausbeute mittels GC Analytik bestimmt. GC Analytik: Für die GC Analyse wird ein Chromatograph der Firma Agilent GC Agilent 7890A mit einer 30 m HP Säule verwendet. Temperaturprofil: 35°, 10 min; 10°/min zu 200°, 16.5 min; das Injektionsvolumen beträgt 1 µL mit einem Split von 50:1.

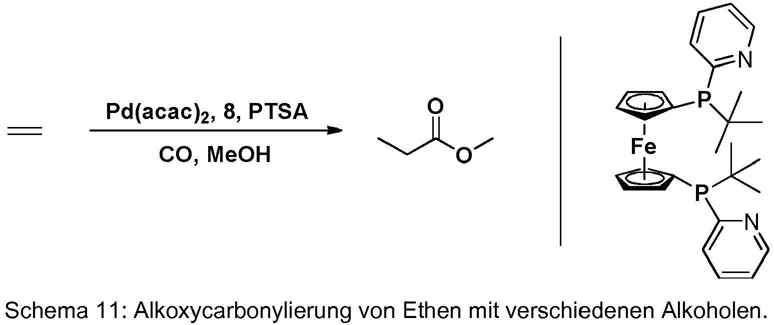

**Methanol:** Ein 100 ml Stahlautoklav wird unter Argon mit Pd(acac)₂ (6.52 mg, 0.04 mol%), **8** (44.3 mg, 0.16 mol%) und PTSA (61.1 mg, 0.6 mol%) befüllt. 20 mL Methanol werden unter Argon zugesetzt. Dann werden 1,5 g Ethen (53,6 mmol) in den Autoklaven überführt (Massekontrolle). Es wird auf 80 °C erwärmt (der Druck beträgt nun ca. 10 bar). Bei dieser Temperatur werden 30 bar CO aufgepresst und die Reaktion wird 20 h unter Rührung durchgeführt. Der Gasverbrauch wird mit Druckaufnehmer am Autoklaven und der Specviewsoftware von Parr Instruments gemessen und stimmt mit dem Ausbeuteverlauf über die Zeit überein.

Der Autoklav wird auf Raumtemperatur heruntergekühlt und der Restdruck langsam abgelassen. Der Inhalt wird in ein 50 ml Schlenkgefäß überführt, es werden 5 ml Isooctan als interner Standard zugesetzt und die Ausbeute mittels GC Analytik bestimmt. Sie beträgt am Ende der Reaktion 100 % an Methylpropionat. Retentionszeit: 6,148 min

**Ethanol:** Ein 100 ml Stahlautoklav wird unter Argon mit Pd(acac)₂ (6.52 mg, 0.04 mol%), **8** (44.3 mg, 0.16 mol%) und PTSA (61.1 mg, 0.6 mol%) befüllt. 20 mL Ethanol werden unter Argon zugesetzt. Dann werden 1,5 g Ethen (53,6 mmol) in den Autoklaven überführt (Massekontrolle). Es wird auf 80 °C erwärmt (der Druck beträgt nun ca. 10 bar). Bei dieser Temperatur werden 30 bar CO aufgepreßt und die Reaktion wird 20 h unter Rührung durchgeführt. Der Gasverbrauch wird gemessen mit Druckaufnehmer und der Specviewsoftware von Parr Instruments und stimmt mit dem Ausbeuteverlauf über die Zeit überein. Der Autoklav wird auf Raumtemperatur heruntergekühlt und der Restdruck langsam abgelassen. Der Inhalt wird in ein 50 ml Schlenkgefäß überführt, es werden 5 ml Isooctan als interner Standard zugesetzt und die Ausbeute mittels GC Analytik bestimmt. Sie beträgt am Ende der Reaktion 100 % an Ethylpropionat. Retentionszeit: 8,896 min

**1-Propanol:** Ein 100 ml Stahlautoklav wird unter Argon mit Pd(acac)₂ (6.52 mg, 0.04 mol%), **8** (44.3 mg, 0.16 mol%) und PTSA (61.1 mg, 0.6 mol%) befüllt. 20 mL 1-Propanol werden unter Argon zugesetzt. Dann werden 1,5 g Ethen (53,6 mmol) in den Autoklaven überführt (Massekontrolle). Es wird auf 80 °C erwärmt (der Druck beträgt nun ca. 10 bar). Bei dieser Temperatur werden 30 bar CO aufgepreßt und die Reaktion wird 20 h unter Rührung durchgeführt. Der Gasverbrauch wird gemessen mit Druckaufnehmer und der Specviewsoftware von Parr Instruments und stimmt mit dem Ausbeuteverlauf über die Zeit überein. Der Autoklav wird auf Raumtemperatur heruntergekühlt und der Restdruck langsam abgelassen. Der Inhalt wird in ein 50 ml Schlenkgefäß überführt, es werden 5 ml Isooctan als interner Standard zugesetzt und die Ausbeute mittels GC Analytik bestimmt. Sie beträgt am Ende der Reaktion 100 % an 1-Propylpropionat. Retentionszeit: 13,342 min

**1-Butanol:** Ein 100 ml Stahlautoklav wird unter Argon mit Pd(acac)₂ (6.52 mg, 0.04 mol%), **8** (44.3 mg, 0.16 mol%) und PTSA (61.1 mg, 0.6 mol%) befüllt. 20 mL 1-Butanol werden unter Argon zugesetzt. Dann werden 1,5 g Ethen (53,6 mmol) in den Autoklaven überführt (Massekontrolle). Es wird auf 80 °C erwärmt (der Druck beträgt nun ca. 10 bar). Bei dieser Temperatur werden 30 bar CO aufgepreßt und die Reaktion wird 20 h unter Rührung durchgeführt. Der Gasverbrauch wird gemessen mit Druckaufnehmer und der Specviewsoftware von Parr Instruments und stimmt mit dem Ausbeuteverlauf über die Zeit überein. Der Autoklav wird auf Raumtemperatur heruntergekühlt und der Restdruck langsam abgelassen. Der Inhalt wird in ein 50 ml Schlenkgefäß überführt, es werden 5 ml Isooctan als interner Standard zugesetzt und die Ausbeute mittels GC Analytik bestimmt. Sie beträgt am Ende der Reaktion 100 % an 1-Butylpropionat. Retentionszeit: 16,043 min

**1-Pentanol:** Ein 100 ml Stahlautoklav wird unter Argon mit Pd(acac)₂ (6.52 mg, 0.04 mol%), **8** (44.3 mg, 0.16 mol%) und PTSA (61.1 mg, 0.6 mol%) befüllt. 20 mL 1-Pentanol werden unter Argon zugesetzt. Dann werden 1,5 g Ethen (53,6 mmol) in den Autoklaven überführt (Massekontrolle). Es wird auf 80 °C erwärmt (der Druck beträgt nun ca. 10 bar). Bei dieser Temperatur werden 30 bar CO aufgepreßt und die Reaktion wird 20 h unter Rührung durchgeführt. Der Gasverbrauch wird gemessen mit Druckaufnehmer und der Specviewsoftware von Parr Instruments und stimmt mit dem Ausbeuteverlauf über die Zeit überein. Der Autoklav wird auf Raumtemperatur heruntergekühlt und der Restdruck langsam abgelassen. Der Inhalt wird in ein 50 ml Schlenkgefäß überführt, es werden 5 ml Isooctan als interner Standard zugesetzt und die Ausbeute mittels GC Analytik bestimmt. Sie beträgt am Ende der Reaktion 100 % an 1-Pentylpropionat. Retentionszeit: 17,949 min

**1-Hexanol:** Ein 100 ml Stahlautoklav wird unter Argon mit Pd(acac)₂ (6.52 mg, 0.04 mol%), **8** (44.3 mg, 0.16 mol%) und PTSA (61.1 mg, 0.6 mol%) befüllt. 20 mL 1-Hexanol werden unter Argon zugesetzt. Dann werden 1,5 g Ethen (53,6 mmol) in den Autoklaven überführt (Massekontrolle). Es wird auf 80 °C erwärmt (der Druck beträgt nun ca. 10 bar). Bei dieser Temperatur werden 30 bar CO aufgepreßt und die Reaktion wird 20 h unter Rührung durchgeführt. Der Gasverbrauch wird gemessen mit Druckaufnehmer und der Specviewsoftware von Parr Instruments und stimmt mit dem Ausbeuteverlauf über die Zeit überein. Der Autoklav wird auf Raumtemperatur heruntergekühlt und der Restdruck langsam abgelassen. Der Inhalt wird in ein 50 ml Schlenkgefäß überführt, es werden 5 ml Isooctan als interner Standard zugesetzt und die Ausbeute mittels GC Analytik bestimmt. Sie beträgt am Ende der Reaktion 100 % an 1-Hexylpropionat. Retentionszeit: 19,486 min

**2-Propanol:** Ein 100 ml Stahlautoklav wird unter Argon mit Pd(acac)₂ (6.52 mg, 0.04 mol%), **8** (44.3 mg, 0.16 mol%) und PTSA (61.1 mg, 0.6 mol%) befüllt. 20 mL 2-Propanol werden unter Argon zugesetzt. Dann werden 1,5 g Ethen (53,6 mmol) in den Autoklaven überführt (Massekontrolle). Es wird auf 80 °C erwärmt (der Druck beträgt nun ca. 10 bar). Bei dieser Temperatur werden 30 bar CO aufgepreßt und die Reaktion wird 20 h unter Rührung durchgeführt. Der Gasverbrauch wird gemessen mit Druckaufnehmer und der Specviewsoftware von Parr Instruments und stimmt mit dem Ausbeuteverlauf über die Zeit überein. Der Autoklav wird auf Raumtemperatur heruntergekühlt und der Restdruck langsam abgelassen. Der Inhalt wird in ein 50 ml Schlenkgefäß überführt, es werden 5 ml Isooctan als interner Standard zugesetzt und die Ausbeute mittels GC Analytik bestimmt. Sie beträgt am Ende der Reaktion 100 % an 2-Propylpropionat. Retentionszeit: 11,212 min

**t-Butanol:** Ein 100 ml Stahlautoklav wird unter Argon mit Pd(acac)₂ (6.52 mg, 0.04 mol%), **8** (44.3 mg, 0.16 mol%) und PTSA (61.1 mg, 0.6 mol%) befüllt. 20 mL t-Butanol werden unter Argon zugesetzt. Dann werden 1,5 g Ethen (53,6 mmol) in den Autoklaven überführt (Massekontrolle). Es wird auf 80 °C erwärmt (der Druck beträgt nun ca. 10 bar). Bei dieser Temperatur werden 30 bar CO aufgepreßt und die Reaktion wird 20 h unter Rührung durchgeführt. Der Gasverbrauch wird gemessen mit Druckaufnehmer und der Specviewsoftware von Parr Instruments und stimmt mit dem Ausbeuteverlauf über die Zeit überein. Der Autoklav wird auf Raumtemperatur heruntergekühlt und der Restdruck langsam abgelassen. Der Inhalt wird in ein 50 ml Schlenkgefäß überführt, es werden 5 ml Isooctan als interner Standard zugesetzt und die Ausbeute mittels GC Analytik bestimmt. Sie beträgt am Ende der Reaktion 47 % an t-Butylpropionat. Retentionszeit: 12,625 min

**3-Pentanol:** Ein 100 ml Stahlautoklav wird unter Argon mit Pd(acac)₂ (6.52 mg, 0.04 mol%), **8** (44.3 mg, 0.16 mol%) und PTSA (61.1 mg, 0.6 mol%) befüllt. 20 mL 3-Pentanol werden unter Argon zugesetzt. Dann werden 1,5 g Ethen (53,6 mmol) in den Autoklaven überführt (Massekontrolle). Es wird auf 80 °C erwärmt (der Druck beträgt nun ca. 10 bar). Bei dieser Temperatur werden 30 bar CO aufgepreßt und die Reaktion wird 20 h unter Rührung durchgeführt. Der Gasverbrauch wird gemessen mit Druckaufnehmer und der Specviewsoftware von Parr Instruments und stimmt mit dem Ausbeuteverlauf über die Zeit überein. Der Autoklav wird auf Raumtemperatur heruntergekühlt und der Restdruck langsam abgelassen. Der Inhalt wird in ein 50 ml Schlenkgefäß überführt, es werden 5 ml Isooctan als interner Standard zugesetzt und die Ausbeute mittels GC Analytik bestimmt. Sie beträgt am Ende der Reaktion 100 % an 3-Pentylpropionat. Retentionszeit: 16,648 min

**Cyclohexanol:** Ein 100 ml Stahlautoklav wird unter Argon mit Pd(acac)₂ (6.52 mg, 0.04 mol%), **8** (44.3 mg, 0.16 mol%) und PTSA (61.1 mg, 0.6 mol%) befüllt. 20 mL Cyclohexanol werden unter Argon zugesetzt. Dann werden 1,5 g Ethen (53,6 mmol) in den Autoklaven überführt (Massekontrolle). Es wird auf 80 °C erwärmt (der Druck beträgt nun ca. 10 bar). Bei dieser Temperatur werden 30 bar CO aufgepreßt und die Reaktion wird 20 h unter Rührung durchgeführt. Der Gasverbrauch wird gemessen mit Druckaufnehmer und der Specviewsoftware von Parr Instruments und stimmt mit dem Ausbeuteverlauf über die Zeit überein. Der Autoklav wird auf Raumtemperatur heruntergekühlt und der Restdruck langsam abgelassen. Der Inhalt wird in ein 50 ml Schlenkgefäß überführt, es werden 5 ml Isooctan als interner Standard zugesetzt und die Ausbeute mittels GC Analytik bestimmt. Sie beträgt am Ende der Reaktion 100 % an Cyclohexylpropionat. Retentionszeit: 19,938 min

**Phenol:** Ein 100 ml Stahlautoklav wird unter Argon mit Pd(acac)₂ (6.52 mg, 0.04 mol%), **8** (44.3 mg, 0.16 mol%) und PTSA (61.1 mg, 0.6 mol%) befüllt. 20 g Phenol werden unter Argon zugesetzt. Phenol wurde als Feststoff ohne Lösemittel zugegeben. Der Schmelzpunkt von Phenol liegt bei 40,5 °C. Alle Komponenten sollten daher bei 80 °C gelöst werden. Dann werden 1,5 g Ethen (53,6 mmol) in den Autoklaven überführt (Massekontrolle). Es wird auf 80 °C erwärmt (der Druck beträgt nun ca. 10 bar). Bei dieser Temperatur werden 30 bar CO aufgepresst und die Reaktion wird 20 h unter Rührung durchgeführt. Der Gasverbrauch wird gemessen mit Druckaufnehmer und der Specviewsoftware von Parr Instruments und stimmt mit dem Ausbeuteverlauf über die Zeit überein. Der Autoklav wird auf Raumtemperatur heruntergekühlt und der Restdruck langsam abgelassen. Der Inhalt wird in ein 50 ml Schlenkgefäß überführt, es werden 5 ml Isooctan als interner Standard zugesetzt und die Ausbeute mittels GC Analytik bestimmt. Sie beträgt am Ende der Reaktion 46 % an Phenylpropionat. Retentionszeit: 20,260 min

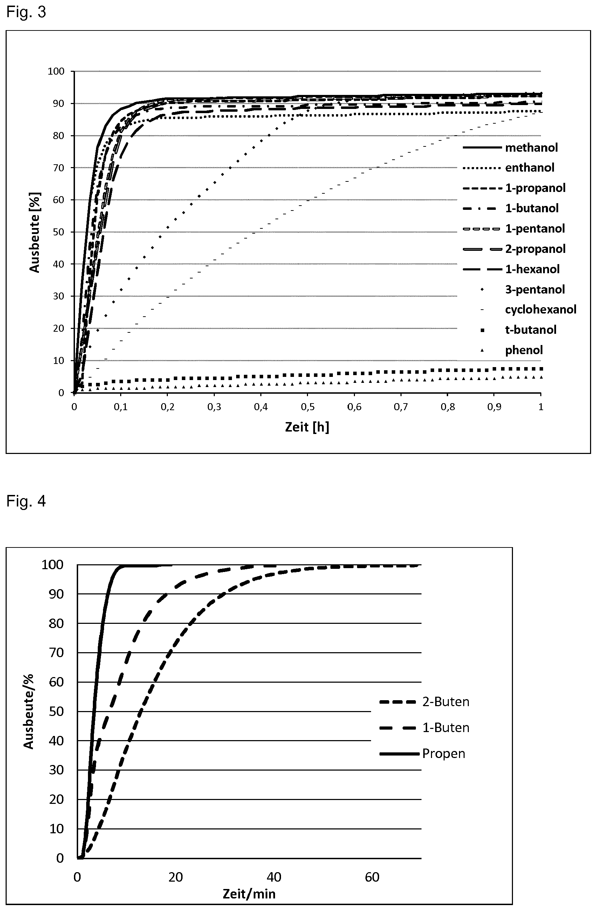

Die Ergebnisse sind in **Figur 3** dargestellt.
**Figur 3****:** Alkoholvariation in der Methoxycarbonylierung von Ethen mit Ligand **8** bei 80 °C und 30 bar CO-Druck.
Wie gut zu erkennen ist, kann nicht nur Methanol in der Alkoxycarbonylierung eingesetzt werden, sondern auch eine Vielzahl anderer Alkohole können ebenfalls verwendet werden. Die korrespondierenden Produkte können in guten bis sehr guten Ausbeuten (zum Teil quantitativ) erhalten werden.

### Umsetzung von 8 mit Propen

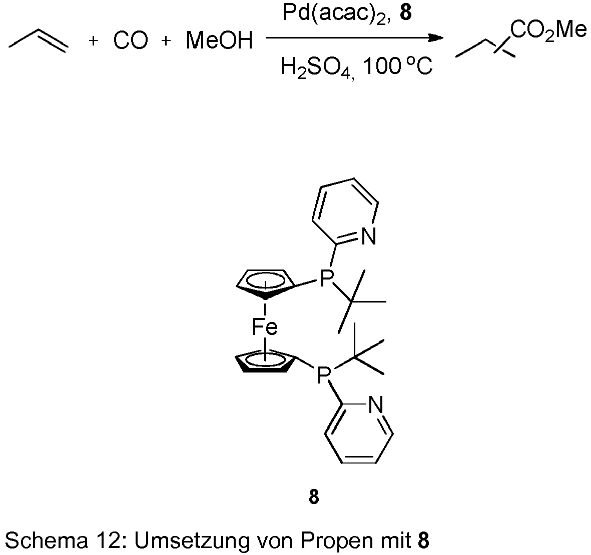

Ein 100 ml Stahlautoklav wird unter Argon mit Pd(acac)₂ (17.5 mg, 0.04 mol%), **8** (119 mg, 0.16 mol%), MeOH (15 mL) und [98% H₂SO₄] (38 uL, 0.5 mol%) befüllt. Dann wird der Autoklav mit Trockeneis heruntergekühlt. Propen (6.06 g, 144 mmol) wurde in einem anderen separaten Zylinder (75 ml, Massekontrolle) einkondensiert. Diese definierte Menge wurde anschließend in den Autoklaven einkondensiert. Dann werden auf den Autoklaven 40 bar CO bei Raumtemperatur aufgepresst. Die Reaktion wurde 30 Minuten bei 100 °C durchgeführt. Nach der Reaktion wird der Autoklav auf Raumtemperatur heruntergekühlt und der Druck abgelassen. Es werden 8,5 ml Isooctan als interner Standard zur Lösung gegeben. Die Ausbeute und Selektivität wurde mittels GC Analyse bestimmt. (Ausbeute: >99%, *n*/*iso*: 77/23)

### Umsetzung von 1-Buten mit 8

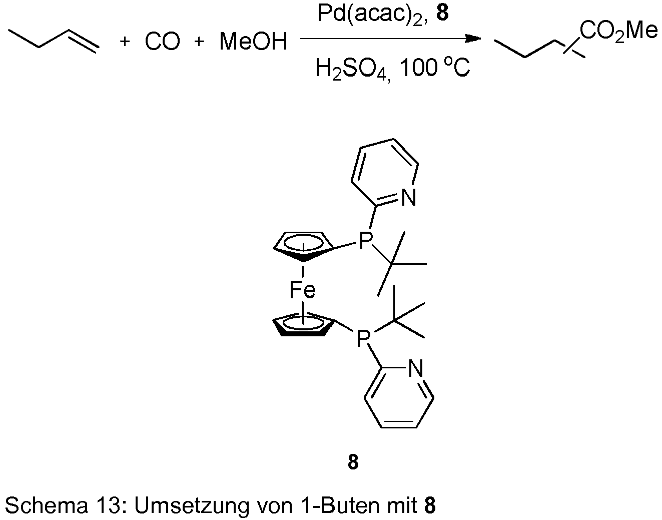

Ein 100 ml Stahlautoklav wird unter Argon mit Pd(acac)₂ (17.5 mg, 0.04 mol%), **8** (119 mg, 0.16 mol%), MeOH (15 mL) und [98% H₂SO₄] (38 uL, 0.5 mol%) befüllt. Dann wird der Autoklav mit Trockeneis heruntergekühlt. 1-Buten (8,04 g, 144 mmol) wurde in einem anderen separaten Zylinder (75 ml, Massekontrolle) einkondensiert. Diese definierte Menge wurde anschließend in den Autoklaven einkondensiert. Dann werden auf den Autoklaven 40 bar CO bei Raumtemperatur aufgepreßt. Die Reaktion wurde 60 Minuten bei 100 °C durchgeführt. Nach der Reaktion wird der Autoklav auf Raumtemperatur heruntergekühlt und der Druck abgelassen. Es werden 8,5 ml Isooctan als interner Standard zur Lösung gegeben. Die Ausbeute und Selektivität wurde mittels GC-Analyse bestimmt. (Ausbeute: >99%, *n*/*iso*: 80/20)

### Umsetzung von 2-Buten mit 8

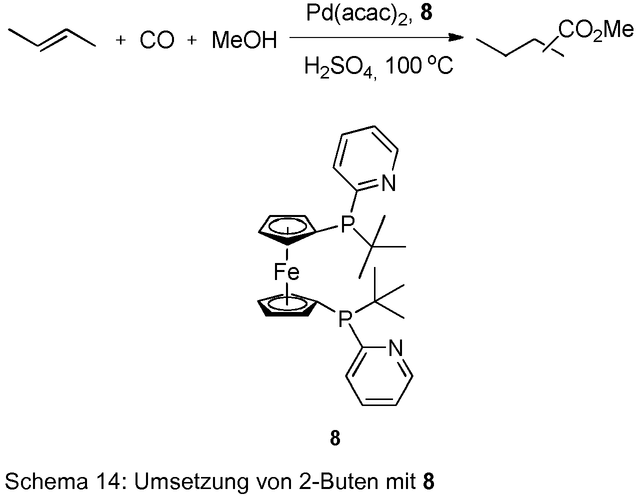

Ein 100 ml Stahlautoklav wird unter Argon mit Pd(acac)₂ (17.5 mg, 0.04 mol%), **8** (119 mg, 0.16 mol%), MeOH (15 mL) und [98% H₂SO₄] (38 uL, 0.5 mol%) befüllt. Dann wird der Autoklav mit Trockeneis heruntergekühlt. 2-Buten (8,04 g, 144 mmol) wurde in einem anderen separaten Zylinder (75 ml, Massekontrolle) einkondensiert. Diese definierte Menge wurde anschließend in den Autoklaven einkondensiert. Dann werden auf den Autoklaven 40 bar CO bei Raumtemperatur aufgepresst. Die Reaktion wurde 60 Minuten bei 100 °C durchgeführt. Nach der Reaktion wird der Autoklav auf Raumtemperatur heruntergekühlt und der Druck abgelassen. Es werden 8,5 ml Isooctan als interner Standard zur Lösung gegeben. Die Ausbeute und Selektivität wurde mittels GC-Analyse bestimmt. (Ausbeute: >99%, *n*/*iso*: 75/25)

Die Ergebnisse sind in **Figur 4** dargestellt. Diese zeigt den Ausbeuteverlauf der oben genannten Reaktionen, der aus der Gasverbrauchskurve umgerechnet wurde. Die Kurve wurde über die gaschromatographisch ermittelte Ausbeute nach Abschluss der Reaktion angepasst.

**Figur 4****:** Versuche zur Methoxycarbonylierung von Propen, 1-Buten und 2-Buten bei 100 °C und 40 bar mit dem Liganden **8.**

Wie der **Figur 4** entnommen werden kann fallen die Umsetzungsgeschwindigkeiten der Olefine mit steigender Kettenlänge. Die Umsetzungsgeschwindigkeit ist für terminale Olefine höher als für Olefine mit innenständiger Doppelbindung. Während Propen in weniger als 10 Minuten vollständig umgesetzt ist werden für 1-Buten ca. 40 Minuten und für 2-Buten fast 60 Minuten für einen vollständigen Umsatz (100 % Ausbeute) benötigt.

### Umsetzung von Raffinat 1 mit Verbindung 8

Es wurden auch technische Gemische, u.a. das so genannte Raffinat 1 getestet. Raffinat 1 setzt sich zusammen aus 42 % Isobuten, 26 % 1-Buten, 17 % cis- und trans-2-Buten sowie 0,3 % 1,3-Butadien und 15 % n-Butan und Isobutan.

Vorschrift: Ein 100 ml-Stahlautoklav wurde unter Argonatmosphäre mit [Pd(acac)₂] (17.4 mg), **8** (118.9 mg) und H₂SO₄ (70.6 mg) beladen. Methanol (15 ml) wurde unter Ar-Atmosphäre zugegeben. Der Autoklav wurde mit Trockeneis gekühlt Danach wurden 8.2 g Raffinat 1 in einen separaten Zylinder einkondensiert (75 ml, Massenkontrolle) und diese definierte Menge an Substrat wurde in den gekühlten Autoklaven einkondensiert. Danach wurde der Autoklav mit 60 bar CO bei Raumtemperatur beaufschlagt. Die Reaktion wurde 20 h bei 100 °C durchgeführt. Danach wurde der Inhalt in einen 50 ml-Schlenkkolben überführt und Isooctan wurde als interner Standard zugegeben. Ausbeute und Selektivität wurden mittels GC-Analyse bestimmt.
Ergebnis: C5-Ester: 9.7 g, n/iso 37/63, MTBE: 2.0 g.

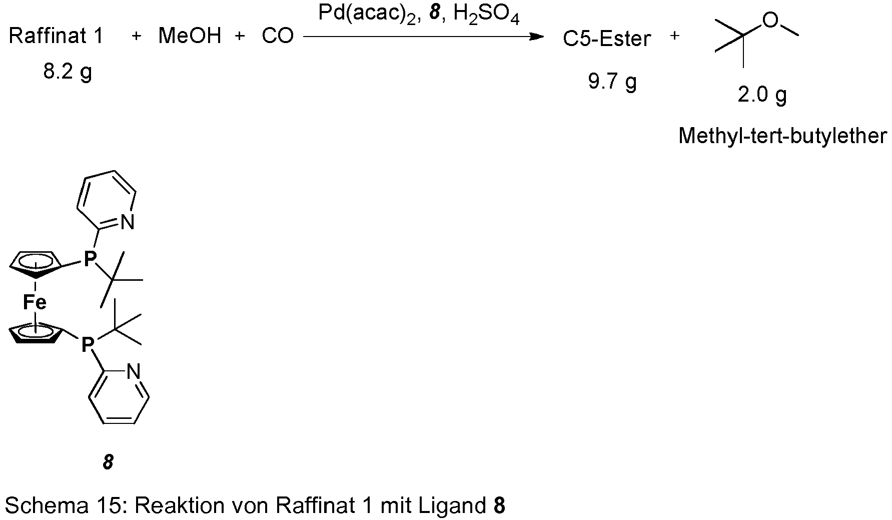

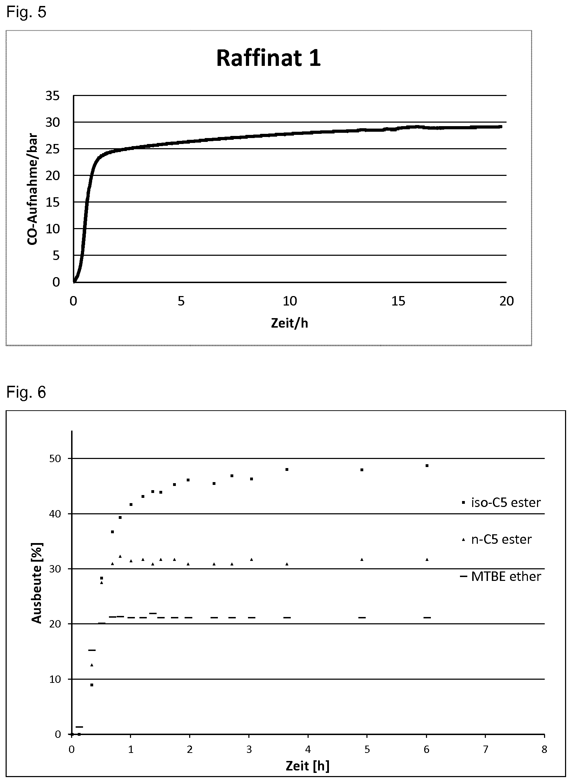

Die Ergebnisse sind ferner in **Figur 5** dargestellt.
**Figur 5****:** Methoxycarbonylierung von Raffinat 1 mit dem Liganden 8 bei 100 °C und 60 bar CO-Druck.

Es konnte somit gezeigt werden, dass auch technisch relevante Gemische, wie hier Raffinat 1, mit dem erfindungsgemäßen Liganden **8** umgesetzt werden können.

### Raffinat 1 mit Probennahme

Weiterhin wurde Raffinat 1 mit dem Liganden **8** umgesetzt.

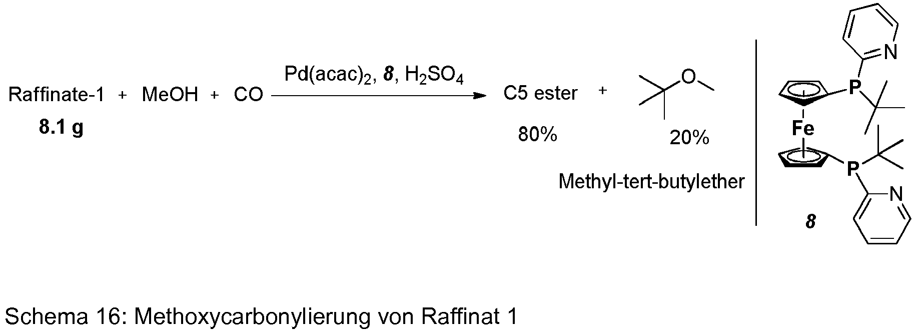

Allgemeine Arbeitsvorschrift. Ein 100 ml Stahlautoklav wird unter Argon mit [Pd(acac)₂] (17.4 mg), **8** (118.9 mg) und H₂SO₄ (70.6 mg) befüllt. Dann werden 15ml MeOH und 10 ml Isooctan als interner Standard zugesetzt. Dann wird der Autoklav mit Trockeneis auf -78 °C heruntergekühlt. Raffinat 1(8,1 g) werden in einem separaten 75 ml Druckzylinder einkondensiert (Massekontrolle). Diese definierte Masse wird nun in den Autoklaven einkondensiert. Der Autoklav wird bei Raumtemperatur mit 50 bar CO befüllt. Es wird auf 100 °C erwärmt und 20 h bei dieser Temperatur gerührt. Während dieser Zeit werden Proben (16 Stück) aus dem Autoklaven mittels eines HPLC Ventils und einer internen Kapillare entnommen. Die Ausbeute und Selektivität wird mittels GC Analytik bestimmt. GC Analytik: Für die GC Analyse wird ein Chromatograph der Firma Agilent GC Agilent 7890A mit einer 30 m HP Säule verwendet. Temperaturprofil: 35°, 10 min; 10°/min zu 200°, 16.5 min; das Injektionsvolumen beträgt 1 µL mit einem Split von 50:1.
Retentionszeit für MTBE: 5.067 min
Retentionszeit für iso-C5 Ester: 12.118 min
Retentionszeit für n-C5 Ester: 13.807 min

Die Ergebnisse sind in **Figur 6** dargestellt

**Figur 6****:** Methoxycarbonylierung von Raffinat 1 bei 100 °C und 50 bar mit Ligand **8.** Am Ende der Reaktion liegen 80 % C5 Ester und 20 % Methyltertbutylether bezogen auf die eingesetzte Menge an Olefinen vor.

Somit ist der Ligand **8** für die Umsetzung eines technisch relevanten Feeds, Raffinat 1, gut geeignet.

**Figur 5** zeigt die Gasaufnahmekurve für den Versuch ohne Probenahme, der 20 Stunden gelaufen ist und zu 9.7 g C5-Ester mit einem n/iso-Verhältnis von 37/63 sowie einem MTBE-Anteil von 2.0 g geführt hat. Der in **Figur 6** durchgeführte Versuch führt zu 32 % n-C5-Ester und 48 % iso-C5-Ester. Dies entspricht einem n/iso-Verhältnis von 33/67. Der Massenanteil an Methyl-tert.-Butylether liegt bei 20 %. Aus **Figur 5** ergibt sich ein Massenanteil von 17 %. Beide Versuche liefern also ähnliche Resultate. Aus **Figur 5** ist zu erkennen, dass die wesentliche Reaktion bereits nach ca. 1 Stunde beendet ist. Auch dies steht in Übereinstimmung mit dem Versuch mit Probenahme in **Figur 6****.**

### Methoxycarbonylierung von Isobuten mit Ligand 3 und 8

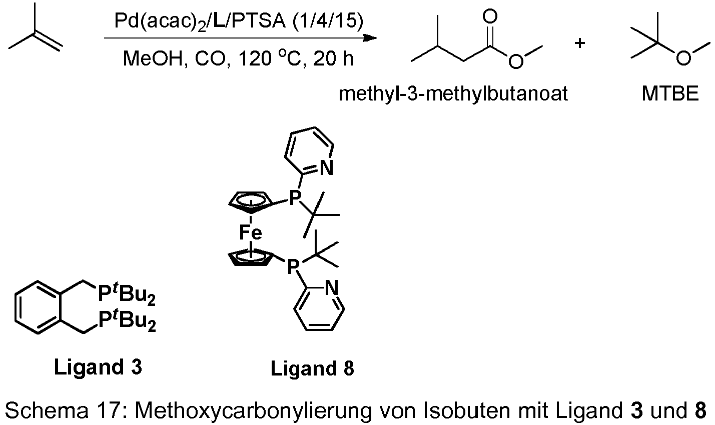

**Ligand 3** (Vergleichsbeispiel): Ein 100 mL Stahlautoklav wird unter Argon mit Pd(acac)₂ (4.9 mg), DTBPMB (25.3 mg), PTSA (45.6 mg) und MeOH (20 mL) befüllt. Anschließend wird der Autoklav mit Trockeneis heruntergekühlt. In einem separaten Druckbehälter werden 2,5 g Isobuten(Massekontrolle) einkondensiert. Diese definierte Masse wird in den Autoklaven einkondensiert. Dann wird der Autoklav bei Raumtemperaturmit 40 bar CO befüllt. Die Reaktion wird 20 Stunden bei 120 °C durchgeführt. Anschließend wird auf Raumtemperatur heruntergekühlt und der Autoklav entspannt, der Inhalt in ein 50 ml Schlenkgefäß überführt und Isoctoan (5 ml als interner Standard) werden zugesetzt. Es erfolgt eine GC Analyse. (GC Analyse (50% Ausbeute an Methyl 3-methylbutanoat, 37% Ausbeute an MTBE).

**Ligand 8:** Ein 100 mL Stahlautoklav wird unter Argon mit Pd(acac)₂ (4.9 mg), **8** (33,1 mg), PTSA (45.6 mg) und MeOH (20 mL) befüllt. Anschließend wird der Autoklav mit Trockeneis heruntergekühlt. In einem separaten Druckbehälter werden 2,5 g Isobuten (Massekontrolle) einkondensiert. Diese definierte Masse wird in den Autoklaven einkondensiert. Dann wird der Autoklav bei Raumtemperatur mit 40 bar CO befüllt. Die Reaktion wird 20 Stunden bei 120 °C durchgeführt. Anschließend wird auf Raumtemperatur heruntergekühlt und der Autoklav entspannt, der Inhalt in ein 50 ml Schlenkgefäß überführt und Isoctoan (5 ml als interner Standard) werden zugesetzt. Es erfolgt eine GC Analyse. (99% Ausbeute an Methyl 3-methylbutanoat)

### Testung einer Mischung von Propen, 1-Buten und 2-Buten

Ferner wurden auch Mischungen von Edukten getestet, d.h. Gemische, die verschiedene ungesättigte Verbindungen enthalten.

Vorschrift: Ein 100 ml-Stahlautoklav wurde unter Argonatmosphäre mit [Pd(acac)₂] (17.4 mg), **8** (118.9 mg) und H₂SO₄ (70.6 mg) beladen. Methanol (15 ml) wurde unter Ar-Atmosphäre zugegeben. Der Autoklav wurde mit Trockeneis gekühlt. Danach wurden 2.83 g, 2-Buten 4.85 mg und Propen (2.2 g) in drei separate Zylinder einkondensiert (75 ml, Massenkontrolle) und diese definierten Mengen an Gassubstrat wurden in den gekühlten Autoklaven einkondensiert. Danach wurde der Autoklav mit 60 bar CO bei Raumtemperatur beaufschlagt. Die Reaktion wurde 20 h bei 100 °C durchgeführt. Danach wurde der Inhalt in einen 50 ml-Schlenkkolben überführt und Isooctan wurde als interner Standard zugegeben. Ausbeute und Selektivität wurden mittels GC-Analyse bestimmt. (Ausbeute: 100 %, C4-Ester: n/iso 79/21,, C5-Ester: n/iso: 75/25).

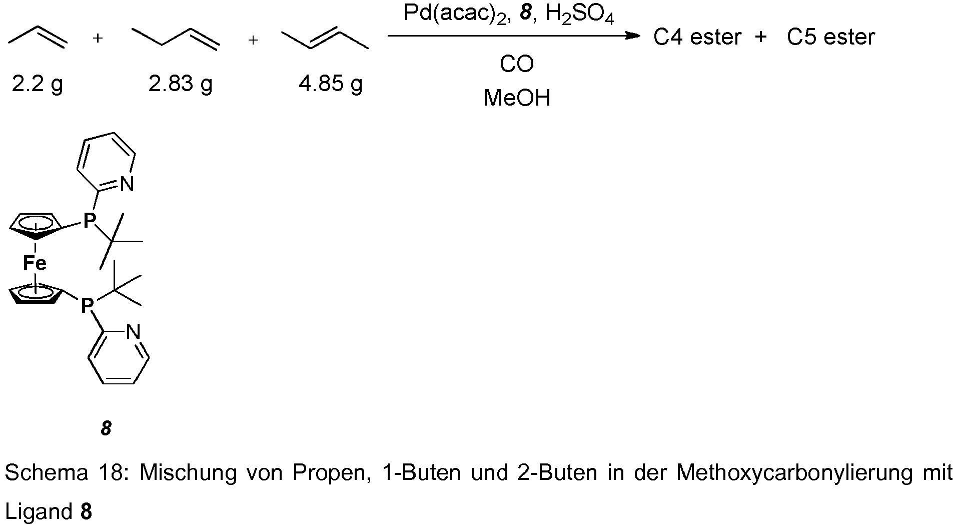

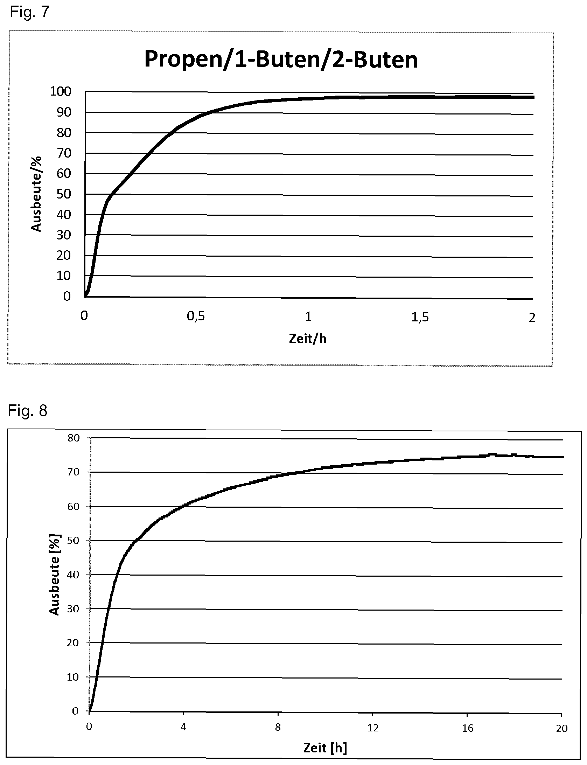

Die Ergebnisse sind in **Figur 7** dargestellt
**Figur 7****:** Methoxycarbonylierung einer Mischung von Propen, 1-Buten und 2-Buten bei 100 °C und 60 bar mit dem Liganden **8.**

Wie **Figur 7** zu entnehmen ist wird mit dem Gemisch aus Propen, 1-Buten und 2-Buten nach etwa 1 Stunde Reaktionszeit annähernd vollständige Ausbeute an den Methoxycarbonylierungsprodukten erreicht.

### Umsetzung von Tetramethylethylen mit verschiedenen Liganden bei verschiedenen Temperaturen

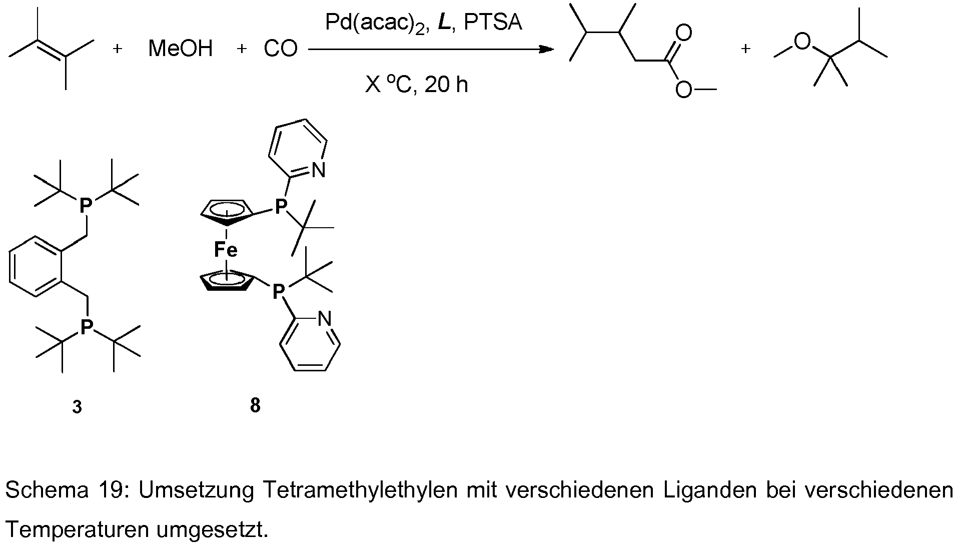

### a) Reaktionstemperatur: 100 °C

**3** (Vergleichsbeispiel): Ein 25 mL Schlenkgefäß wurde mit [Pd(acac)₂] (4.87 mg, 0.1 mol%), p-Toluolsulfonsäure (PTSA) (24.32 mg, 0.8 mol%) und MeOH (8 mL) befüllt. Ein 4 mL-Fläschchen wurde mit **3** (6.3 mg, 0.4 mol%) befüllt und ein Magnetrührstab hinzugegeben. Danach wurden 2 mL der klaren gelben Lösung und Tetramethylethylen (478 µL, 4 mmol) mit einer Spritze injiziert. Das Fläschchen wurde auf einen Probenhalter gesetzt, der wiederum unter Argon-Atmosphäre in einen 300 mL Parr-Autoklaven eingesetzt wurde. Nach dreimaligem Spülen des Autoklaven mit Stickstoff wurde der CO-Druck auf 40 bar eingestellt. Die Reaktion lief bei 100 °C 20 Stunden. Nach Abschluss der Reaktion wurde der Autoklav auf Raumtemperatur abgekühlt und vorsichtig entspannt. Isooctan (200 µL) wurde als interner GC-Standard zugesetzt. Ausbeute und Regioselektivität wurden mittels GC bestimmt. (Umsatz: 40%, keine Ester-Produktausbeute; Ether-Produktausbeute 38 %).

**8:** Ein 25 mL Schlenkgefäß wurde mit [Pd(acac)₂] (4.87 mg, 0.1 mol%), p-Toluolsulfonsäure (PTSA) (24.32 mg, 0.8 mol%) und MeOH (8 mL) befüllt. Ein 4 mL-Fläschchen wurde mit **8** (8.3 mg, 0.4 mol%) befüllt und ein Magnetrührstab hinzugegeben. Danach wurden 2 mL der klaren gelben Lösung und Tetramethylethylen (478 µL, 4 mmol) mit einer Spritze injiziert. Das Fläschchen wurde auf einen Probenhalter gesetzt, der wiederum unter Argon-Atmosphäre in einen 300 mL Parr-Autoklaven eingesetzt wurde. Nach dreimaligem Spülen des Autoklaven mit Stickstoff wurde der CO-Druck auf 40 bar eingestellt. Die Reaktion lief bei 100 °C 20 Stunden. Nach Abschluss der Reaktion wurde der Autoklav auf Raumtemperatur abgekühlt und vorsichtig entspannt. Isooctan (200 µL) wurde als interner GC-Standard zugesetzt. Ausbeute und Regioselektivität wurden mittels GC bestimmt. (Umsatz: 65%, Ester-Produktausbeute: 37 %, Ether-Produktausbeute 27 %).

### b) Reaktionstemperatur: 120 °C

**3** (Vergleichsbeispiel): Ein 25 mL Schlenkgefäß wurde mit [Pd(acac)₂] (4.87 mg, 0.1 mol%), p-Toluolsulfonsäure (PTSA) (24.32 mg, 0.8 mol%) und MeOH (8 mL) befüllt. Ein 4 mL-Fläschchen wurde mit **3** (6.3 mg, 0.4 mol%) befüllt und ein Magnetrührstab hinzugegeben. Danach wurden 2 mL der klaren gelben Lösung und Tetramethylethylen (478 µL, 4 mmol) mit einer Spritze injiziert. Das Fläschchen wurde auf einen Probenhalter gesetzt, der wiederum unter Argon-Atmosphäre in einen 300 mL Parr-Autoklaven eingesetzt wurde. Nach dreimaligem Spülen des Autoklaven mit Stickstoff wurde der CO-Druck auf 40 bar eingestellt. Die Reaktion lief bei 120 °C 20 Stunden. Nach Abschluss der Reaktion wurde der Autoklav auf Raumtemperatur abgekühlt und vorsichtig entspannt. Isooctan (200 µL) wurde als interner GC-Standard zugesetzt. Ausbeute und Regioselektivität wurden mittels GC bestimmt. (Umsatz: 54%, keine Ester-Produktausbeute; Ether-Produktausbeute 52 %).

**8:** Ein 25 mL Schlenkgefäß wurde mit [Pd(acac)₂] (4.87 mg, 0.1 mol%), p-Toluolsulfonsäure (PTSA) (24.32 mg, 0.8 mol%) und MeOH (8 mL) befüllt. Ein 4 mL-Fläschchen wurde mit **8** (8.3 mg, 0.4 mol%) befüllt und ein Magnetrührstab hinzugegeben. Danach wurden 2 mL der klaren gelben Lösung und Tetramethylethylen (478 µL, 4 mmol) mit einer Spritze injiziert. Das Fläschchen wurde auf einen Probenhalter gesetzt, der wiederum unter Argon-Atmosphäre in einen 300 mL Parr-Autoklaven eingesetzt wurde. Nach dreimaligem Spülen des Autoklaven mit Stickstoff wurde der CO-Druck auf 40 bar eingestellt. Die Reaktion lief bei 120 °C 20 Stunden. Nach Abschluss der Reaktion wurde der Autoklav auf Raumtemperatur abgekühlt und vorsichtig entspannt. Isooctan (200 µL) wurde als interner GC-Standard zugesetzt. Ausbeute und Regioselektivität wurden mittels GC bestimmt. (Umsatz: 90%, Ester-Produktausbeute: 60 %, Ether-Produktausbeute 28 %).

### Methoxycarbonylierung von C5 Olefinen

Arbeitsvorschrift: Ein 100 ml Stahlautoklav wird unter Argon mit [Pd(acac)₂] (10.95 mg, 0.04 mol%), **8** (74.31 mg, 0.16 mol%) und H₂SO₄ (44.1 mg, 0.5 mol%) befüllt. Anschließend werden 10 ml MeOH, 1-Penten (0.5 g), 2-Penten (2.21 g), 2-Methyl-1-buten (1.27 g), und 2-Methyl-2-buten (1.3 g) unter Argon zugesetzt. Dann wird der Autoklav mittels Trockeneis auf -78 °C heruntergekühlt. 1,1 g 3-Methyl-1-buten (1.1 g) werden in einen separaten Druckbehälter einkondensiert (Massekontrolle) und diese definierte Menge in den Autoklaven einkondensiert. Anschließend wird der Autoklav bei Raumtemperatur mit 50 bar CO befüllt. Unter Rührung wird die Reaktion 20 h bei 100 °C durchgeführt. Dann wird der Autoklav auf Raumtemperatur heruntergekühlt und der Restdruck langsam abgelassen. Der Inhalt wird in eine 50 ml Schlenkgefäß überführt und es werden 5 ml Isooctan als interner Standard zugesetzt. Die Ausbeute wird mittels GC Analytik bestimmt. Sie beträgt am Ende der Reaktion 76 % an C6 Methylestern.
GC Analytik: Für die GC Analyse wird ein Chromatograph der Firma Agilent GC Agilent 7890A mit einer 30 m HP Säule verwendet. Temperaturprofil: 35°, 10 min; 10°/min zu 200°, 16.5 min; das Injektionsvolumen beträgt 1 µL mit einem Split von 50:1.
Retentionszeiten für die C6-Methylester und deren Isomere : 14.547-16.362 min (Hauptpeak:16.362 min)

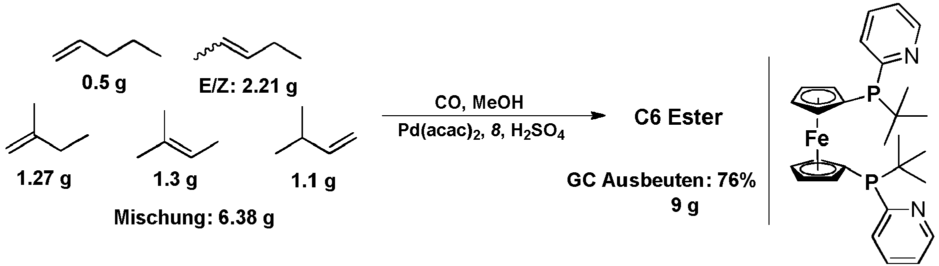

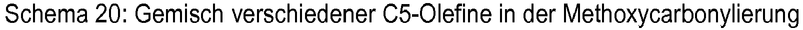

Die Ergebnisse sind in **Figur 8** dargestellt.
**Figur 8****:** Methoxycarbonylierung eines Gemischs von C5-Olefinen bei 100 °C und 50 bar CO-Druck mit dem Liganden **8.**
Wie deutlich ersichtlich ist, können die korrespondierenden C6-Ester als Gemisch in guten Ausbeuten (deutlich >50%) erhalten werden.

### Umsetzung von Di-n-Buten mit dem Liganden 8

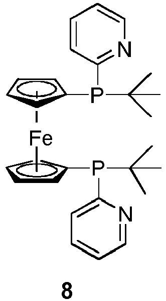

Weiterhin wurde ein Versuch mit konstantem Druck und Gasverbrauchsmessung mit **8** bei 20 bar Gesamtdruck durchgeführt.

Experimentalbeispiel: Ein 100 ml Stahlautoklav wird unter Argon mit [Pd(acac)₂] (5,85 mg, 0.04 mol%), und dem entsprechenden Liganden **8** (39,6 mg, 0.16 mol%) und p-Toluolsulfonsäure (PTSA 54,7 mg, 0.6 mol%) befüllt. Anschließend werden MeOH (30 ml) und Di-n-Buten (7,54 ml, 48 mmol) hinzugefügt. Der Autoklav wird auf 120 °C erhitzt und anschließend wird CO bis zu einem Gesamtdruck von 20 bar aufgepresst. Dieser Druck wird durch Zudosierung von CO aus einem Druckvorratsgefäß bei 20 bar konstant gehalten. Die Reaktion wird 20 Stunden durchgeführt und der Gasverbrauch im Druckvorratsgefäß gemessen. Anschließend wird der Autoklav heruntergekühlt und der Druck langsam abgelassen. Der Autoklaveninhalt wird in ein Schlenkgefäß überführt und 5 ml Isooctan als interner Standard hinzugefügt. Die Ausbeute wurde mittels GC-Analyse bestimmt (86 % Ausbeute, n:iso = 75:25).

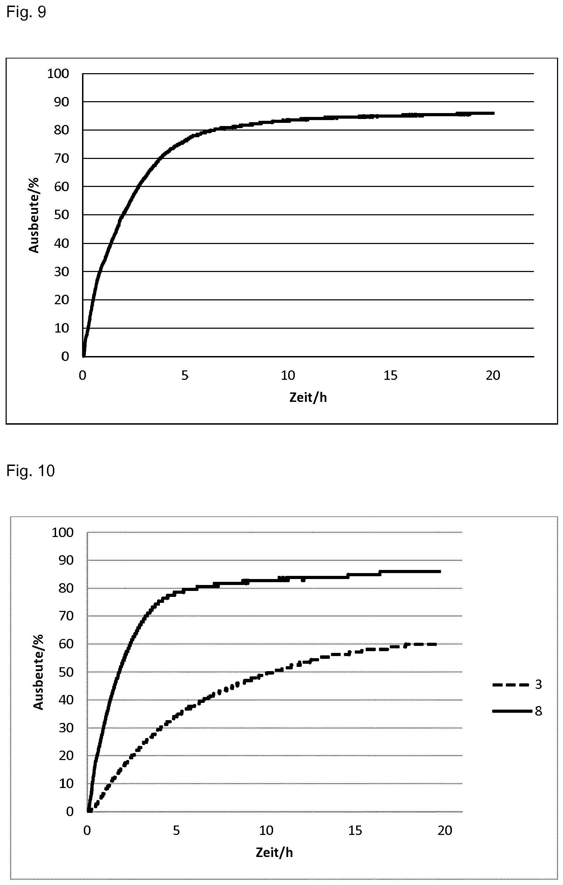

Die Ergebnisse sind in **Figur 9** dargestellt.

**Figur 9****:** Methoxycarbonylierung von Di-n-Buten mit Ligand 8 bei 120 °C und 20 bar mit konstantem CO-Druck

Bereits nach 5 Stunden wird mit dem Liganden **8** eine Ausbeute an Methylisononanoat (MINO) von mehr als 80 % erreicht; Ausbeute und n:iso-Verhältnis nach 20 Stunden entsprechen dem Versuch mit Ligand 8 bei 120 °C und 40 bar unter instationärem CO-Druck (siehe oben). Ein geringerer CO-Druck von 20 bar während der Reaktion ist somit ohne Verlust an Ausbeute und Selektivität anwendbar.

### Methoxycarbonylierung von Di-n-Buten mit Liganden 3 und 8

Um einen guten Vergleich der Liganden in der Methoxycarbonylierung von Di-n-Buten zu haben, wurden Experimente mit Gasverbrauchsmessungen durchgeführt.

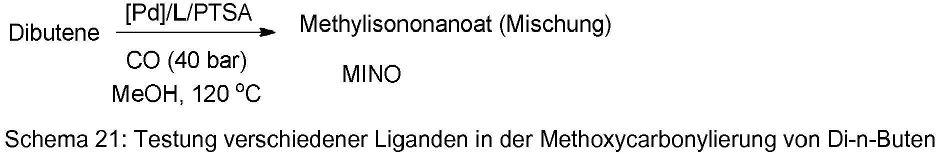

**3** (Vergleichsbeispiel): Ein 100 ml Stahlautoklav wird unter Argon mit [Pd(acac)₂] (5.85 mg, 0.04 mol%), **3** (30.3 mg, 0.16 mol%) befüllt. Anschließend werden MeOH (30 ml) und Di-n-Buten (7,54 ml, 48 mmol) und PTSA (54,7 mg, 0.6 mol%) hinzugefügt. Der Autoklav wird bei Raumtemperatur mit 40 bar CO der Reinheit 4.7 befüllt und die Reaktion wird 20 Stunden bei 120 °C durchgeführt. Anschließend wird der Autoklav heruntergekühlt und der Druck langsam abgelassen. Der Autoklaveninhalt wird in ein Schlenkgefäß überführt. Es werden 5 ml Isooctan als interner Standard zugesetzt und die Ausbeute und Selektivität mittels GC Analyse bestimmt (60 % Ausbeute an MINO, *n*/*iso:* 93/7).

**8:** Ein 100 ml Stahlautoklav wird unter Argon mit [Pd(acac)₂] (5.85 mg, 0.04 mol%), **8** (39,6 mg, 0.16 mol%) befüllt. Anschließend werden MeOH (30 ml) und Di-n-Buten (7,54 ml, 48 mmol) und PTSA (54,7 mg, 0.6 mol%) hinzugefügt. Der Autoklav wird bei Raumtemperatur mit 40 bar CO der Reinheit 4.7 befüllt und die Reaktion wird 20 Stunden bei 120 °C durchgeführt. Anschließend wird der Autoklav heruntergekühlt und der Druck langsam abgelassen. Der Autoklaveninhalt wird in ein Schlenkgefäß überführt. Es werden 5 ml Isooctan als interner Standard zugesetzt und die Ausbeute und Selektivität mittels GC Analyse bestimmt (86 % Ausbeute an MINO, *n*/*iso:* 75/25).

**Figur 10** zeigt die Gasverbrauchskurven (bzw. Ausbeute Zeit Verlauf) der getesteten Systeme. **Figur 10****:** Methoxycarbonylierung von Di-n-Buten mit **3** und **8** bei 120 °C und 40 bar CO.

Aus den Gasverbrauchsmessungen und den Experimentalbeispielen geht eindeutig hervor, das **8** schneller als **3** ist. Obwohl die n-Selektivität mit 75 % geringer als bei den Reaktionen mit **3** als Liganden ausfällt, ist hinsichtlich einer möglichen technischen Realisierung und der möglichst hohen Raum-Zeitausbeute dem Liganden **8** der Vorzug zu geben.

Weiterhin wurde ein Versuch mit konstantem Druck und Gasverbrauchsmessung mit **8** bei 20 bar Gesamtdruck durchgeführt.

Experimentalbeispiel: Ein 100 ml Stahlautoklav wird unter Argon mit [Pd(acac)₂] (5,85 mg, 0.04 mol%), und dem entsprechenden Liganden **8** (39,6 mg, 0.16 mol%) und p-Touluolsulfonsäure (PTSA 54,7 mg, 0.6 mol%) befüllt. Anschließend werden MeOH (30 ml) und Di-n-Buten (7,54 ml, 48 mmol) hinzugefügt. Der Autoklav wird auf 120 °C erhitzt und anschließend wird CO bis zu einem Gesamtdruck von 20 bar aufgepresst. Dieser Druck wird durch Zudosierung von CO aus einem Druckvorratsgefäß bei 20 bar konstant gehalten. Die Reaktion wird eine Stunde durchgeführt und der Gasverbrauch im Druckvorratsgefäß gemessen. Anschließend wird der Autoklav heruntergekühlt und der Druck langsam abgelassen. Der Autoklaveninhalt wird in ein Schlenkgefäß überführt und 5 ml Isooctan als interner Standard hinzugefügt. Die Ausbeute wurde mittels GC-Analyse bestimmt (86 % Ausbeute, n:iso = 75:25).

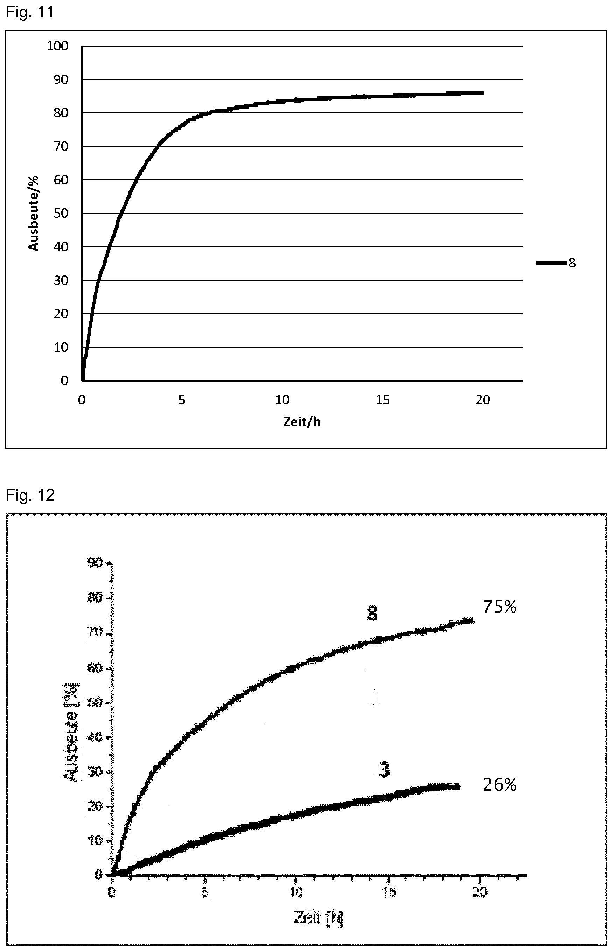

Die Ergebnisse sind in **Figur 11** dargestellt.
**Figur 11****:** Ausbeutekurve für die Methoxycarbonylierung von Di-n-Buten mit 8 als Liganden bei 20 bar konstantem Gesamtdruck und 120 °C.

Es zeigt sich eine gleiche Performance wie bei dem Experiment 40 bar CO nicht konstant. Dies bedeutet, dass die Methoxycarbonylierung von Di-n-Buten mit **8** als Liganden über einen gewissen CO Druckbereich unabhängig vom CO Druck ist, und technisch günstige niedrigere Drücke unter 20 bar realisierbar sind.

### Umsetzung von Di-n-Buten weiteren Liganden (Vergleichsversuche im 12fach-Autoklaven)

Die Umsetzung von Di-n-Buten mit Hilfe von verschiedenen Liganden erfolgte nach folgender Vorschrift:
Vorschrift: Ein 50 mL Schlenkgefäß wurde mit [Pd(acac)₂] (3.9 mg, 0.04 mol%), MeSO₃H (Methansulfonsäure) (13 uL, 0.6 mol%) und MeOH (20 mL) befüllt. Ein 4 mL-Fläschchen wurde mit Ligand X (0.16 mol%) befüllt und ein Magnetrührstab hinzugegeben. Danach wurden 1.25 mL der klaren gelben Stammlösung und Di-n-Buten (315 µL, 2mmol) mit einer Spritze injiziert. Das Fläschchen wurde auf einen Probenhalter gesetzt, derwiederum unter Argon-Atmosphäre in einen 600 mL Parr-Autoklaven eingesetzt wurde. Nach dreimaligem Spülen des Autoklaven mit Stickstoff wurde der CO-Druck auf 40 bar eingestellt. Die Reaktion lief bei 120 °C 20 Stunden. Nach Abschluss der Reaktion wurde der Autoklav auf Raumtemperatur abgekühlt und vorsichtig entspannt. Isooctan wurde als interner GC-Standard zugesetzt. Ausbeute und Regioselektivität wurden mittels GC bestimmt.

Die Ergebnisse sind im nachfolgenden Schema 22 zusammengefasst:

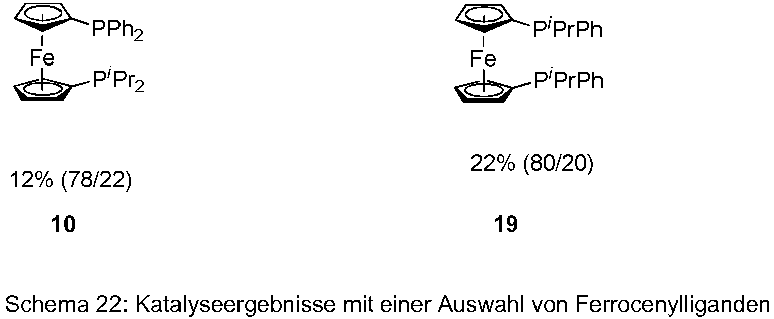

### Bestimmung der Raum-Zeit-Ausbeute RZA

Unter der Raum-Zeit-Ausbeute (RZA) versteht man die spezifische Produktleistung (in einem Reaktor gebildete Produktmenge) eines Reaktionsgefäßes (Reaktor) pro Raum und Zeit, beispielsweise t (Tonnen) Produkt pro Kubikmeter und Zeit oder kg pro Liter und Sekunde.

Vorschrift: In einem ausgeheizten Schlenkkolben werden jeweils 1,6 mol% PTSA (180 mg), 0,04 mol% Pd(acac)₂ (7,5 mg) und 0,16 mol% Ligand **3** oder **8** vorgelegt. Dann werden 6,26 ml (150 mmol) Methanol (tech.) und 9,39 ml (60 mmol) Di-n-Buten zugegeben und in einen 100 ml Autoklaven überführt. Der Autoklav wird dann 2-mal mit 10 bar CO gespült, mit 6 bar CO beladen und auf 100 °C erhitzt. Dann wird der Autoklav mittels Gasbürette mit 12 bar CO beladen und unter konstantem CO-Druck (12 bar) 20 h bei 100 °C gerührt. Nach Beendigung der Reaktion wird Isooctan (interner Standard) und 10 ml EtOAc zugegeben. Die organische Phase wurde per GC analysiert.

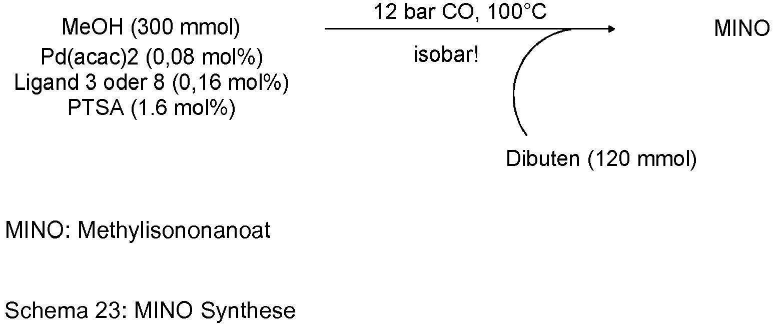

Die Ergebnisse sind in **Figur 12** dargestellt.
**Figur 12****:** Gasverbrauchskurven von Reaktionen mit **3** und **8.**

### C-18-Olefine

Methyloleat (Alfa Aesar, H311358, LOT:10164632)

### Umsetzung von Methyloleat mit den Liganden 3 und 8

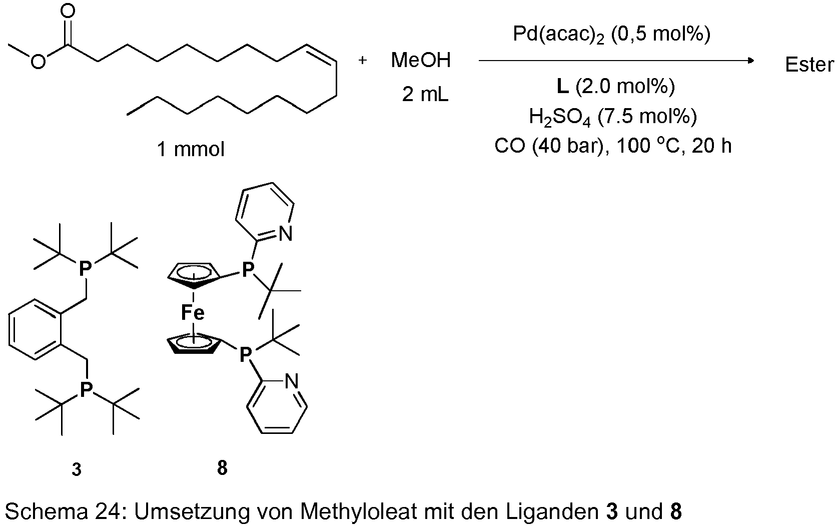

**3** (Vergleichsbeispiel): Ein 25 mL Schlenkgefäß wurde mit [Pd(acac)₂] (4.57 mg, 0.05 mol%), H₂SO₄ (22.05 mg, 7.5 mol%) und MeOH (6 mL) befüllt. Ein 4 mL-Fläschchen wurde mit **3** (7.9 mg, 2.0 mol%) befüllt und ein Magnetrührstab hinzugegeben. Danach wurden 2 mL der klaren gelben Lösung und Methyloleat (339 µL, 1 mmol) mit einer Spritze injiziert. Das Fläschchen wurde auf einen Probenhalter gesetzt, der wiederum unter Argon-Atmosphäre in einen 300 mL Parr-Autoklaven eingesetzt wurde. Nach dreimaligem Spülen des Autoklaven mit Stickstoff wurde der CO-Druck auf 40 bar eingestellt. Die Reaktion lief bei 100 °C 20 Stunden. Nach Abschluss der Reaktion wurde der Autoklav auf Raumtemperatur abgekühlt und vorsichtig entspannt. Isooctan (100 µL) wurde als interner GC-Standard zugesetzt. Ausbeute und Regioselektivität wurden mittels GC bestimmt. Ausbeute an linearem Ester: 54 %, keine verzweigten Ester.

**8:** Ein 25 mL Schlenkgefäß wurde mit [Pd(acac)₂] (4.57 mg, 0.05 mol%), H₂SO₄ (22.05 mg, 7.5 mol%) und MeOH (6 mL) befüllt. Ein 4 mL-Fläschchen wurde mit **8** (10.3 mg, 2.0 mol%) befüllt und ein Magnetrührstab hinzugegeben. Danach wurden 2 mL der klaren gelben Lösung und Methyloleat (339 µL, 1 mmol) mit einer Spritze injiziert. Das Fläschchen wurde auf einen Probenhalter gesetzt, der wiederum unter Argon-Atmosphäre in einen 300 mL Parr-Autoklaven eingesetzt wurde. Nach dreimaligem Spülen des Autoklaven mit Stickstoff wurde der CO-Druck auf 40 bar eingestellt. Die Reaktion lief bei 100 °C 20 Stunden. Nach Abschluss der Reaktion wurde der Autoklav auf Raumtemperatur abgekühlt und vorsichtig entspannt. Isooctan (100 µL) wurde als interner GC-Standard zugesetzt. Ausbeute und Regioselektivität wurden mittels GC bestimmt. Ausbeute an linearem Ester: 98 %, keine verzweigten Ester.

Anhand der Ergebnisse wird deutlich, dass der erfindungsgemäße Ligand **8** besser zur Umsetzung von Methyloleat geeignet ist als der Ligand 3 aus dem Stand der Technik.

### Umsetzung verschiedener Olefine unter optimierten Bedingungen

Die Bedingungen der Methoxycarbonylierung von Di-n-Buten wurden wie folgt optimiert:

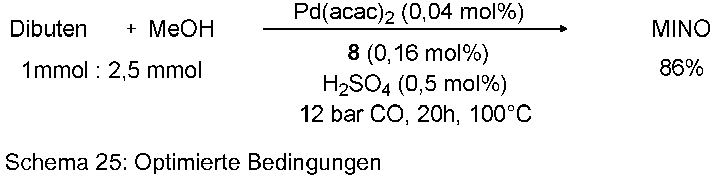

Die optimierten Bedingungen wurden auf eine Reihe von Alkenen angewendet (Tabelle 1).

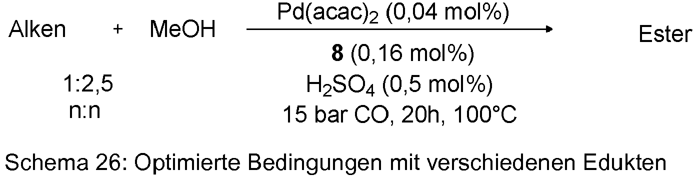

Vorschrift: In ausgeheizte Glasvials werden jeweils 1 mg (0,04 mol%) Pd(acac)₂ und 7,2 mg (0,16 mol%) Ligand **8** vorgelegt und mit je 812 µL (20 mmol) Methanol (techn.) und 8 mmol Alken versetzt. Dann werden je 2 µL (0,5 mol%) H₂SO₄ (98%ig) (100 µL einer Schwefelsäurelösung in Methanol enthalten 2 µL Schwefelsäure) zugegeben.
Die Reaktionen werden im Autoklaven 2-mal mit 10 bar CO gespült, mit 15 bar CO beladen und bei 100 °C 20 h gerührt. Nach Beendigung der Reaktion werden jeweils Isooctan (interner Standard) und 1 ml EtOAc zugegeben. Die organische Phase wird per GC analysiert.

Die Ergebnisse sind in Tabelle 1 zusammengestellt.

**Tabelle 1: Substrattestung mit verschiedenen Alkenen**

| Alken | Alken [%] | Ester [%] | n-Selektivität [%] |
|---|---|---|---|
| Di-n-Buten | 16 | 82 | 79 |
| 1-Octen | 0 | 100 | 72 |
| 1-Decen | 0 | 100 | 72 |
| 1-Hexen | 0 | 100 | 78 |
| 2-Octen | 2 | 98 | 72 |
| Limonen | 27 | 14 | - |
| Vinylcyclohexen | 13 | 52 | 95 |
| 1,7-Octadien | 27 | 26 | 65 |
| Methyl-10-undecanoat | 13 | 86 | 75 |
| Methyloleat | 23 | 75 | nicht zuzuordnen |
| Methyl-3-Pentenoat | 23 | 74 | 82 |

Die n-Selektivität in Tabelle 1 ist definiert als der Anteil der terminalen Methoxycarbonylierung bezogen auf die Gesamtausbeute der Methoxycarbonylierungsprodukte.

Es zeigt sich, daß lineare terminale Olefine wie 1-Octen, 1-Decen, 1-Hexen, sowie 2-Octen quantitative Ester Ausbeuten liefern. Ebenfalls gute Ausbeuten liefern Methyl-3-pentenoat, Methyloleat und Methylundecenoat. Bei Vinylcyclohexen wird zu 52 % monomethoxycarbonyliert und auch teilweise wird auch die interne Doppelbindung methoxycarbonyliert (35 %). Octadien wird zu 55% einfach methoxycarbonyliert und zu 26 % doppelt.

Die beschriebenen Experimente zeigen, dass sich die erfindungsgemäßen Verbindungen als Katalysatorliganden für die Alkoxycarbonylierung einer Vielzahl ethylenisch ungesättigter Verbindungen eignen. Insbesondere werden mit den erfindungsgemäßen Verbindungen bessere Ausbeuten erzielt als mit den aus dem Stand der Technik bekannten zweizähnigen Phosphinliganden, wie 1,2-Bis(di-tert-butylphosphinomethyl)benzol (DTBPMB, Ligand **3**), 1,1'-Bis(diphenylphosphino)ferrocen (Ligand **59**), 1-(Diphenylphosphino)-1'-(diisopropylphosphino)ferrocen (Ligand **10**) und 1,1'-Bis(isopropylphenylphosphino)ferrocen (Ligand **19**). Des Weiteren ermöglichen die erfindungsgemäßen Verbindungen auch die Alkoxycarbonylierung von technisch bedeutsamen, langkettigen Olefinen wie Di-n-Buten und 2-Octen, sowie von Olefingemischen, wie dem beschriebenen Raffinat 1.

## Patentansprüche

1. Verbindung gemäß Formel (**I**) 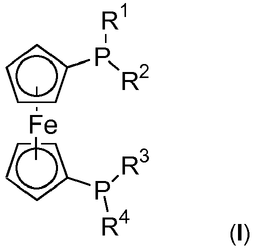 wobei
R¹, R², R³, R⁴ jeweils unabhängig voneinander ausgewählt sind aus -(C₁-C₁₂)-Alkyl,-(C₃-C₁₂)-Cycloalkyl, -(C₃-C₁₂)-Heterocycloalkyl, -(C₆-C₂₀)-Aryl, -(C₃-C₂₀)-Heteroaryl; mindestens einer der Reste R¹, R², R³, R⁴ für einen -(C₆-C₂₀)-Heteroarylrest mit mindestens sechs Ringatomen steht;
und
R¹, R², R³, R⁴, falls diese für -(C₁-C₁₂)-Alkyl, -(C₃-C₁₂)-Cycloalkyl, -(C3-C12)-Heterocycloalkyl, -(C₆-C₂₀)-Aryl, -(C₃-C₂₀)-Heteroaryl oder -(C₆-C₂₀)-Heteroaryl stehen, jeweils unabhängig voneinander mit einem oder mehreren Substituenten ausgewählt aus
-(C₁-C₁₂)-Alkyl, -(C₃-C₁₂)-Cycloalkyl, -(C₃-C₁₂)-Heterocycloalkyl, -O-(C₁-C₁₂)-Alkyl, -O-(C₁-C₁₂)-Alkyl-(C₆-C₂₀)-Aryl, -O-(C₃-C₁₂)-Cycloalkyl, -S-(C₁-C₁₂)-Alkyl, -S-(C₃-C₁₂)-Cycloalkyl, -COO-(C₁-C₁₂)-Alkyl, -COO-(C₃-C₁₂)-Cycloalkyl, -CONH-(C₁-C₁₂)-Alkyl,-CONH-(C₃-C₁₂)-Cycloalkyl, -CO-(C₁-C₁₂)-Alkyl, -CO-(C₃-C₁₂)-Cycloalkyl, -N-[(C₁-C₁₂)-Alkyl]₂, -(C₆-C₂₀)-Aryl, -(C₆-C₂₀)-Aryl-(C₁-C₁₂)-Alkyl, -(C₆-C₂₀)-Aryl-O-(C₁-C₁₂)-Alkyl,-(C₃-C₂₀)-Heteroaryl, -(C₃-C₂₀)-Heteroaryl-(C₁-C₁₂)-Alkyl, -(C₃-C₂₀)-Heteroaryl-O-(C₁-C₁₂)-Alkyl, -COOH, -OH, -SO₃H, -NH₂, Halogen substituiert sein können.

2. Verbindung nach Anspruch 1,
wobei mindestens zwei der Reste R¹, R², R³, R⁴ für einen -(C₆-C₂₀)-Heteroarylrest mit mindestens sechs Ringatomen stehen.

3. Verbindung nach einem der Ansprüche 1 bis 2,
wobei die Reste R¹ und R³ jeweils für einen -(C₆-C₂₀)-Heteroarylrest mit mindestens sechs Ringatomen stehen.

4. Verbindung nach einem der Ansprüche 1 bis 3,
wobei die Reste R¹ und R³ jeweils für einen -(C₆-C₂₀)-Heteroarylrest mit mindestens sechs Ringatomen stehen;
R² für -(C₆-C₂₀)-Heteroaryl mit mindestens sechs Ringatomen steht oder ausgewählt ist aus -(C₁-C₁₂)-Alkyl, -(C₃-C₁₂)-Cycloalkyl, -(C₃-C₁₂)-Heterocycloalkyl, -(C₆-C₂₀)-Aryl; und R⁴ ausgewählt ist aus -(C₁-C₁₂)-Alkyl, -(C₃-C₁₂)-Cycloalkyl, -(C3-C12)-Heterocycloalkyl, -(C₆-C₂₀)-Aryl.

5. Verbindung nach einem der Ansprüche 1 bis 4,
wobei die Reste R¹ und R³ jeweils für einen -(C₆-C₂₀)-Heteroarylrest mit mindestens sechs Ringatomen stehen;
und R² und R⁴ ausgewählt sind aus -(C₁-C₁₂)-Alkyl, -(C₃-C₁₂)-Cycloalkyl, -(C₃-C₁₂)-Heterocycloalkyl, -(C₆-C₂₀)-Aryl.

6. Verbindung nach einem der Ansprüche 1 bis 5,
wobei die Reste R¹ und R³ jeweils für einen -(C₆-C₂₀)-Heteroarylrest mit mindestens sechs Ringatomen stehen;
und R² und R⁴ für -(C₁-C₁₂)-Alkyl stehen.

7. Verbindung nach einem der Ansprüche 1 bis 6,
wobei R¹, R², R³, R⁴, falls diese für einen Heteroarylrest stehen, jeweils unabhängig voneinander ausgewählt sind aus Pyridyl, Pyridazinyl, Pyrimidyl, Pyrazinyl, Benzofuranyl, Indolyl, Isoindolyl, Benzimidazolyl, Chinolyl, Isochinolyl.

8. Verbindung nach einem der Ansprüche 1 bis 7,
gemäß einer der Formeln (**8**), (**14**) und (**15**)

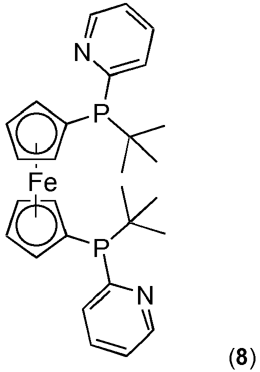

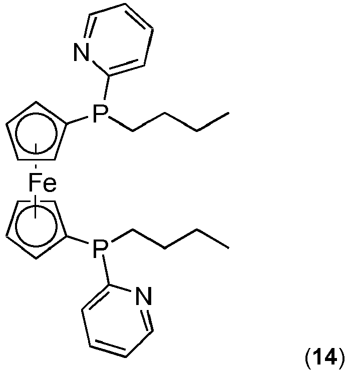

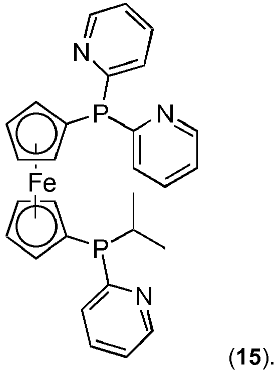

9. Komplex umfassend Pd und eine Verbindung nach einem der Ansprüche 1 bis 8.

10. Verfahren umfassend die Verfahrensschritte:
a) Vorlegen einer ethylenisch ungesättigten Verbindung;
b) Zugabe einer Verbindung nach einem der Ansprüche 1 bis 8 und einer Verbindung, welche Pd umfasst,
oder Zugabe eines Komplexes nach Anspruch 9;
c) Zugabe eines Alkohols;
d) Zuführen von CO;
e) Erwärmen des Reaktionsgemisches, wobei die ethylenisch ungesättigte Verbindung zu einem Ester umgesetzt wird.

11. Verfahren nach Anspruch 10,
wobei die ethylenisch ungesättigte Verbindung ausgewählt ist aus Ethen, Propen, 1-Buten, *cis-* und/oder *trans*-2-Buten, iso-Buten, 1,3-Butadien, 1-Penten, *cis-* und/oder *trans*-2-Penten, 2-Methyl-1-buten, 3-Methyl-1-buten, 2-Methyl-2-buten, Hexen, Tetramethylethylen, Hepten, 1-Octen, 2-Octen, Di-n-Buten, oder Mischungen davon.

12. Verfahren nach einem der Ansprüche 10 bis 11,
wobei die Verbindung, welche Pd umfasst, in Verfahrenschritt b) ausgewählt ist aus Palladiumdichlorid, Palladium(II)-acetylacetonat, Palladium(II)-acetat, Dichloro(1,5-cyclooctadiene)palladium(II), Bis(dibenzylideneaceton)palladium, Bis(acetonitrile)dichloropalladium(II), Palladium(cinnamyl)dichlorid.

13. Verfahren nach einem der Ansprüche 10 bis 12,
wobei der Alkohol in Verfahrensschritt c) ausgewählt ist aus Methanol, Ethanol, 1-Propanol, 1-Butanol, 1-Pentanol, 1-Hexanol, 2-Propanol, *tert*-Butanol, 3-Pentanol, Cyclohexanol, Phenol, oder Mischungen davon.

14. Verwendung einer Verbindung gemäß einem der Ansprüche 1 bis 8 oder eines Komplexes gemäß Anspruch 9 zur Katalyse einer Alkoxycarbonylierungsreaktion.

## Claims

1. Compound of formula (**I**) 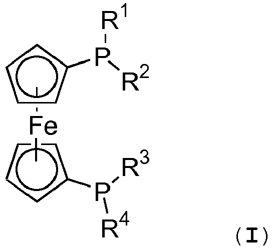 where
R¹, R², R³, R⁴ are each independently selected from - (C₁-C₁₂) -alkyl, -(C₃-C₁₂)-cycloalkyl, -(C₃-C₁₂)-heterocycloalkyl, -(C₆-C₂₀)-aryl, -(C₃-C₂₀)-heteroaryl;
at least one of the R¹, R², R³, R⁴ radicals is a-(C₆-C₂₀)-heteroaryl radical having at least six ring atoms;
and
R¹, R², R³, R⁴, if they are -(C₁-C₁₂)-alkyl, -(C₃-C₁₂)-cycloalkyl, -(C₃-C₁₂) -heterocycloalkyl, -(C₆-C₂₀)-aryl, -(C₃-C₂₀)-heteroaryl or -(C₆-C₂₀)-heteroaryl, may each independently be substituted by one or more substituents selected from
-(C₁-C₁₂) -alkyl, -(C₃-C₁₂)-cycloalkyl, -(C₃-C₁₂)-heterocycloalkyl, -O-(C₁-C₁₂) -alkyl, -O-(C₁-C₁₂)-alkyl-(C₆-C₂₀)-aryl, -O-(C₃-C₁₂) -cycloalkyl, -S-(C₁-C₁₂)-alkyl, -S-(C₃-C₁₂) -cycloalkyl, -COO-(C₁-C₁₂)-alkyl, -COO-(C₃-C₁₂) -cycloalkyl, -CONH-(C₁-C₁₂)-alkyl, -CONH-(C₃-C₁₂)-cycloalkyl, -CO-(C₁-C₁₂)-alkyl, -CO-(C₃-C₁₂)-cycloalkyl, -N-[(C₁-C₁₂)-alkyl]₂, -(C₆-C₂₀)-aryl, -(C₆-C₂₀)-aryl-(C₁-C₁₂)-alkyl, -(C₆-C₂₀)-aryl-O-(C₁-C₁₂)-alkyl, -(C₃-C₂₀)-heteroaryl, -(C₃-C₂₀)-heteroaryl-(C₁-C₁₂)-alkyl, -(C₃-C₂₀)-heteroaryl-O-(C₁-C₁₂)-alkyl, -COOH, -OH, -SO₃H, -NH₂, halogen.

2. Compound according to Claim 1,
where at least two of the R¹, R², R³, R⁴ radicals are a -(C₆-C₂₀)-heteroaryl radical having at least six ring atoms.

3. Compound according to either of Claims 1 and 2,
where the R¹ and R³ radicals are each a -(C₆-C₂)-heteroaryl radical having at least six ring atoms.

4. Compound according to any of Claims 1 to 3,
where the R¹ and R³ radicals are each a -(C₆-C₂₀)-heteroaryl radical having at least six ring atoms; R² is -(C₆-C₂₀)-heteroaryl having at least six ring atoms or is selected from -(C₁-C₁₂)-alkyl, -(C₃-C₁₂)-cycloalkyl, -(C₃-C₁₂)-heterocycloalkyl, -(C₆-C₂₀)-aryl;
and R⁴ is selected from -(C₁-C₁₂)-alkyl, -(C₃-C₁₂)-cycloalkyl, -(C₃-C₁₂)-heterocycloalkyl, -(C₆-C₂₀)-aryl.

5. Compound according to any of Claims 1 to 4,
where the R¹ and R³ radicals are each a -(C₆-C₂₀)-heteroaryl radical having at least six ring atoms; and R² and R⁴ are selected from -(C₁-C₁₂)-alkyl,-(C₃-C₁₂)-cycloalkyl, -(C₃-C₁₂)-heterocycloalkyl,-(C₆-C₂₀)-aryl.

6. Compound according to any of Claims 1 to 5,
where the R¹ and R³ radicals are each a -(C₆-C₂₀)-heteroaryl radical having at least six ring atoms; and R² and R⁴ are -(C₁-C₁₂)-alkyl.

7. Compound according to any of Claims 1 to 6,
where R¹, R², R³, R⁴, if they are a heteroaryl radical, are each independently selected from pyridyl, pyridazinyl, pyrimidyl, pyrazinyl, benzofuranyl, indolyl, isoindolyl, benzimidazolyl, quinolyl, isoquinolyl.

8. Compound according to any of Claims 1 to 7,
of one of the formulae (**8**), (**14**) and (**15**)

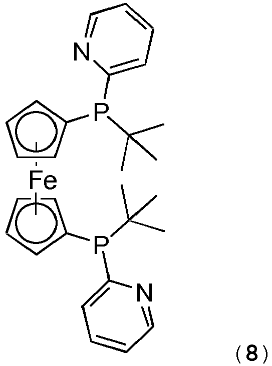

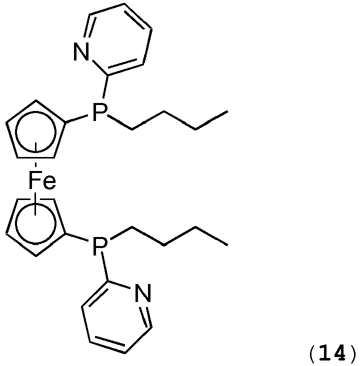

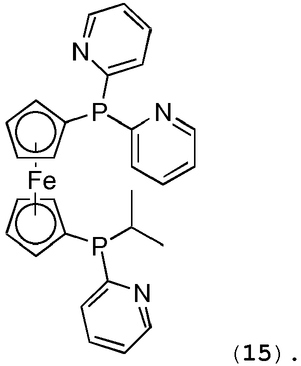

9. Complex comprising Pd and a compound according to any of Claims 1 to 8.

10. Process comprising the following process steps:
a) initially charging an ethylenically unsaturated compound;
b) adding a compound according to any of Claims 1 to 8 and a compound comprising Pd,
or adding a complex according to Claim 9;
c) adding an alcohol;
d) feeding in CO;
e) heating the reaction mixture, with conversion of the ethylenically unsaturated compound to an ester.

11. Process according to Claim 10,
wherein the ethylenically unsaturated compound is selected from ethene, propene, 1-butene, *cis-* and/or *trans*-2-butene, isobutene, 1,3-butadiene, 1-pentene, *cis-* and/or *trans*-2-pentene, 2-methyl-1-butene, 3-methyl-1-butene, 2-methyl-2-butene, hexene, tetramethylethylene, heptene, 1-octene, 2-octene, di-n-butene, and mixtures thereof.

12. Process according to either of Claims 10 and 11,
wherein the compound comprising Pd in process step b) is selected from palladium dichloride, palladium(II) acetylacetonate, palladium(II) acetate, dichloro(1,5-cyclooctadiene)palladium(II), bis(dibenzylideneacetone)palladium, bis(acetonitrile)dichloropalladium(II), palladium(cinnamyl) dichloride.

13. Process according to any of Claims 10 to 12,
wherein the alcohol in process step c) is selected from methanol, ethanol, 1-propanol, 1-butanol, 1-pentanol, 1-hexanol, 2-propanol, *tert*-butanol, 3-pentanol, cyclohexanol, phenol, and mixtures thereof.

14. Use of a compound according to any of Claims 1 to 8 or of a complex according to Claim 9 for catalysis of an alkoxycarbonylation reaction.

## Revendications

1. Composé selon la formule (I) 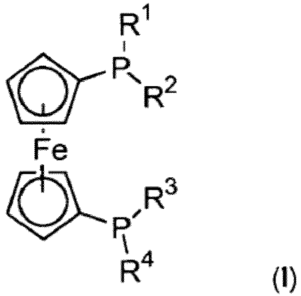 dans laquelle
R¹, R², R³, R⁴ sont chacun choisis indépendamment les uns des autres parmi alkyle en (C₁-C₁₂), cycloalkyle en (C₃-C₁₂), hétérocycloalkyle en (C₃-C₁₂), aryle en (C₆-C₂₀), hétéroaryle en (C₃-C₂₀) ; au moins un des radicaux R¹, R², R³, R⁴ représentant un radical hétéroaryle en (C₆-C₂₀) contenant au moins six atomes de cycle ;
et
R¹, R², R³, R⁴, si ceux-ci représentent alkyle en (C₁-C₁₂), cycloalkyle en (C₃-C₁₂), hétérocycloalkyle en (C₃-C₁₂), aryle en (C₆-C₂₀), hétéroaryle en (C₃-C₂₀) ou hétéroaryle en (C₆-C₂₀), peuvent être substitués chacun indépendamment les uns des autres avec un ou plusieurs substituants choisis parmi :
alkyle en (C₁-C₁₂), cycloalkyle en (C₃-C₁₂), hétérocycloalkyle en (C₃-C₁₂), -O-alkyle en (C₁-C₁₂), -O-alkyle en (C₁-C₁₂) -aryle en (C₆-C₂₀), -O-cycloalkyle en (C₃-C₁₂), -S-alkyle en (C₁-C₁₂), -S-cycloalkyle en (C₃-C₁₂), -COO-alkyle en (C₁-C₁₂), -COO-cycloalkyle en (C₃-C₁₂),-CONH-alkyle en (C₁-C₁₂), -CONH-cycloalkyle en (C₃-C₁₂),-CO-alkyle en (C₁-C₁₂), -CO-cycloalkyle en (C₃-C₁₂), -N-[alkyle en (C₁-C₁₂)]₂, aryle en (C₆-C₂₀), aryle en (C₆-C₂₀)-alkyle en (C₁-C₁₂), aryle en (C₆-C₂₀) -O-alkyle en (C₁-C₁₂), hétéroaryle en (C₃-C₂₀), hétéroaryle en (C₃-C₂₀)-alkyle en (C₁-C₁₂), hétéroaryle en (C₃-C₂₀)-O-alkyle en (C₁-C₁₂),-COOH, -OH, -SO₃H, -NH₂, halogène.

2. Composé selon la revendication 1, dans lequel au moins deux des radicaux R¹, R², R³, R⁴ représentent un radical hétéroaryle en (C₆-C₂₀) contenant au moins six atomes de cycle.

3. Composé selon l'une quelconque des revendications 1 à 2, dans lequel les radicaux R¹ et R³ représentent chacun un radical hétéroaryle en (C₆-C₂₀) contenant au moins six atomes de cycle.

4. Composé selon l'une quelconque des revendications 1 à 3, dans lequel les radicaux R¹ et R³ représentent chacun un radical hétéroaryle en (C₆-C₂₀) contenant au moins six atomes de cycle ;
R² représente hétéroaryle en (C₆-C₂₀) contenant au moins six atomes de cycle ou est choisi parmi alkyle en (C₁-C₁₂), cycloalkyle en (C₃-C₁₂), hétérocycloalkyle en (C₃-C₁₂), aryle en (C₆-C₂₀) ;
et R⁴ est choisi parmi alkyle en (C₁-C₁₂), cycloalkyle en (C₃-C₁₂), hétérocycloalkyle en (C₃-C₁₂), aryle en (C₆-C₂₀).

5. Composé selon l'une quelconque des revendications 1 à 4, dans lequel les radicaux R¹ et R³ représentent chacun un radical hétéroaryle en (C₆-C₂₀) contenant au moins six atomes de cycle ;
et R² et R⁴ sont choisis parmi alkyle en (C₁-C₁₂), cycloalkyle en (C₃-C₁₂), hétérocycloalkyle en (C₃-C₁₂), aryle en (C₆-C₂₀).

6. Composé selon l'une quelconque des revendications 1 à 5, dans lequel les radicaux R¹ et R³ représentent chacun un radical hétéroaryle en (C₆-C₂₀) contenant au moins six atomes de cycle ;
et R² et R⁴ représentent alkyle en (C₁-C₁₂).

7. Composé selon l'une quelconque des revendications 1 à 6, dans lequel R¹, R², R³, R⁴, si ceux-ci représentent un radical hétéroaryle, sont chacun choisis indépendamment les uns des autres parmi pyridyle, pyridazinyle, pyrimidyle, pyrazinyle, benzofuranyle, indolyle, isoindolyle, benzimidazolyle, quinolyle, isoquinolyle.

8. Composé selon l'une quelconque des revendications 1 à 7, selon l'une quelconque des formules (8), (14) et (15)

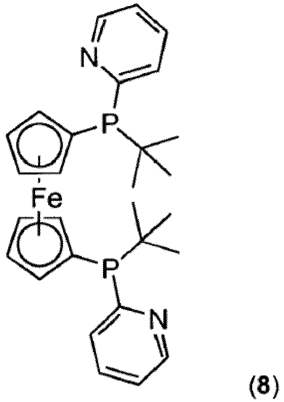

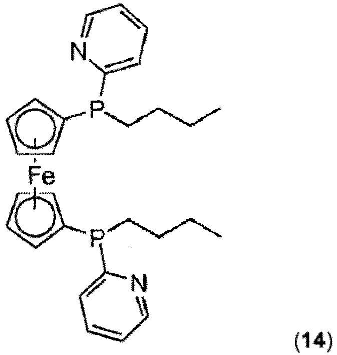

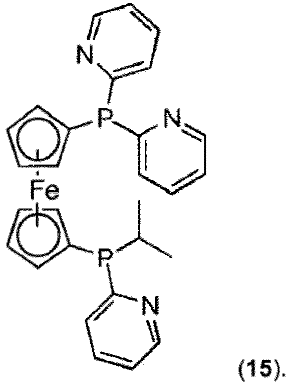

9. Complexe comprenant Pd et un composé selon l'une quelconque des revendications 1 à 8.

10. Procédé comprenant les étapes de procédé suivantes :
a) le chargement d'un composé éthyléniquement insaturé ;
b) l'ajout d'un composé selon l'une quelconque des revendications 1 à 8 et d'un composé qui comprend Pd, ou l'ajout d'un complexe selon la revendication 9 ;
c) l'ajout d'un alcool ;
d) l'introduction de CO ;
e) le chauffage du mélange réactionnel, le composé éthyléniquement insaturé étant transformé en un ester.

11. Procédé selon la revendication 10, dans lequel le composé éthyléniquement insaturé est choisi parmi l'éthène, le propène, le 1-butène, le *cis-* et/ou le *trans*-2-butène, l'iso-butène, le 1,3-butadiène, le 1-pentène, le *cis-* et/ou le *trans*-2-pentène, le 2-méthyl-1-butène, le 3-méthyl-1-butène, le 2-méthyl-2-butène, l'hexène, le tétraméthyléthylène, l'heptène, le 1-octène, le 2-octène, le di-n-butène ou leurs mélanges.

12. Procédé selon l'une quelconque des revendications 10 à 11, dans lequel le composé qui comprend Pd à l'étape de procédé b) est choisi parmi le dichlorure de palladium, l'acétylacétonate de palladium (II), l'acétate de palladium (II), le dichloro(1,5-cyclooctadiène)palladium (II), le bis(dibenzylidène-acétone)palladium, le bis(acétonitrile)dichloropalladium (II), le dichlorure de palladium(cinnamyle).

13. Procédé selon l'une quelconque des revendications 10 à 12, dans lequel l'alcool à l'étape de procédé c) est choisi parmi le méthanol, l'éthanol, le 1-propanol, le 1-butanol, le 1-pentanol, le 1-hexanol, le 2-propanol, le tert-butanol, le 3-pentanol, le cyclohexanol, le phénol ou leurs mélanges.

14. Utilisation d'un composé selon l'une quelconque des revendications 1 à 8 ou d'un complexe selon la revendication 9 pour la catalyse d'une réaction d'alcoxycarbonylation.
